(19) Europäisches Patentamt
European Patent Office
Office européen des brevets

(11) Publication number: **0 519 869 A2**

(12) **EUROPEAN PATENT APPLICATION**

(21) Application number: **92810449.6**

(22) Date of filing: **10.06.92**

(51) Int. Cl.$^5$: **C12N 15/29,** C12N 15/54, C12Q 1/68, C12N 15/82, A01H 5/00, A01H 1/02

The applicant has filed a statement in accordance with Rule 28 (4) EPC (issue of a sample only to an expert). Accession number(s) of the deposit(s): ATCC 67113 and ATCC 75036.

(30) Priority: **19.06.91 US 717331**

(43) Date of publication of application: **23.12.92 Bulletin 92/52**

(84) Designated Contracting States: **AT BE CH DE DK ES FR GB GR IT LI LU MC NL PT SE**

(71) Applicant: **CIBA-GEIGY AG**
**Klybeckstrasse 141**
**CH-4002 Basel (CH)**

(72) Inventor: **Nasrallah, June B.**
**109 Brook Drive**
**Ithaca, NY 14850 (US)**
Inventor: **Nasrallah, Mikhail E.**
**109 Brook Drive**
**Ithaca, NY 14850 (US)**
Inventor: **Stein, Joshua**
**8 Rickard Street**
**Cortland, NY 13048 (US)**

# (54) A receptor protein kinase gene encoded at the self-incompatibility locus.

(57) Described herein is a S receptor kinase gene (SRK), derived from the S locus in *Brassica oleracea*, having a extracellular domain highly similar to the secreted product of the S-locus glycoprotein gene.

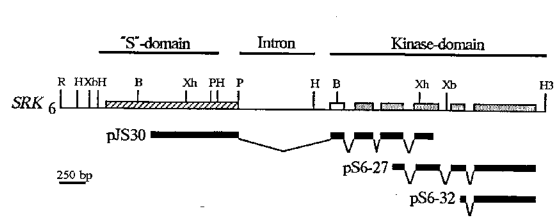


FIGURE 2

EP 0 519 869 A2

The present invention was made in the field of plant genetic engineering. In particular, the invention relates to DNA molecules comprising previously undescribed SRK genes and to the respective peptide sequences being encoded by the said DNA sequence.

Further comprised by the present invention are methods of cloning additional SRK genes; vectors containing the SRK genome; transgenic plants transformed with the SRK genome; the uses of SLG probes to isolate the SRK genome from plant-derived materials; and S-domain, transmembrane and kinase domain sequences of the SRK genomes; methods for imparting self-incompatibility to recipient plants by transforming self-incompatibility genes into appropriate plant strains; the establishment of transgenic plants containing the SRK and SRK-linked-SLG genomes; and the use of such transgenic plants to produce self-incompatible offspring.

Pollination and the subsequent invasive growth of pollen tubes into the female stigmatic and pistil tissues prior to fertilization provides an opportunity to study cell-cell interactions in flowering plants. In crucifers such as *Brassica oleracea*, self recognition between pollen and stigma is controlled by the multi-allelic self-incompatibility, or S locus. Pollen germination and/or tube growth are arrested at the stigma surface if pollen and stigma are borne by plants having identical S locus alleles. This arrest prevents self-fertilization and is termed the self-incompatibility (SI) response. Two related genes have been identified at the S locus using molecular methods. Of these, only one gene, the S locus glycoprotein (SLG) gene has been characterized extensively. SLG encodes a secreted glycoprotein that is highly polymorphic in different S-locus alleles, and its expression in stigmatic papillae and anthers, is consistent with models for SI in which both pollen and stigma bear recognition determinants derived from the S locus.

Plant science has recognized for many years that hybridization of closely related plants often results in the production of an offspring (F1) generation having a combination of desirable traits previously possessed separately by the parent plants. Also, hybrid plants of various crop species commonly possess vigor or heterosis which significantly contributes to the species' crop yield. Accordingly, many hybrid crosses are of considerable economic importance. Since plants selected for hybridization studies are commonly capable of undergoing both self-pollination (i.e., the plants are "self-compatible") and cross-pollination, the desired hybrid crosses have been difficult to achieve on a reliable basis while operating on a commercial scale. In order to achieve hybrid crosses on a commercial scale, it is necessary to control cross-pollination in the substantial absence of self-pollination (i.e., plants that are "self-incompatible").

In more than 3,000 species representing diverse angiosperm families, self-fertilization is blocked by a genetic controlled mechanism termed "self-incompatibility". This phenomenon facilitates out-crossing by inhibiting growth of self pollen in pistil tissue. Following self-pollination, interaction between the stigma or style and pollen elicits a defined morphological response preventing normal pollen tube growth. Fertilization is thus prevented as the pollen tubes are unable to penetrate the full length of the stigma and style, and gamete nuclei in the pollen are unable to reach female gametophyte tissue. The specificity of this interaction is determined by one or more genetic loci, and requires the identity of alleles carried by the male and female parents. The control of incompatibility is attributed to a single genetic locus with multiple alleles. As with other allelomorphs, any two alleles may be carried by a diploid plant; incompatibility occurs if the alleles expressed in pollen and pistil are identical.

Incompatibility systems are generally divided into two groupings: (i) the gametophytic system in which the phenotype of a pollen grain is determined by the haploid pollen genotype; and (ii) the sporophytic system in which the pollen phenotype is determined by the genotype of the parental plant. In the sporophytic system of incompatibility, exemplified by the genus *Brassica*, and other crucifers belonging to the family *Brassicaceae* (which includes broccoli, cabbage, kale, mustards, oil seed crops and radish), the self-incompatible inhibition is localized at the stigma outer surface and occurs within minutes by failure of self-pollen to germinate and/or invade the papillar cells on the stigma surface. Thus, self-fertilization is prevented. In *Brassicaceae*, self-incompatibility is is under control of a single multi-allelic locus (numerous alleles have been identified in natural populations), the S-locus, and phenotype of pollen is determined by diploid of the sporophyte [see Bateman (1955)]. While the mechanism of pollen recognition is not understood, observations that self-incompatibility responses occur when the same S-allele is active in pollen and stigma suggest pollen inhibition is based on expression of a single S-locus gene in the male and female structures of the flower. Sporophytic control of pollen phenotype has furthermore been postulated to result from expression of this S-locus gene in the tapetum, the layer of sporophytic cells that lines the anther and functions to nourish developing pollen grains [see Heslop-Harrison (1968) and (1975)]. Based largely on ultrastructural observations, this cellular layer is thought to act as nurse cells for developing microspores, and to synthesize and release materials that are incorporated into the outer coat (exine) of pollen grains just prior to and during tapetal dissolution late in pollen development.

A gene involved in the specific recognition of self-pollen is also expected to be sporophytically expressed in the host's anthers during pollen development. While blot analysis of anther RNA has identified a low-abundance transcript with homology to the S-locus glycoprotein gene [see Nasrallah and Nasrallah (1989)] these studies could not distinguish between expression of the S-locus glycoprotein related genes found in the *Brassica* genome.

Perhaps the most experimentally tractable route towards molecular analysis of genetic control for incompatibility is suggested by detection of antigens specific to various S-locus alleles in stigma homogenates from different *Brassica* strains which have been shown to correspond to glycoproteins and may be resolved in various electrophoretic systems. Several lines of evidence suggest these glycoproteins play an important role in incompatibility: (1) mobilities of these molecules vary in stigma extracts from *Brassica* strains with different S-locus alleles; (2) these molecules are found exclusively on the stigma surface (specifically in papillar cells), the site of the initial contact between the pollen and stigma; (3) an increased rate of synthesis of these S-locus specific glycoproteins in the developing stigma correlates well with onset of the incompatibility reaction in the stigma; and (4) glycoproteins in the stigma of self-incompatible lines of *B. oleracea* (kale), *B. campestris*, and related *Raphanus sativus* (radish) have been identified which co-segregate with their corresponding S-alleles in the F1 and F2 generations, indicating that the gene responsible for this polymorphism is at the S-locus.

These glycoproteins have been designated S-locus specific glycoprotein ("SLSG"). Each S-genotype has its own unique glycoprotein, and to date more than twenty different SLSG have been correlated with S-alleles. Furthermore, SLSG encoded by different S alleles exhibit extensive polymorphism and are distinguishable on the basis of their characteristic charge, molecular weight and antigenic properties. Also, SLSG accumulate during stigma development and are synthesized at a maximal rate coincident with acquisition of the developing stigma for the ability to reject self-pollen.

Cell-specific expression of these genes in the stigma are well characterized. S-locus gene transcripts were localized by *in situ* hybridization to the papillar cells of the stigma surface [see Nasrallah *et al* (1988)], and S-locus gene glycoprotein products were shown by ultra structural immunocytochemistry to accumulate in the papillar cell walls [see Kandasamy *et al* (1989)]. These results, and the genetic linkage of S-locus glycoprotein genes to the S-locus, have led to one aspect of the present invention, that is, that these genes encode the stigmatic determinants of self-incompatibility.

The S-locus glycoprotein genes, and specifically those genes from *B. oleracea* which have been designated as $S_6$, $S_{13}$, $S_{14}$ and $S_{22}$, and the $S_8$ genome from *B. campestris,* are described, for example, in EP-A 436 467 and Nasrallah *et al*, 1988.

Within the scope of the present invention a second S-locus-linked gene was discovered, which encodes a putative receptor protein kinase, and was designated SRK, for S-Receptor Kinase. Although only a limited number of SRK alleles are described herein, the term is meant to be broadly defined in that mutants or variants of the DNA sequences described herein for the SRK gene are to be considered part of the present invention provided that such mutants and variants express essentially similar peptides having essentially the same function as the peptides expressed by the SRK alleles described herein. The structure of this putative receptor is similar to that predicted in a recently described maize root cDNA clone, ZMPK1, whose function has not been determined [see Walker and Zhang (1990)]. Similar forces appear to have shaped the evolution of SRK and SLG, such that alleles of SRK also encode distinctive polymorphic variant. And like SLG, SRK is transcribed specifically in pistils and anthers. These facts implicate SRK not only as a determinant of pollen recognition, but as a primary transmitter of the recognition signal that leads to the SI response.

The present invention has a great number of different aspects including the description of DNA molecules comprising previously undescribed SRK genes and the respective peptide sequences being encoded by the said SRK genes, preferably previously undescribed $SRK_2$ DNA and peptide sequences and previously undescribed $SRK_6$ DNA and peptide sequences; methods of cloning additional SRK genes; vectors containing the SRK genome; transgenic plants transformed with the SRK genome; the uses of SLG probes to isolate the SRK genome from plant-derived materials; and S-domain, transmembrane and kinase domain sequences of the SRK genomes; methods for imparting self-incompatibility to recipient plants by transforming self-incompatibility genes into appropriate plant strains; the establishment of transgenic plants containing the SRK and SRK-linked-SLG genomes; and the use of such transgenic plants to produce self-incompatible offspring.

The present invention is primerily directed to an isolated and preferably an essentially pure DNA molecule comprising an SRK encoding DNA sequence and preferably the complete SRK gene comprising an S-locus binding domain, a transmembrane domain, and a protein kinase domain and to the SRK polypeptides being produced by the said SRK encoding DNA sequences or genes upon expression. Preferred within this invention is an isolated, essentially pure DNA molecule comprising the complete SRK gene, wherein the said S-locus binding domain is substantially homologous to an SLG gene, perferably to an SLG gene from *Brassica*, but most preferably to an SLG gene selected from the group consisting of *Brassica oleracea* S2, *Brassica oleracea* S6, *Brassica oleracea* S13, *Brassica oleracea* S14, *Brassica oleracea* S22, and *Brassica campestris* S8.

Substantial sequence homology means close structural and also functional relationship between sequences of nucleotides or amino acids. For example, substantially homologous DNA sequences may be 60% homologous, preferably 70% and most preferably 80% to 90% homologous, and substantially homologous amino acid sequences my typically be 50 % to 60 % homologous or more. Homology also includes a relationship wherein one or several subsequences of nucleotides or amino acids are missing, or subsequences with additional nucleotides or amino acids are interdispersed.

Also preferred is an isolated DNA molecule comprising an SRK encoding DNA sequence or gene comprising an S-locus binding domain, a transmembrane domain, and a protein kinase domain, wherein the kinase domain has a degree of homology, preferably a degree of homology of at least 35%, with the kinase domain beeing selected from the group consisting of the SRK6, SRK2, and ZMPK1 genes.

Further preferred is an isolated and preferably essentially pure DNA molecule, wherein said kinase domain is homologous to a kinase region from an SRK gene selected from the group consisting of the SRK2 gene of *Brassica oleracea*, *Brassica oleracea* SRK6 gene, *Brassica oleracea* SRK13 gene, *Brassica oleracea* SRK14 gene, *Brassica oleracea* SRK22 gene, and *Brassica campestris* SRK8 gene.

Especially preferred is an isolated and preferably essentially pure DNA molecule comprising an $SRK_2$ or an $SRK_6$ encoding DNA sequence or gene and the corresponding polypeptide sequences being produced by the said SRK genes upon expression.

More particularly and especially preferred, the present invention relates, in part, to the nucleotide sequence of the $SRK_6$ coding region as follows:

```
ATG AAA GGT GCA CGA AAC ATC TAT CAC CAT TCT TAC ATG 39
TCC TTT TTG CTC GTC TTC GTT GTC ATG ATT CTA ATT CAT 78
CCT GCC CTT TCG ATC TAT ATC AAC ACT TTG TCG TCT ACA 117
GAA TCT CTT ACA ATC TCA AGC AAC AAA ACA CTT GTA TCT 156
CCC GGT AGT ATC TTC GAG GTC GGC TTC TTC AGA ACC AAT 195
TCT CGT TGG TAT CTC GGG ATG TGG TAC AAG AAA GTG TCC 234
GAC AGA ACC TAT GTA TGG GTT GCC AAC AGA GAT AAC CCA 273
CTC TCC AAT GCC ATT GGA ACC CTC AAA ATC TCA GGC AAT 312
AAT CTT GTC CTC CTT GAT CAC TCC AAT AAA CCT GTT TGG 351
TGG ACG AAT CTT ACT AGA GGA AAT GAG AGA TCT CCG GTG 390
GTG GCT GAG CTT CTC GCT AAC GGA AAC TTC GTG ATG CGA 429
GAC TCC AGT AAC AAC GAC GCA AGT GAA TAC TTG TGG CAA 468
AGT TTC GAT TAC CCT ACG GAT ACT TTG CTT CCA GAG ATG 507
AAA CTG GGT TAC AAC CTC AAA ACA GGG TTG AAC AGG TTC 546
CTT ACA TCA TGG AGA AGT TCA GAT GAT CCA TCA AGC GGG 585
AAT TTC TCG TAC AAG CTC GAA ACC CAA AGT CTT CCT GAG 624
TTT TAT CTA TCG CGG GAG AAC TTT CCA ATG CAT CGG AGT 663
GGT CCA TGG AAT GGA ATC CGA TTT AGT GGC ATA CCA GAG 702
GAC CAA AAG CTG AGT TAC ATG GTG TAC AAT TTC ATA GAG 741
AAT AAT GAA GAG GTC GCT TAT ACA TTC CGA ATG ACC AAC 780
AAC AGC TTC TAC TCG AGA TTG ACA CTA ATT TCC GAA GGG 819
TAT TTT CAG CGA CTG ACG TGG TAT CCG TCA ATA AGG ATA 858
TGG AAC AGG TTC TGG TCT TCT CCA GTG GAC CCC CAG TGT 897
GAT ACT TAC ATA ATG TGT GGA CCT TAC GCT TAC TGT GAC 936
GTG AAC ACA TCA CCG GTT TGT AAC TGT ATC CAA GGG TTC 975
AAT CCC CGG AAT ATA CAG CAG TGG GAT CAG AGA GTC TGG 1014
```

GCA GGT GGG TGT ATA AGG AGG ACG CAG CTT AGC TGC AGT 1053
GGA GAT GGT TTT ACC AGG ATG AAG AAG ATG AAG TTG CCA 1092
GAA ACT ACG ATG GCG ACT GTC GAC CGT AGT ATT GGT GTG 1131
AAA GAA TGT AAG AAG AGG TGC ATT AGC GAT TGT AAT TGT 1170
ACC GCT TTT GCA AAT GCA GAT ATC CGG AAT GGT GGG TCG 1209
GGT TGT GTG ATT TGG ACC GAA CGC CTT GAG GAT ATC CGG 1248
AAT TAC GCT ACT GAC GCT ATT GAC GGT CAA GAT CTT TAT 1287
GTC AGA TTG GCT GCA GCT GAT ATC GCT AAG AAG AGA AAC 1326
GCG AGT GGG AAA ATT ATA AGT TTG ACT GTT GGT GTT AGT 1365
GTT CTG CTT CTG CTG ATC ATG TTC TGC CTC TGG AAA AGA 1404
AAA CAA AAG CGA GCA AAA GCA AGT GCA ATA TCC ATT GCA 1443
AAT ACA CAG AGA AAC CAA AAC TTG CCT ATG AAC GAG ATG 1492
GTA CTA TCA AGC AAG AGA GAG TTT TCT GGA GAG TAC AAA 1521
TTT GAG GAA CTG GAA CTT CCA TTG ATA GAG ATG GAA ACT 1560
GTT GTC AAA GCC ACC GAA AAT TTC TCC AGC TGT AAC AAA 1599
CTC GGA CAA GGT GGT TTT GGT ATT GTT TAC AAG GGA AGA 1638
TTA CTT GAC GGG AAA GAA ATT GCA GTA AAA AGG CTA TCA 1677
AAG ACG TCA GTT CAA GGG ACT GAT GAG TTT ATG AAT GAG 1716
GTG ACA CTA ATT GCG AGG CTT CAG CAT ATA AAC CTT GTT 1755
CAA GTT CTT GGC TGT TGC ATT GAA GGA GAT GAG AAG ATG 1794
TTG ATA TAT GAG TAT TTG GAA AAT TTA AGC CTT GAT TCT 1833
TAT CTC TTT GGT AAA ACC CGA AGG TCT AAG CTA AAT TGG 1882
AAT GAG AGA TTC GAC ATT ACC AAT GGT GTT GCT CGA GGG 1911
CTT TTA TAT CTT CAT CAA GAC TCA CGG TTT AGG ATA ATC 1950
CAC AGA GAT TTG AAA GTA AGT AAC ATT TTG CTT GAC AAA 1989
AAT ATG ATC CCA AAG ATC TCG GAT TTT GGG ATG GCC AGG 2028
ATA TTT GAA AGG GAC GAA ACG GAA GCT AAC ACA ATG AAG 2067
GTG GTC GGA ACA TAC GGC TAC ATG TCC CCG GAA TAC GCA 2106
ATG TAT GGG ATA TTC TCG GAA AAA TCA GAT GTT TTC AGT 2145
TTT GGA GTC ATA GTT CTT GAA ATT GTT AGT GGA AAG AAG 2184
AAC AGA GGA TTC TAC AAC TTG GAC TAC GAA AAC GAT CTC 2223
CTA AGC TAT GTA TGG AGT CGT TGG AAG GAA GGA AGA GCG 2272
CTA GAA ATC GTA GAT CCC GTC ATC GTA GAT TCA CTG TCA 2301
TCA CAG CCA TCA ATA TTT CAA CCA CAA GAA GTC CTA AAA 2340
TGT ATT CAA ATT GGT CTC TTG TGT GTT CAA GAA CTT GCA 2379
GAG CAC AGA CCA GCG ATG TCG TCT GTG GTT TGG ATG TTT 2418

GGA AGT GAA GCA ACA GAG ATT CCT CAG CCT AAA CCG CCA 2457
GGT TAT TGC GTC AGA AGA AGT CCT TAT GAA CTT GAT CCT 2496
TCA TCA AGT TGG CAA TGT GAC GAA AAT GAA TCC TGG ACG 2535
GTG AAC CAG TAC ACC TGC TCA GTC ATT GAT GCC CGG 2571
TAATATGATA GCTGAGTGAT TCAATATCAT ATGTGAAAGA GGGAAAATAA 2621
AATCTCATTA GATAAGTAGG TTATTTCGAT AACCACTTCT TGTTATTTTC 2671
TGGCGGTGTT GTCATTATCC CCTTTATATT AAAAAGAAGC ATTTGTATTA 2721
AATCCCCTTG CCTCAAGAGA TATTCACAAG AATACTATTG TGACGTGACA 2771
GCCTCACTAT CGTTTAAACA TTACAATGCT GACGTGTGGC TTGTAAATAG 2821
CTTCTCAGAC CA 2833

In this sequence, bases 1-478 are obtainable from the genomic clone; the remaining sequence is obtainable from cDNA clones pJS30 (nucleotides 479-2001), pS6-27 (nucleotides 1723-2763), and pS6-32 (nucleotides 2180-2833). The underlined nucleotides denote the positions of the putative signal-yielding peptide (1-97), and the transmembrane domains (1340-1399). The S-locus binding domain of this DNA molecule has about 70% homology to an SLG gene from *Brassica*.

The corresponding amino acid sequence being encoded by the coding region of the above $SRK_6$ gene according to the present invention is:

Met Lys Gly Ala Arg Asn Ile Tyr His His Ser Tyr Met Ser Phe
5 10 15
Leu Leu Val Phe Val Val Met Ile Leu Ile His Pro Ala Leu Ser
20 25 30
Ile Tyr Ile Asn Thr Leu Ser Ser Thr Glu Ser Leu Thr Ile Ser
35 40 45
Ser Asn Lys Thr Leu Val Ser Pro Gly Ser Ile Phe Glu Val Gly
50 55 60
Phe Phe Arg Thr Asn Ser Arg Trp Tyr Leu Gly Met Trp Tyr Lys
65 70 75
Lys Val Ser Asp Arg Thr Tyr Val Trp Val Ala Asn Arg Asp Asn
80 85 90
Pro Leu Ser Asn Ala Ile Gly Thr Leu Lys Ile Ser Gly Asn Asn
95 100 105
Leu Val Leu Leu Asp His Ser Asn Lys Pro Val Trp Trp Thr Asn

110 115 120
Leu Thr Arg Gly Asn Glu Arg Ser Pro Val Val Ala Glu Leu Leu
125 130 135
Ala Asn Gly Asn Phe Val Met Arg Asp Ser Ser Asn Asn Asp Ala
140 145 150
Ser Glu Tyr Leu Trp Gln Ser Phe Asp Tyr Pro Thr Asp Thr Leu
155 160 165
Leu Pro Glu Met Lys Leu Gly Tyr Asn Leu Lys Thr Gly Leu Asn
170 175 180
Arg Phe Leu Thr Ser Trp Arg Ser Ser Asp Asp Pro Ser Ser Gly
185 190 195
Asn Phe Ser Tyr Lys Leu Glu Thr Gln Ser Leu Pro Glu Phe Tyr
200 205 210
Leu Ser Arg Glu Asn Phe Pro Met His Arg Ser Gly Pro Trp Asn
215 220 225
Gly Ile Arg Phe Ser Gly Ile Pro Glu Asp Gln Lys Leu Ser Tyr
230 235 240
Met Val Tyr Asn Phe Ile Glu Asn Asn Glu Glu Val Ala Tyr Thr
245 250 255
Phe Arg Met Thr Asn Asn Ser Phe Tyr Ser Arg Leu Thr Leu Ile
260 265 270
Ser Glu Gly Tyr Phe Gln Arg Leu Thr Trp Tyr Pro Ser Ile Arg
275 280 285
Ile Trp Asn Arg Phe Trp Ser Ser Pro Val Asp Arg Gln Cys Asp
290 295 300
Thr Tyr Ile Met Cys Gly Pro Tyr Ala Tyr Cys Asp Val Asn Thr
305 310 315
Ser Pro Val Cys Asn Cys Ile Gln Gly Phe Asn Pro Arg Asn Ile
320 325 330
Gln Gln Trp Asp Gln Arg Val Trp Ala Gly Gly Cys Ile Arg Arg
335 340 345
Thr Gln Leu Ser Cys Ser Gly Asp Gly Phe Thr Arg Met Lys Lys
350 355 360
Met Lys Leu Pro Glu Thr Thr Met Ala Thr Val Asp Arg Ser Ile
365 370 375
Gly Val Lys Glu Cys Lys Lys Arg Cys Ile Ser Asp Cys Asn Cys

380 385 390
Thr Ala Phe Ala Asn Ala Asp Ile Arg Asn Gly Gly Ser Gly Cys
395 400 405
Val Ile Trp Thr Glu Arg Leu Glu Asp Ile Arg Asn Tyr Ala Thr
410 415 420
Asp Ala Ile Asp Gly Gln Asp Leu Tyr Val Arg Leu Ala Ala Ala
425 430 435
Asp Ile Ala Lys Lys Arg Asn Ala Ser Gly Lys Ile Ile Ser Leu
440 445 450
Thr Val Gly Val Ser Val Leu Leu Leu Leu Ile Met Phe Cys Leu
455 460 465
Trp Lys Arg Lys Gln Lys Arg Ala Lys Ala Ser Ala Ile Ser Ile
470 475 480
Ala Asn Thr Gln Arg Asn Gln Asn Leu Pro Met Asn Glu Met Val
485 490 495
Leu Ser Ser Lys Arg Glu Phe Ser Gly Glu Tyr Lys Phe Glu Glu
500 505 510
Leu Glu Leu Pro Leu Ile Glu Met Glu Thr Val Val Lys Ala Thr
515 520 525
Glu Asn Phe Ser Ser Cys Asn Lys Leu Gly Gln Gly Gly Phe Gly
530 535 540
Ile Val Tyr Lys Gly Arg Leu Leu Asp Gly Lys Glu Ile Ala Val
545 550 555
Lys Arg Leu Ser Lys Thr Ser Val Gln Gly Thr Asp Glu Phe Met
560 565 570
Asn Glu Val Thr Leu Ile Ala Arg Leu Gln His Ile Asn Leu Val
575 580 585
Gln Val Leu Gly Cys Cys Ile Glu Gly Asp Glu Lys Met Leu Ile
590 595 600
Tyr Glu Tyr Leu Glu Asn Leu Ser Leu Asp Ser Tyr Leu Phe Gly
605 610 615
Lys Thr Arg Arg Ser Lys Leu Asn Trp Asn Glu Arg Phe Asp Ile
620 625 630
Thr Asn Gly Val Ala Arg Gly Leu Leu Tyr Leu His Gln Asp Ser
635 640 645
Arg Phe Arg Ile Ile His Arg Asp Leu Lys Val Ser Asn Ile Leu

| | | | | | | | | | | | | | | |
|---|---|---|---|---|---|---|---|---|---|---|---|---|---|---|
| | | | | 650 | | | | | 655 | | | | | 660 |
| Leu | Asp | Lys | Asn | Met | Ile | Pro | Lys | Ile | Ser | Asp | Phe | Gly | Met | Ala |
| | | | | 665 | | | | | 670 | | | | | 675 |
| Arg | Ile | Phe | Glu | Arg | Asp | Glu | Thr | Glu | Ala | Asn | Thr | Met | Lys | Val |
| | | | | 680 | | | | | 685 | | | | | 690 |
| Val | Gly | Thr | Tyr | Gly | Tyr | Met | Ser | Pro | Glu | Tyr | Ala | Met | Tyr | Gly |
| | | | | 695 | | | | | 700 | | | | | 705 |
| Ile | Phe | Ser | Glu | Lys | Ser | Asp | Val | Phe | Ser | Phe | Gly | Val | Ile | Val |
| | | | | 710 | | | | | 715 | | | | | 720 |
| Leu | Glu | Ile | Val | Ser | Gly | Lys | Lys | Asn | Arg | Gly | Phe | Tyr | Asn | Leu |
| | | | | 725 | | | | | 730 | | | | | 735 |
| Asp | Tyr | Glu | Asn | Asp | Leu | Leu | Ser | Tyr | Val | Trp | Ser | Arg | Trp | Lys |
| | | | | 740 | | | | | 745 | | | | | 750 |
| Glu | Gly | Arg | Ala | Leu | Glu | Ile | Val | Asp | Pro | Val | Ile | Val | Asp | Ser |
| | | | | 755 | | | | | 760 | | | | | 765 |
| Leu | Ser | Ser | Gln | Pro | Ser | Ile | Phe | Gln | Pro | Gln | Glu | Val | Leu | Lys |
| | | | | 770 | | | | | 775 | | | | | 780 |
| Cys | Ile | Gln | Ile | Gly | Leu | Leu | Cys | Val | Gln | Glu | Leu | Ala | Glu | His |
| | | | | 785 | | | | | 790 | | | | | 795 |
| Arg | Pro | Ala | Met | Ser | Ser | Val | Val | Trp | Met | Phe | Gly | Ser | Glu | Ala |
| | | | | 800 | | | | | 805 | | | | | 810 |
| Thr | Glu | Ile | Pro | Gln | Pro | Lys | Pro | Pro | Gly | Tyr | Cys | Val | Arg | Arg |
| | | | | 815 | | | | | 820 | | | | | 825 |
| Ser | Pro | Tyr | Glu | Leu | Asp | Pro | Ser | Ser | Ser | Trp | Gln | Cys | Asp | Glu |
| | | | | 830 | | | | | 835 | | | | | 840 |
| Asn | Glu | Ser | Trp | Thr | Val | Asn | Gln | Tyr | Thr | Cys | Ser | Val | Ile | Asp |
| | | | | 845 | | | | | 850 | | | | | 855 |
| Ala | Arg | | | | | | | | | | | | | |

In the preceding amino acid sequence, the putative signal peptide and transmembrane domain are underlined. The kinase subdomains, of which there are eleven, are shown extending from amino acid 523 to 814.

In addition, the amino acid sequence for the $SRK_2$ coding region according to the present invention is:

Met Lys Gly Val Gln Asn Ile Tyr His His Ser Tyr Thr Phe Ser
5 10 15
Phe Leu Leu Val Phe Leu Val Leu Ile Leu Phe His Pro Ala Leu
20 25 30
Ser Ile Tyr Val Asn Thr Leu Ser Ser Ser Glu Ser Leu Thr Ile
35 40 45
Ser Ser Asn Arg Thr Leu Val Ser Pro Gly Gly Val Phe Glu Leu
50 55 60
Gly Phe Phe Lys Pro Leu Gly Arg Ser Arg Trp Tyr Leu Gly Ile
65 70 75
Trp Tyr Lys Lys Ala Pro Trp Lys Thr Tyr Ala Trp Val Ala Asn
80 85 90
Arg Asp Asn Pro Leu Ser Ser Ser Ile Gly Thr Leu Lys Ile Ser
95 100 105
Gly Asn Asn Leu Val Leu Leu Ser Gln Ser Thr Asn Thr Val Trp
110 115 120
Ser Thr Asn Leu Thr Arg Gly Asn Ala Arg Ser Pro Val Ile Ala
125 130 135
Glu Leu Leu Pro Asn Gly Asn Phe Val Ile Arg His Ser Asn Val
140 145 150
Asn Lys Asp Ser Ser Gly Phe Leu Trp Gln Ser Phe Asp Phe Pro
155 160 165
Thr Asp Thr Leu Leu Pro Glu Met Lys Leu Gly Tyr Asp Leu Lys
170 175 180
Thr Gly Arg Asn Arg Phe Leu Thr Ser Trp Lys Gly Ser Asp Asp
185 190 195
Pro Ser Ser Gly Asn Phe Val Tyr Lys Leu Asp Ile Arg Arg Gly
200 205 210
Leu Pro Glu Phe Ile Leu Ile Asn Gln Phe Leu Asn Gln Arg Val
215 220 225
Glu Thr Gln Arg Ser Gly Pro Trp Asn Gly Met Glu Phe Ser Gly
230 235 240
Ile Pro Glu Val Gln Gly Leu Asn Tyr Met Val Tyr Asn Tyr Thr
245 250 255
Glu Asn Ser Glu Glu Ile Ala Tyr Ser Phe His Met Thr Asn Gln

260 265 270
Ser Ile Tyr Ser Arg Leu Thr Leu Val Ser Glu Leu Thr Leu Asp
275 280 285
Arg Leu Thr Trp Ile Pro Pro Ser Arg Asp Trp Trp Ser Leu Phe
290 295 300
Trp Thr Leu Pro Thr Asp Val Cys Asp Pro Leu Tyr Leu Cys Gly
305 310 315
Ser Tyr Ser Tyr Cys Asp Leu Ile Thr Ser Pro Asn Cys Asn Cys
320 325 330
Ile Arg Gly Phe Val Pro Lys Asn Pro Gln Gln Trp Asp Leu Arg
335 340 345
Asp Gly Thr Arg Gly Cys Val Arg Thr Thr Gln Met Ser Cys Ser
350 355 360
Gly Asp Gly Phe Leu Arg Leu Asn Asn Met Asn Leu Pro Asp Thr
365 370 375
Lys Thr Ala Thr Val Asp Arg Thr Met Asp Val Lys Lys Cys Glu
380 385 390
Glu Arg Cys Leu Ser Asp Cys Asn Cys Thr Ser Phe Ala Ile Ala
395 400 405
Asp Val Arg Asn Gly Gly Leu Gly Cys Val Phe Trp Thr Gly Glu
410 415 420
Leu Val Ala Ile Arg Lys Phe Ala Val Gly Gly Gln Asp Leu Tyr
425 430 435
Val Arg Leu Asn Ala Ala Asp Leu Asp Ile Ser Ser Gly Glu Lys
440 445 450
Arg Asp Arg Thr Gly Lys Ile Ile Gly Trp Ser Ile Gly Ser Ser
455 460 465
Val Met Leu Ile Leu Ser Val Ile Leu Phe Cys Phe Trp Arg Arg
470 475 480
Arg Gln Lys Gln Ala Lys Ala Asp Ala Thr Pro Ile Val Asn Asn
485 490 495
Gln Val Leu Met Asn Glu Val Val Leu Pro Arg Lys Lys Arg Asn
500 505 510
Phe Ser Gly Glu Asp Asp Val Glu Asn Leu Glu Leu Pro Leu Met
515 520 525
Glu Phe Glu Ala Val Val Thr Ala Thr Glu His Phe Ser Asp Phe

530 535 540
Asn Lys Val Gly Lys Gly Gly Phe Gly Val Val Tyr Lys Gly Arg
545 550 555
Leu Val Asp Gly Gln Glu Ile Ala Val Lys Arg Leu Ser Glu Met
560 565 570
Ser Ala Gln Gly Thr Asp Glu Phe Met Asn Glu Val Arg Leu Met
575 580 585
Gln Ser Phe Ser His Asn Asn Leu Val Arg Leu Leu Gly Cys Cys
590 595 600
Val Tyr Glu Gly Glu Lys Ile Leu Ile Tyr Glu Tyr Leu Glu Asn
605 610 615
Leu Ser Leu Asp Ser His Leu Asp Glu Thr Arg Ser Cys Met Leu
620 625 630
Asn Trp Gln Met Arg Phe Asp Ile Ile Asn Gly Ile Ala Arg Gly
635 640 645
Leu Leu Tyr Leu His Gln Asp Ser Arg Phe Arg Ile Ile His Arg
650 655 660
Asp Leu Lys Ala Ser Asn Val Leu Leu Asp Lys Asp Met Thr Pro
665 670 675
Lys Ile Ser Asp Phe Gly Met Ala Arg Ile Phe Gly Arg Asp Glu
680 685 690
Thr Glu Ala Asp Thr Arg Lys Val Val Gly Thr Tyr Gly Tyr Met
695 700 705
Ser Pro Glu Tyr Ala Met Asn Gly Thr Phe Ser Met Lys Ser Asp
710 715 720
Val Phe Ser Phe Gly Val Ile Leu Leu Glu Ile Ile Ser Gly Lys
725 730 735
Arg Asn Lys Gly Leu Cys Asp Leu Asp Ser Ser Leu Asn Leu Leu
740 745 750
Gly Cys Val Trp Arg Asn Trp Lys Glu Gly Gln Gly Leu Glu Ile
755 760 765
Val Asp Lys Val Ile Ile Asp Ser Ser Ser Pro Thr Phe Arg Pro
770 775 780
Arg Glu Ile Leu Arg Cys Leu Gln Ile Gly Leu Leu Cys Val Gln
785 790 795
Glu Arg Val Glu Asp Arg Pro Met Met Ser Ser Val Val Leu Met

```
                    800                     805                     810
Leu Gly Ser Glu Ala Ala Leu Ile Pro Gln Pro Lys Gln Pro Gly
                    815                     820                     825
Tyr Cys Val Ser Gly Ser Ser Leu Glu Thr Tyr Ser Arg Arg Asp
                    830                     835                     840
Asp Glu Asn Cys Thr Val Asn Gln Ile Thr Met Ser Ile Ile Asp
                    845                     850                     855
Ala Arg
```

This amino acid sequence corresponds, in part, to the following predicted sequence of the $SRK_2$ coding region determined by the removal of putative promoter and intron sequences from the sequence of the $SRK_2$ gene which included all exons and introns, said $SRK_2$ gene being also especially preferred within the scope of this invention. Thus, the initial 2571 nucleotides in the following nucleic acid sequence, the structural gene, was used to determine the polypeptide sequence given above.

```
ATG AAA GGG GTA CAG AAC ATT TAC CAC CAT TCT TAC ACC 39
TTC TCG TTC TTG CTA GTC TTC CTT GTC TTG ATT CTA TTT 78
CAT CCT GCC CTT TCG ATC TAT GTC AAC ACT TTA TCG TCT 117
TCA GAG TCT CTC ACA ATC TCG AGC AAT AGA ACA CTT GTA 156
TCT CCC GGT GGA GTC TTC GAG CTT GGT TTC TTC AAA CCC 195
TTG GGA CGC TCG CGA TGG TAT CTG GGA ATA TGG TAT AAA 234
AAA GCC CCC TGG AAA ACC TAC GCA TGG GTC GCC AAC AGA 273
GAC AAC CCT CTC TCC AGT TCT ATT GGA ACC CTC AAA ATC 312
TCT GGC AAC AAT CTT GTC CTG CTA AGT CAG TCT ACT AAC 351
ACT GTT TGG TCG ACA AAT CTT ACT AGA GGA AAT GCG AGA 390
TCT CCG GTG ATA GCA GAG CTT CTT CCC AAC GGT AAT TTT 429
GTA ATA AGA CAC TCC AAC AAC AAA GAC TCA AGT GGA TTC 468
TTG TGG CAG AGT TTC GAT TTT CCG ACA GAT ACT TTA CTT 507
CCG GAG ATG AAA CTA GGT TAC GAT CTC AAA ACA GGG CGC 546
AAC AGG TTC CTT ACA TCG TGG AAA GGT TCA GAT GAT CCG 585
TCA AGC GGG AAT TTC GTG TAC AAA CTC GAC ATT CGA AGG 624
GGA TTG CCT GAG TTT ATT CTT ATA AAT CAA TTT TTG AAT 663
CAA CGT GTT GAA ACG CAA AGG AGC GGT CCT TGG AAT GGA 702
ATG GAG TTT AGT GGC ATA CCG GAG GTG CAG GGA TTA AAT 741
TAC ATG GTT TAC AAT TAT ACG GAG 765 AAC AGT GAG GAG 780
```

ATC GCT TAC TCG TTC CAT ATG ACC AAC CAA AGC ATC TAC 819
TCC AGA TTG ACA GTC AGT GAG TTG ACA CTC GAT CGA TTG 858
ACG TGG ATC CCG CCA TCA CGG GAT TGG AGC CTC TTC TGG 897
ACT TTA CCA ACG GAC GTG TGC GAT CCG CTT TAC TTA TGT 936
GGA TCT TAT TCT TAC TGT GAC CTA ATT ACG TCA CCT AAC 975
TGT AAC TGT ATT AGA GGG TTC GTT CCC AAG AAC CCG CAG 1014
CAG TGG GAC TTG AGA GAC GGA ACA CGG GGG TGT GTG AGG 1053
ACG ACG CAG ATG AGC TGT AGT GGA GAT GGG TTT TTG CGG 1092
CTA AAC AAT ATG AAT TTG CCG GAT ACT AAG ACG GCA ACT 1131
GTG GAT CGG ACA ATG GAT GTG AAA AAA TGT GAA GAG AGG 1170
TGT CTT AGC GAT TGT AAC TGT ACT TCG TTT GCT ATT GCG 1209
GAT GTT CGG AAT GGA GGA TTG GGT TGT GTG TTT TGG ACC 1248
GGA GAG CTC GTT GCG ATC AGG AAA TTC GCC GTC GGT GGT 1287
CAA GAT CTT TAC GTC AGA TTG AAT GCT GCT GAT CTA GAT 1326
ATT TCC TCG GGT GAG AAG AGA GAC CGA ACT GGA AAA ATC 1365
ATA GGT TGG AGT ATT GGA TCC AGC GTT ATG CTT ATT CTG 1404
AGT GTT ATC TTG TTC TGC TTT TGG AGG AGG AGA CAA AAG 1443
CAA GCA AAA GCA GAT GCA ACA CCT ATT GTG GGA AAT CAA 1492
GTT CTA ATG AAC GAG GTG GTG TTA CCA AGA AAG AAG AGA 1521
AAT TTT TCT GGA GAG GAC GAT GTA GAA AAT TTG GAA CTT 1560
CCA TTG ATG GAG TTT GAA GCT GTT GTC ACA GCC ACC GAA 1599
CAT TTC TCT GAT TTT AAC AAG GTC GGA AAA GGT GGT TTT 1638
GGT GTT GTT TAC AAG GGA AGG TTA GTT GAC GGG CAA GAA 1677
ATT GCA GTG AAG AGA CTA TCG GAA ATG TCA GCT CAA GGT 1716
ACC GAT GAG TTC ATG AAC GAA GTT AGG CTA ATG CAA AGC 1755
TTC AGC CAC AAT AAT CTT GTC CGA CTT CTT GGC TGT TGT 1794
GTT TAT GAG GGC GAG AAG ATC TTA ATT TAC GAG TAC TTG 1833
GAG AAT CTA AGC CTC GAT TCT CAT CTC TTT GAT GAA ACC 1882
AGA AGC TGT ATG TTA AAT TGG CAA ATG AGA TTT GAT ATT 1911
ATC AAT GGT ATT GCC CGA GGG CTT CTC TAT CTT CAC CAA 1950
GAT TCA CGG TTT AGA ATC ATC CAC AGG GAT TTG AAA GCA 1989
AGC AAT GTC TTG CTT GAT AAA GAT ATG ACT CCA AAA ATT 2028
TCA GAC TTT GGA ATG GCT AGG ATC TTT GGA CGG GAT GAG 2067
ACG GAA GCT GAC ACG AGG AAG GTG GTC GGA ACT TAT GGC 2106
TAC ATG TCT CCA GAA TAT GCG ATG AAC GGG ACA TTC TCA 2145
ATG AAG TCA GAT GTG TTC AGT TTT GGG GTC TTG CTT CTT 2184

GAA ATT ATA AGT GGC AAG AGG AAC AAA GGC TTA TGC GAC 2223
TCG GAT AGT AGC CTT AAT CTT CTC GGA TGT GTA TGG AGG 2272
AAT TGG AAA GAA GGT CAA GGT CTA GAG ATA GTA GAC AAG 2301
GTC ATC ATA GAT TCT TCA TCA CCA ACG TTC AGG CCA CGT 2340
GAA ATC TTA AGA TGC TTA CAA ATT GGC CTC TTG TGT GTT 2379
CAA GAA CGT GTG GAG GAT AGA CCA ATG ATG TCG TCA GTA 2418
GTT TTG ATG CTC GGA AGT GAA GCT GCA TTG ATT CCT CAA 2457
CCT AAA CAG CCA GGA TAT TGC GTC AGC GGA AGT TCT CTT 2496
GAA ACT TAT TCT AGG CGT GAC GAT GAA AAT TGC ACA GTG 2535
AAC CAA ATC ACC ATG TCG ATC ATT GAC GCT CGG TAA TAT 2574
GAT AGT CTT TGA TAA TAT TCT CAC TAT TAA AGT TTT ACT 2613
AAA TGG AAA AAA AGA GTT TTA CAA GTT GAG TGA CAA AGC 2652
GTG CCA AAC TCT TCA GTC TAT CGA AAT TTT CAT TCA TCC 2691
TCT GTA TAT TCT CTC GAA TTG GTT TCG TTA TTT CGA GTC 2730
AAT TCA CAG TCA ACA ACT GCA G 2749

It is a further embodiment of the present invention to provide a method which is directed to the isolation of an essentially pure DNA molecule comprising a SRK encoding DNA sequence or gene and to the use of both a SLG encoding DNA sequence or gene and an SRK encoding sequence or gene, and which will be described in detail below. In this method use may be made of SRK and SLG encoding DNA sequences or genes which have been isolated from a homozygous self-incompatible donor plant having a genotype selected from the group consisting of *Brassica oleracea* S2, *Brassica oleracea* S6, *Brassica oleracea* S13, *Brassica oleracea* S14, *Brassica oleracea* S22, and *Brassica campestris* S8. The SLG and SRK genes derived from an S-allele homozygote genotype share about 90% DNA and amino sequence identity in their S regions. This observation suggests that these genes act in concert to determine S-allele specificity. The determination of self-incompatibility phenotype thus appears to require a minimum expression of the two linked S-locus linked genes.

In particular, the present invention relates to a method of isolating and identifying an SRK encoding DNA sequence or gene comprising

(a) isolating one or more DNA sequences from an SLG gene clone from a self-incompatible plant;

(b) using the isolated sequences isolated from an SLG clone to screen a genomic library from a self-incompatible plant;

(c) isolating those clones from the genomic DNA library which hybridize to the DNA sequences isolated from an SLG gene clone; and

(d) sequencing those clones from the genomic DNA library which hybridize and identify SRK encoding DNA sequences or genes.

More particularly, the present invention relates to a method for isolating and identifying an SRK encoding DNA sequence or gene comprising:

(a) isolating a genomic SLG clone from a self-incompatible plant;

(b) using hybridization to identify putative SRK encoding DNA sequences or genes which are SLG-related and kinase-related residue on the genomic clone;

(c) sequencing the putative SRK encoding DNA sequences or genes identified above to determine if one or more encode an SRK gene;

(d) if no SRK encoding DNA sequences or genes are identified on the genomic clone, using the end sequences of the clone insert as probes to isolate new genomic clones from the adjacent region of the chromosome;

(e) using hybridization to determine if any putative SRK encoding DNA sequences or genes reside on the new genomic clones;

(f) sequencing the putative SRK encoding DNA sequences or genes identified above to determine if one or more encode an SRK polypeptide; and

(g) repeating the steps above until an SRK encoded DNA sequence or genes located near the SLG gene is identified.

The present invention further relates to a method of imparting self-incompatibility to recipient plants comprising isolating a DNA sequence or a gene which encodes for the expression of an S Receptor Kinase from a self-incompatible plant donor, which preferably is a self-incompatible strain of *Brassica* carrying the SLG gene; and integrating the isolated DNA sequence or gene into the genome of a recipient plant that does not ordinarily produce S-receptor kinase whereby the recipient plant is self-incompatible.

In a specific embodiment of the invention the isolated DNA sequence encoding the expression of an S Receptor Kinase is operably linked to one or more further structural genes coding, for example, for the production of an S-locus specific glycoprotein and/or for a polypeptide that is capable of imparting a herbicide tolerance to one or more herbicides.

Also comprised by the present invention are plants normally lacking a S Receptor Kinase Gene which have been successfully transformed with a transformation vector which comprises a S Receptor Kinase Gene.

Further comprised is a method for obtaining a plant with an acquired self-incompatibility trait which comprises.

(a) providing a first plant having a SLG encoding DNA sequence or gene within its genome;
(b) providing a second plant having a SRK encoding DNA sequence or gene within its genome;
(c) crossing the first and second plants to obtain a $F_1$ generation;
(d) and selecting those plants from the $F_1$ generation which demonstrate an acquired self-incompatibility trait.

It is a further objective of the present invention to provide a method of altering the self-incompatibility phenotype of a recipient plant comprising:

($a_1$) isolating an SRK encoding DNA sequence or gene which encodes an S receptor kinase; and
($a_2$) optionally isolating an SLG encoding DNA sequence or gene which encodes an S-locus glycoprotein in the same genotype as that of the isolated SRK gene; and
(b) integrating the isolated SRK encoding DNA sequence or gene abd optionally the isolated SLG encoding DNA sequence or gene into the genome of a recipient plant that does not naturally produce a S receptor kinase produced by the isolated SRK gene whereby the recipient plant is incompatible with plants or pollen of the same genotype as that of the isolated SRK gene.

For the isolation of DNA molecules encoding a S Receptor Kinase (SRK) there are preferably used as starting material plants being homozygous for the self-incompatibility allels. Preferred in this respect are plants of the family *Brasicaceae* such as, for example, *B. oleracea* or *B. campestris*. Especially preferred as starting material for the isolation of SRK genes are homozygous self-incompatible donor plants having a genotype selected from the group consisting of *Brassica oleracea* S2, *Brassica oleracea* S6, *Brassica oleracea* S13, *Brassica oleracea* S14, *Brassica oleracea* S22, and *Brassica campestris* S8.

For the isolation of DNA molecules encoding a S Receptor Kinase (SRK) genomic or cDNA gene libraries may first be established. These libraries can be created by customary routine methods very well known to the person skilled in that field. The basic methods of creating genomic or cDNA gene libraries are described in detail, for example, in Maniatis *et al* (1982), while the transfer and application of those methods to plant systems are described, for example, in Mohnen *et al* (1985). The preparation of the cDNA library from poly($A^+$) RNA isolated from stigmas of $S_6S_6$ homozygous plants as described in detail in the following examples, is reported in Narallah *et al*, 1987. Genomic DNA and cDNA can be obtained in various ways. Genomic DNA, for example, can, using known methods, be extracted from suitable plant material such as, for example healthy leaf tissue and purified. For the production of cDNA, there is generally used as starting material mRNA, which can be isolated from selected cells or tissues, but especially from cells or tissues that are known or at least supposed to have high concentrations of SRK encoding DNA sequences. The isolated mRNA can then be used in a reverse transcription as the matrix for the production of a corresponding cDNA.

The methods of isolating poly($A^+$) RNA and of producing cDNA are known to the person skilled in the art.

The extracted and purified DNA preparations are then cleaved into fragments for the subsequent cloning. The genomic DNA or cDNA to be cloned may be fragmented to a size suitable for insertion into a cloning vector either by mechanical shearing or, preferably, by cleavage with suitable restriction enzymes. Suitable cloning vectors which are already being used as a matter of routine for the creation of genomic and/or cDNA gene libraries include, for example, phage vectors, such as the $\lambda$ Charon phages, or bacterial vectors, such as the *E.* coli plasmid pBR322. Further suitable cloning vectors are known to the person skilled in the art and/or can be obtained commercially such as, for example, in form of the GIGAPACK kit, which is available from STRATAGENE.

From the gene libraries created in the manner as described hereinbefore, suitable clones containing the SRK gene or parts thereof can then be identified in a screening programme, for example with the aid of suitable oligonucleotide probes (probe molecule), and then isolated. Various methods are available for identifying suitable clones, for example differential colony hybridisation or plaque hybridisation. Immunological detection methods based on identification of the specific translation products may also be used.

As probe molecule there may be used, for example, a DNA fragment that has already been isolated beforehand from a structurally related gene such as, for example, the SLG gene and that is capable of hybridisation with the corresponding section of sequence within the SRK encoding gene according to the invention.

Provided that the amino acid sequence of the gene to be isolated or at least parts of that sequence are known, a corresponding DNA sequence can be drawn up on the basis of that sequence information. Since the genetic code is known to be degenerate, different codons can in the majority of cases be used for one and the same amino acid. As a result, apart from a few exceptional cases, a particular amino acid sequence can as a rule be coded for by a whole series of oligonucleotides that are similar to one another. However, care must be taken to ensure that only one member of that series of oligonucleotides actually corresponds with the corresponding sequence within the gene that is being sought. In order to limit from the outset the number of possible oligonucleotides, the rules on the use of codons laid down by Lathe R *et al*, 1985, which take account of the frequency with which a particular codon is actually used in eukaryotic cells, may, for example, be applied.

On the basis of that information it is thus possible to draw up oligonucleotide molecules that can be used as probe molecules for the identification and isolation of suitable clones by hybridising the said probe molecules with genomic DNA or cDNA in one of the methods described above.

In order to facilitate detection of the desired DNA sequence or gene coding for a SRK polypeptide, the above-described DNA probe molecule can be labelled with a suitable readily detectable group. Within the scope of this invention, a detectable group is to be understood as being any material having a particular readily identifiable physical or chemical property. Such materials are already widely used especially in the field of immunoassays, and the majority of them may also be employed in the present application. Special mention may be made at this point of enzymatically active groups, for example enzymes, enzyme substrates, coenzymes and enzyme inhibitors, and also of fluorescent and luminescent agents, chromophores and radioisotopes, for example $^{3}H$, $^{35}S$, $^{32}P$, $^{125}I$ and $^{14}C$. The ready detectability of those labels is based on the one hand on their inherent physical properties (e.g. fluorescent labels, chromophores, radioisotopes) and on the other hand on their reaction and binding properties (e.g. enzymes, substrates, coenzymes, inhibitors).

Also suitable as a probe molecule is a single-strand cDNA derived from a poly(A)+ RNA, which in turn is isolated from a tissue or a cell of a suitable plant. Especially preferred within the scope of this invention is a $^{32}P$-labelled $SLG_6$ cDNA insert from plasmid pBOS6, described by Nasrallah *et al* [Nature 326: 6113, 1987]. General methods relating to hybridisation are described, for example, in Maniatis T *et al*, 1982 and in Haymes BT *et al*, 1985.

Those clones within the above-described gene libraries which are capable of hybridisation with a probe molecule and which can be identified by means of one of the above-mentioned detection methods can then be analysed further in order to determine in detail the extent and nature of the sequence coding for a SRK polypeptide.

An alternative method of cloning DNA molecules encoding a S Receptor Kinase is based on the creation of a gene library composed of expression vectors. In that method, analogously to the methods already described above, genomic DNA, but preferably cDNA, is first isolated from a cell or a tissue capable of expressing a SRK polypeptide and is then spliced into a suitable expression vector. The gene libraries so created can then be screened using suitable measures, preferably using anti-SRK antibodies, and those clones selected which contain the desired gene or at least part of that gene as an insert.

Using the methods described above it is thus possible to isolate a DNA molecule encoding a S Receptor Kinase from a plant being homozygous for the self-incompatibility allels, but especially a $SRK_6$ or $SRK_2$ gene from a respective *Brassica* plant, said SRK gene comprising an S-locus binding domain, a transmembrane domain, and a protein kinase domain. For further characterisation of the SRK genes, the DNA sequences purified and isolated as described above are subjected to sequence analysis. The previously isolated chitinase gene is first cleaved into fragments by means of suitable restriction enzymes and then cloned in suitable cloning vectors, for example the M13 vectors mp18 and mp19. The sequencing is carried out in the $5' \rightarrow 3'$ direction, the dideoxynucleotide chain termination method according to Sanger *et al*, 1977 or the method according to Maxam and Gilbert, 1980 preferably being used. In order to avoid errors in sequencing, it is advantageous to sequence the two DNA strands in parallel. The analysis of the nucleotide sequence and of the corresponding amino acid sequence is advantageously computer assisted using suitable computer software such as, for example, the IBI Pustell and GCG version 6.0 (Univ. of Wisconsin Biotechnology Center) software packages.

Taking measures as hereinbefore described one will be able to identify and isolate SRK encoding DNA sequences such as, for example, the $SRK_6$ and $SRK_2$ encoding DNA sequences or genes, which are preferred within this invention. As described in greater detail in the following examples, the structure of $SRK_6$ can be determined by sequencing both strands of a 4.8 kb genomic fragment and 3 overlapping $S_6S_6$ stigma cDNA clones, pS6-27 (1105 bp), pS6-32 (686 bp), which clones can be isolated from the $S_6S_6$ cDNA library by screen-

ing with the kinase-domain probe of the $SRK_6$ gene, and pJS30 (1566 bp) (Fig. 2A), which clone can be isolated following amplification by the polymerase chain reaction (PCR) of $S_6S_6$ stigma cDNA using, for example, the mRNA GeneAmp kit (Perkin-Elmer/Cetus).

Comparison of the genomic and cDNA sequences revealed 7 exons interspaced with 6 introns ranging in size from 76 bp (intron 3) to 898 bp (intron 1). The coding region (Fig. 2B) has a single open reading frame that predicts a protein of 857 amino acids. Two alternative polyadenylation sites generate a 3′ noncoding region of either 192 bp or 261 bp.

The predicted $SRK_6$ polypeptide (98,071 daltons) consists of several domains. Each domain is encoded by separate exons or groups of exons, consistent with the view that multi-domain proteins evolved by exon-shuffling. The 437 amino acid region encoded by exon 1 defines the "S" domain" which is highly similar to $SLG_6$. The hydrophobic residues (-32 to -1) at the N-terminus constitute a putative signal peptide that is 67 % identical to the signal sequence of $SLG_6$ (Figs. 2B & 3). The rest of the "S" domain (residues 1-406) is 89 % identical to $SLG_6$, and contains a cluster of 12 cysteine residues, a hallmark of the S-multigene fatty. Exon 2 encodes a 20 amino acid hydrophobic stretch (residues 415-434) that is likely to form a membrane spanning helix. As is typical of transmembrane domains, 6 of the 8 amino acids that follow this region are basic. The remaining sequence, encoded by exons 3-7, contains a putative protein kinase catalytic domain (residues 489-781) that is flanked by a 55 amino acid juxtamembrane domain and a 44 amino acid carboxy-terminus domain. A diagnostic feature of protein kinases is the presence of 11 conserved subdomains wherein lie 15 invariant or nearly invariant amino acid residues. These subdomains as well as all 15 conserved residues were identified in the predicted protein sequence of $SRK_6$ (Figs. 2B & 3). The substrate specificity of protein kinases, either serine/threonine or tyrosine, generally correlates with consensus sequences within subdomains VI and VII. The $SRK_6$ sequence in subdomains VI (Asp-Leu-Lys-Val-Ser-Asn) and VIII (Gly-Thr-Tyr-Gly-Tyr-Met-Ser-Pro-Glu) agrees strongly with the consensus for the serine/threomine kinases. A database search with the $SRK_6$ polypeptide sequence revealed greatest similarity, after SLG, to maize ZmPK1, showing conservation with both the "S" domain (29 % identity) and the kinase domain (36 % identity). Significant similarity was also found with the catalytic domains of other protein kinases, particularly those of tyrosine kinases and the raf serine/threonine kinase.

These results suggest that SRK6 encodes an integral membrane protein. The "S" domain plus 9 residues encoded by exon 2 would be located extracellularly, and the putative kinase domain and flanking regions would be oriented toward the cytoplasm. Or 10 potential N-glycosylation sites (N-X-S/T), 7 would occur extracellularly (Fig. 2B), and 5 of these are conserved with the 9 sites in $SLG_6$. This structure is similar to receptor tyrosine kinases, which have a glycosylated extracellular domain responsible for ligand-binding, a single-pass transmembrane domain, and an intracellular tyrosine kinase catalytic domain. The existence of receptors with possible serine/threonine kinase specificity has only recently been suggested by the description of ZmpK1, the *Caenorhabditis elagans* daf-1 gene, and the SRK gene reported here. However, sequence-based predictions of kinase substrate specificity can be misleading, and only direct biochemical evidence will determine whether these genes represent a new class of receptor kinases.

A further preferred embodiment of the present invention comprises an essentially pure DNA molecule comprising a DNA sequence encoding a $SRK_2$ polypeptide, which DNA molecule can be obtained using the method according to the invention. In particular, in screening the genomic DNA library referred to hereinbefore with a pair of S locus-linked genes isolated previously from the $S_2$ homozygote genotype and designated SLG-2A and SLG-2B [Chen *et al*, 1990] one will be able to identify a clone comprising a DNA molecule encoding a $SRK_2$ polypeptide.

More specifically, as set forth in the above Chen *et al* publication, the DNA sequence of both of these genomic EcoRI fragments are described in the following sequence alignment map, in which identical nucleotides are indicated by "."; two gaps "--" are introduced to optimize alignment between the two sequences:

```
SLG-2A ATGAAAGGGGTACAGAACATTTACCACCATTCTTACACCTTCTCGTTCTT
SLG-2B ..................................................

SLG-2A GCTAGTCTTCCTTGTCTTGATTCTATTTCATCCTGCCCTTTCGATCTATG
SLG-2B ..................................................

SLG-2A TCAACACTTTATCGTCTTCAGAGTCTCTCACAATCTCGAGCAATAGAACA
SLG-2B ..................................................
```

```
SLG-2A CTTGTATCTCCCGGTGGAGTCTTCGAGCTTGGTTTCTTCAAACCCTTGGG
SLG-2B ..................................................

SLG-2A ACGCTCGCAATGGTATCTCGGAATATGGTATAAGAAAGTCTCCCAGAAAA
SLG-2B .......G.........G...............A....C.C..TG.....

SLG-2A CCTACGCATGGGTCGCAAACAGAGACAACCCTCTCACTAATTCCATTGGA
SLG-2B ................C..................T.C.G...T......

SLG-2A ACTCTCAAAATCTCTGGCAACAATCTTGTCCTGCTAGGTCAGTCTAATAA
SLG-2B ..C.................................A.........C...

SLG-2A CACTGTTTGGTCGACAAATCTTACTAGAGAAAATGTGAGATCACCAGTGA
SLG-2B .............................G.....C......T..G....

SLG-2A TAGCAGAGCTTCTTCCCAACGGTAATTTTGTAATGAGATACTCCAGCAAC
SLG-2B ..................................A...C......A....

SLG-2A AAAGACTCAAGTGGATTCTTGTGGCAGAGTTTCGATTTTCCGACAGATAC
SLG-2B ..................................................

SLG-2A TTTACTTCCGGATATGAAACTAGGTTACGATCTCAAAACAGGGCGTAACA
SLG-2B ...........G.................................C....

SLG-2A GAATTCTTACATCATGGAGAAGTTCAGATGATCCGTCAAGCGGAATACC
SLG-2B .GT.C........G...A.G...........................TT.

SLG-2A ACGTACAAAATCGACACTCAAAGGGGATTGCCAGAGTTTATTCTTA---A
SLG-2B GT.......C......T..G............T.............TAA.

SLG-2A TCAAG---------GACGTTATGAAATGCAAAGGAGCGGTCCGTGGAATG
SLG-2B ....TTTTTGAATCA....GT.....C...............T.......

SLG-2A GAATGGAGTTTAGTGGCATACCGGAGGTGCAAGGATTAAATTACATGGTT
SLG-2B ...............................G..................
```

```
SLG-2A TACAATTATACGGAGAACAGTGAGGAGATCTCTTACACGTTCCATATGAC
SLG-2B ..............................G.....T.............

SLG-2A CAACCAAAGCATCTACTCCAGATTGACAGTCAGTGACTATACACTTAATC
SLG-2B ....................................G.TG.....CG...

SLG-2A GATTGACGTGGATCCCGCCATCACGGGCATGGAGCATGTTCTGGACTTTA
SLG-2B ...........................AT......C.C............

SLG-2A CCGACGGACGTGTGCGATCCACTTTACTTATGTGGATCTTATTCTTACTG
SLG-2B ..A.................G.............................

SLG-2A TGACCTAATTACGTCACCTAACTGTAACTGTATTAGAGGGTTCGTTCCCA
SLG-2B ..................................................

SLG-2A AGAACCCGCAGCAGTGGGACTTGAGAGACGGAACACAGGGGTGTGTGAGG
SLG-2B ....................................G.............

SLG-2A ACGACGCAGATGAGCTGTAGTGGAGATGGCTTTTTGCGGCTAAACAATAT
SLG-2B .............................G....................

SLG-2A GAATTTGCCGGATACTAAGACGGCGACTGTGGATCGGATAATTGATGTGA
SLG-2B ........................A.............C...G.......

SLG-2A AAAAATGCGAAGAGAGGTGTCTAAGTGATTGTAACTGTACGTCGTTTGCT
SLG-2B ......G...............T..C..............T.........

SLG-2A ATTGCGGATGTTCGAAACGGCGAATTGGGTTGTGTGTTTTGGACCGGAGA
SLG-2B ..............G..T..A.G...........................

SLG-2A GCTCGTTGAGATCAGGAAATTTGCCGTCGGTGGTCAAGATCTTTACGTCA
SLG-2B ........C............C............................

SLG-2A GGTTGAATGCTGCTGATCTAGGTTAG
SLG-2B .A........................
```

The predicted amino acid sequences of SLG-2A, SLG-2B, and SLG-13 are shown in the alignment map provided in the following, in which amino acid residues (single letter codes) that are identical between SLG-13 and SLG-2A are boxed "=" indicates residues conserved between SLG-2A and SLG-2B; "*" represent spaces introduced for optimal alignment; potential glycosylation sites found in SLG-2A, SLG-13 and SLG-2B are indicated by open circles; and arrowheads delineate the interspersed A, B, C, D conserved and variable domains.

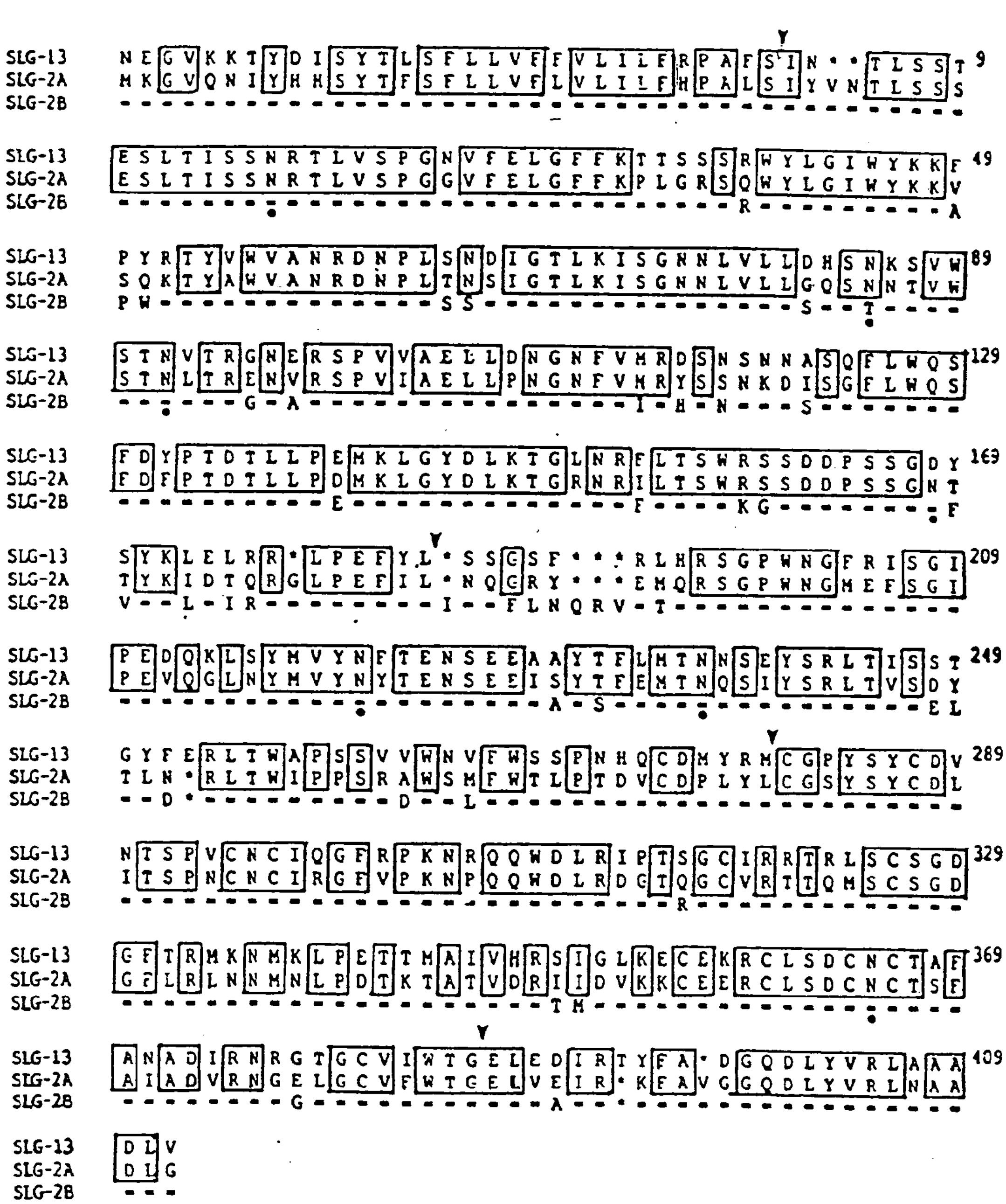

The isolation of the cDNA clones corresponding to SLG-2A had identified this gene as the $S_2$ homologue of $SLG_6$, and this gene is therefore referred to as $SLG_2$. In a preferred embodiment of the present invention, a 7.2 kb fragment of the genomic clone is isolated using the SLG-2B sequence as a probe and sequenced. In analogy to the $SLG_2$ gene referred to above, the complete gene of which SLG-2B represents the S-domain, is designated as $SRK_2$, the homologue of $SRK_6$ in plants with the S2 recognition genotype.

Of the 7 $SRK_2$ exons, only the first is reported in the prior publication. The location of the remaining 6 exons begins ≈ 2.2 kb downstream of the first. Strikingly, both the $SRK_6$ and $SRK_2$ genomic sequences have an in-frame stop codon (TAG) within intron 1, position 2 nucleotides downstream of the 5′ splice site. The placement of this stop codon relative to the "S" domain of SRK exactly matches the position of the stop codon used by SLG (Fig. 4A), suggesting that it has functional relevance. For example, the stop codon could be retained in alternative transcripts of SRK, causing the production of a truncated SLG-like protein consisting of only the "S" domain.

The predicted 855 amino acid SRK2 polypeptide sequence (Fig. 3) contains all of the structural features predicted for SRK6, however $SRK_2$ and $SRK_6$ are only 68 % conserved. The most highly conserved domains are the "S" (67 %) and kinase (71 %) domains (Fig. 4B). This level of divergence is surprisingly high for allelic variants, yet it parallels the polymorphism observed between alleles of SLG, since the SLG6 and SLG2 protein sequences are 68 % identical. In contrast, each SRK "S" domain is more similar to its corresponding SLG allele (≈90 % amino acid identity; Fig. 4B) thus, SRK and SLG are structurally related genes pairs. As a unit, they have apparently evolved in concert within each S genotype, while diverging extensively between genotypes.

As described in the following examples, to study the expression of the $SRK_6$ gene, poly(A) RNA from pistils, anthers, leaves, cotyledons and roots are subjected to gel blot analysis using separate probes derived from the kinase domain, the "S" domain, and the first intron of $SRK_6$ (see Fig. 2A). The most intense hybridization signals are obtained in mature pistils where transcripts ranged in size from 1.6 kb to 5.9 kb (Fig. 5A). Some of these transcript species are assigned to genes other than $SRK_6$. In particular, the highly abundant "S"-homologous 1.6 kb transcript was previously shown to be derived from the SLG gene in experiments with transgenic tobacco. Only the transcript species of 3.0 kb (arrowhead), 4.1 kb (asterisk) and 2.3 kb (circle) are unambiguously assigned to the $SRK_6$ gene, and their characterization is described in detail.

The levels of fully spliced and unspliced $SRK_6$ transcripts are regulated in anthers during the rapid succession of pollen development stages (Fig. 5B). Maximal expression of $SRK_6$ is observed in anthers at the binucleate microspore stage. At the uninucleate stage there are low levels of transcript, and by the trinucleate stage no expression is detected. This regulation of message quantity cannot be attributed to general variation of RNA levels since RNA from all stages contained equal amount of actin transcript (Fig. 5B).

In order to verify the origin of these transcripts, the $SRK_6$ gene is introduced into a recipient *B. oleracea* strain that carries the $S_2$ allele. The results of the transformation experiments clearly show that upon transformation into a suitable recipient plant, the SRK gene can be specifically transcribed in pistils and anthers. However, SRK transcripts are substantially less abundant in mature pistils than SLG transcripts, since they produced a 140-180 fold weaker hybridization signal with the "S"-domain probe.

Within the scope of the present invention several lines of evidence are providedt that imply that SRK functions in the mechanism of SI.

First, SRK is located at the S locus, as is independently demonstrated for two different SRK alleles.

Second, the predicted products of these SRK alleles show the high degree of polymorphism expected for proteins responsible for cellular recognition specificity. Finally, SRK transcription is active in the male and female reproductive organs, from which are derived the two interacting cell types that mediate recognition. A possible model for this recognition might involve the allele-specific binding of a putative ligand which might also be encoded at the S locus. Alternatively, SRK and SLG might display allele specific homophilic binding. The concerted evolution of the SLG/SRK gene pair suggests that their products interact functionally, perhaps as components of a ligand binding complex or as unassociated competitors for this binding. Moreover, by phosphorylating intracellular substrates, SRK might couple these initial molecular recognition events to the signal transduction chain that lead to pollen acceptance or rejection. Preliminary evidence for the existence of multiple SRK-like genes, some of which may be transcribed in leaves of *Brassica*, and the finding that ZmPK1 is transcribed in vegetative tissues of maize, suggests that SRK is a specialized member of a universal class of cell-cell communication receptors that function in higher plants.

Based upon the data provided in this description, it is clear that SLG and SRK are tightly linked genetically and that they reside at the S locus. This conclusion is clear because it is possible to isolate $SRK_6$, the SRK gene derived from a *B. oleracea* $S_6$ homozygote, from a collection of SLG-homologous genomic clones. Further evidence in this instance is provided by the detection of genetic segregation of RFLP markers. A perfect correlation is observed between the presence of the SLG6 allele, the presence of the SRK6 allele, and the S6 incompatibility specificity as determined by pollination assays.

The SRK genes may be transformed into appropriate plants either alone or together with SLG genes. This may be done by placing the SRK gene on a single vector, or when transformation together with an SLG gene is desired, by placing the SRK and SLG genes on separate vectors or physically linking the genes on a single vector. In addition, separate plants may be transformed with either a single SLG or SRK gene, and these plants may then be crossed with conventional techniques to produce an $F_1$ generation plant containing both sequences within its genome.

The plants which are the product of transformation with the SRK and/or SRK-SLG gene according to the present invention may be incompatible with either pollen of the same genotype as that of the isolated SRK gene or the pollen of the transformed plant may then be rendered incompatible with plants of the same genotype as that of the SRK gene.

A representative scheme for carrying out transformation experiments using constructs comprising DNA sequences or genes encoding SRK either alone or together with DNA sequences or genes encoding SLG for imparting self-incompatability to the transformed plant material may look as follows:

- Isolation of SRK genes from two class I S-genotypes [$S_{13}$, $S_{14}$ and $S_{22}$ genotypes of *B. oleracea* and the $S_8$ genotype of *B. campestris*].
- Transformation of *B. napus*:
- Transformation with two different class I SRK genes.

- Transformation with the previously isolated SLG and SRK genes from the B. *oleracea* $S_2$ (class II) genotype.
- Selection of transgenic plants with high expression of the SRK trnsgene for class I genes, and with high expression of the SLG and SRK transgenes for class II genes.
- The establishment of transgenic lines carrying an SRK/SLG gene pair by crossing SRK transformants to previously generated SLG transformants.
- Evaluation of self-incompatibility phenotype in progeny plants.

To become expressed in the plant cell the SRK and SLG encoding DNA sequences are preferably cloned into a suitable vector for plant transforamtion which preferably comprise one or more promoter or regulatory DNA sequences to promote tissue specific expression of the coding DNA sequence in the transformed plant cell. Most preferably the complete SRK6 and SLG gene sequences are used for transformation which sequences already comprise all regulatory sequences necessary for tissue specific expression of the respective genes.

The transformation of the plant material can be carried out in a number of ways that are well known to those of skill in the art. For example, methods of transforming plant cells include microinjection [Crossway *et al* (1986); Neuhaus (1987)], electroporation [Riggs *et al* (1986)], *Agrobacterium* mediated transformation (Hinchee *et al* (1988)], direct gene transfer (Paszkowski *et al*, (1984)], and ballistic particle acceleration using, for example, devices available from Agracetus, Inc., Madison, Wisconsin and Dupont, Inc., Wilmington, Delaware [see, for example, Sanford *et al*, U.S. Patent 4,945,050; and McCabe *et al*, (1988)].

Preferred in the present invention is a method for introducing genetic material into plant cells which comprises, for example, bringing plant cells into contact with *Agrobacterium* comprising the DNA to be introduced. This may be achieved by infecting sensitive plant cells or by co-cultivating protoplasts derived from plant cells.

Transformation of the plant cells includes separating transformed cells from those that have not been transformed. One convenient method for such separation or selection is to incorporate into the material to be inserted into the transformed cell a gene for a selection marker. As a result only those cells that have been successfully transformed will contain the marker gene. The translation product of the marker gene will then confer a phenotypic trait that will make selection possible. Usually the phenotypic trait is the ability to survive in the presence of some chemical agent, such as an antibiotic, e.g., kanamycin, G418, paromomycin, etc., which is placed in a selection media.

Some examples of genes that confer antibiotic resistance include, for example, those coding for neomycin phosphotransferase [kanamycin resistance, Velten *et al* (1984)]; hygromycin phosphotransferase [hygromycin resistance, van den Elzen *et al*., (1985)], the kanamycin resistance (NPT II) gene derived from Tn5 [Bevan *et al* (1983); McBride *et al* (1990)), the PAT gene described in Thompson *et al* (1987), and chloramephenicol acetyltransferase.

An example of a gene useful primarily as a screenable marker in tissue culture for identification of plant cells containing genetically engineered vectors is a gene that encodes an enzyme producing a chromogenic product. One example is the gene encoding for production of beta-glucuronidase (GUS). This enzyme is widely used and its preparation and use is described in Jefferson (1987).

Once the transformed plant cells have been cultured on the selection media, surviving cells are selected for further study and manipulation. Selection methods and materials are well known to those of skill in the art, allowing one to choose surviving cells with a high degree of predictability that the chosen cells will have been successfully transformed with exogenous DNA.

Especially preferred within the scope of this invention for introducing DNA into a plant cell by means of *Agrobacterium* is the so-called disk transformation using *Agrobacterium* [Horsch *et al* (1985)]. Sterile disks from a suitable target plant, preferably sterile flowering stem disks of, for example, *B. oleracea* or *B. napus*, are incubated with *Agrobacterium* cells comprising one of the SRK and/or SLG encoding DNA sequences according to the invention, and are then transferred into or onto a suitable nutrient medium. Especially suitable, and therefore preferred within the scope of this invention, are LS media that have been solidified by the addition of agar and enriched with one or more of the plant growth regulators customarily used, especially those selected from the group of the auxins consisting of a-naphthylacetic acid, picloram, 2,4,5-trichlorophenoxyacetic acid, 2,4-dichlorophenoxyacetic acid, indole-3-butyric acid, indole-3-lactic acid, indole-3-succinic acid, indole-3-acetic acid and p-chlorophenoxyacetic acid, and from the group of the cytokinins consisting of kinetin, 6-benzyladenine, 2-isopentenyladenine and zeatin. The preferred concentration of auxins and cytokinins is in the range of from 0.1 mg/l to 10 mg/l.

After incubation for several days, but preferably after incubation for 2 to 3 days at a temperature of from 20°C to 40°C, preferably from 23°C to 35°C and more especially at 25°C and in diffuse light, the stem disks are transferred to a suitable medium for the purpose of shoot induction. Especially preferred for the selection of the transformants is an LS medium that does not contain auxin but contains cytokinin instead, and to which a selective substance has been added dependent on the marker gene used. The cultures are kept in the light and are transferred to fresh medium at suitable intervals, but preferably at intervals of one week. Developing green shoots are cut out and cultured further in a medium that induces the shoots to form roots. Especially preferred within the scope of this invention is an LS medium that does not contain auxin or cytokinin but to which a selective substance has been added for the selection of the transformants.

In addition to the methods for identifying additional SRK genes using SLG clones to identify and isolate SRK genes described herein, the known homology among SLG genes may also provide other SLG probes for isolating the SRK gene, and these methods using modified but closely related probes are deemed to be within the scope of the present invention. Furthermore, additional techniques may be used for obtaining SRK genes. One such technique described in Nasrallah *et al*, 1987 reports the cloning of a cDNA encoding a SLG protein from stigmas of self-incompatible plants.

Thus, while having illustrated and described the preferred embodiments of the present invention, it is to be understood that this invention is capable of variation and modification and is therefore not considered to be limited to the precise terms set forth, but is intended to encompass all such changes and alterations which may be made for adapting the invention to various usages and conditions. Accordingly, such changes and alterations are properly intended to be within the full range of equivalents, and therefore within the purview of the following claims.

Accordingly, while the following examples illustrate the present invention with respect to the $SRK_6$ and $SLG_6$ genes, the present invention can also be practiced by one skilled in the art with other self-incompatible plants wherein self-incompatibility is determined by an S-locus, which preferably includes both an SLG and an SRK gene. For example, the present invention can be readily practiced with respect to plants utilizing genes isolated from the $S_2$, $S_6$, $S_{13}$, $S_{16}$ and $S_{22}$ alleles of *Brassica* and related genera.

As many of the recombinant DNA techniques employed in this invention are a matter of routine for the person skilled in the art, it is better to give a short description of these generally used techniques here rather than to describe them every time they occur. Except where there is a specific indication to the contrary, all these procedures are described in the Maniatis et al (1982) reference.

A. Cleaving with restriction endonucleases:

A reaction batch typically contains about 50 to 500 μg/ml of DNA in the buffer solution recommended by the manufacturer, New England Biolabs, Beverly, MA. 2 to 5 units of restriction endonucleases are added for each g of DNA and the reaction batch is incubated for from one to three hows at the temperature recommended by the manufacturer. The reaction is terminated by heating at 65íC for 10 minutes or by extraction with phenol, followed by precipitation of the DNA with ethanol. This technique is also described on pages 104 to 106 of the Maniatis *et al* reference.

B. Treatment of the DNA with polymerase in order to produce blunt ends:

50 to 500 μg/ml of DNA fragments are added to a reaction batch in the buffer recommended by the manufacturer (New England Biolabs). The reaction batch contains all four deoxynucleotide triphosphates in concentrations of 0.2 mM. The reaction takes place over a period of 30 minutes at 15°C and is then terminated by heating at 65°C for 10 minutes. For fragments obtained by cleaving with restriction endonucleases that produce 5′-projecting ends, such as EcoRI and BamHI, the large or Klenow fragment of DNA polymerase is used. For fragments obtained by means of endonucleases that produce 3′-projecting ends, such as PstI and SacI, the T4-DNA polymerase is used. The use of these two enzymes is described on pages 113 to 121 of the Maniatis *et al* reference.

C. Agarose gel electrophoresis and purification of DNA fragments from gels:

Agarose gel electrophoresis is carried out in a horizontal apparatus, as described on pages 150 to 163 of the Maniatis reference. The buffer used is the Tris-borate buffer described therein. The DNA fragments are stained using 0.5 mg/ml of ethidium bromide which is either present in the gel or tank buffer during electrophoresis or is added after electrophoresis. The DNA is made visible by illumination with long-wave ultraviolet light. The preferred wave-length is approximately 300 to 310 nm. Especially preferred is a wave-length of 302 nm. If the fragments are to be separated from the gel, an agarose is used that gels at low temperature and is obtainable from Sigma Chemical, St. Louis, Missouri. After the electrophoresis the desired fragment is cut out, placed in a plastics test tube, heated at 65°C for about 15 minutes, extracted three times with phenol and precipitated twice with ethanol. As an alternative, the DNA can be isolated from the agarose with the aid of the Geneclean kit (Bio 101 Inc., La Jolla, CA).

D. Addition of synthetic linker fragments to DNA ends:

If it is desired to add a new endonuclease cleavage site to the end of a DNA molecule, the molecule is optionally first treated with DNA-polymerase in order to produce blunt ends, as described in the above section. About 0.1 to 1.0 μg of this fragment is added to about 10 ng of phosphorylated linker DNA (New England Biolabs) in a volume of 20 to 30 μl with 2 μl of T4 DNA ligase (New England Biolabs), and 1 mM ATP in the buffer recommended by the manufacturer. After incubation overnight at 15°C, the reaction is terminated by heating at 65°C for 10 minutes. The reaction batch is diluted to about 100 μl in a buffer appropriate for the restriction endonuclease that cleaves the synthetic linker sequence. About 50 to 200 units of this endonuclease are added. The mixture is incubated for 2 to 6 hours at the appropriate temperature, then the fragment is subjected to agarose gel electrophoresis and purified as described above. The resulting fragment will then have ends with endings that were produced by cleaving with the restriction endonuclease. These ends are usually cohesive, so that the resulting fragment can then readily be linked to other fragments having the same cohesive ends.

E. Removal of 5′-terminal phosphates from DNA fragments:

During the plasmid cloning steps, the treatment of the vector plasmid with phosphatase reduces the recircularisation of the vector (discussed on page 13 of Maniatis). After cleavage of the DNA with the correct restriction endonuclease, one unit of calf intestinal alkaline phosphatase (Boehringer-Mannheim) is added. The DNA is incubated at 37°C for one hour and then extracted twice with phenol and precipitated with ethanol.

F. Linking of the DNA fragments:

If fragments having complementary cohesive ends are to be linked to one another, about 100 ng of each fragment are incubated in a reaction mixture of 20 to 40 μl containing about 0.2 unit of T4 DNA ligase (New England Biolabs) in buffer recommended by the manufacturer. Incubation is carried out for 1 to 20 hows at 15°C. If DNA fragments having blunt ends are to be linked, they are incubated as above except that the amount of T4 DNA ligase is increased to 2 to 4 units.

G. Transformation of DNA into *E. coli*:

*E. coli* strain HB101 is used for most of the experiments. DNA is introduced into E. coli using the calcium chloride possess, as described by Maniatis *et al* on pages 250 and 251.

H. Screening of *E. coli* for plasmids:

After transformation, the resulting colonies of *E. coli* are tested for the presence of the desired plasmid by means of a rapid plasmid isolation possess. Two customary processes are described on pages 366 to 369 of the Maniatis *et al* reference.

I. Large-scale isolation of plasmid DNA:

Processes for the isolation of plasmids from *E. coli* on a large scale are described on pages 88 to 94 of the Maniatis *et al* reference.

J. Cloning in M13 phage vectors:

In the following description it is to be understood that the double-strand replicative form of the phage M13 derivatives is used for routine processes, such as cleaving with restriction endonuclease, linking etc.

Unless there is a specific indication to the contrary, enzymes can be obtained from either Boehringer or Biolabs (BRL), and are used in accordance with the manufacturer's instructions unless otherwise indicated.

K. Southern blot analysis:

The extracted DNA is first treated with restriction enzymes, then subjected to electrophoresis in a 0.8 % to 1 % agarose gel, transferred to a nitrocellulose membrane and hybridized with the DNA to be detected which has previously been subjected to nicktranslation (DNA-specific activities of 5 x 108 to 10 x 108 c.p.m./μg). The filters are washed three times for 1 hour each time with an aqueous solution of 0.03M sodium citrate and 0.3M sodium chloride at 65°C. The hybridized DNA is made visible by blackening an X-ray film over a period of 24 to 48 hours.

L. Western blot analysis:

After SDS-polyacrylamide gel electrophoresis, the proteins are transferred electro-phoretically to a nitrocellulose or nylon filter. This filter is then pre-treated with a blocking agent (for example 5 % skim milk powder in PBS: in the following referred to as milk/PBS). The filter is then incubated for several hows with an antiserum that reacts with the compound to be detected. The filter pre-treated in this manner is washed several times with milk/PBS and then incubated with a commercially available secondary antibody that is coupled to an enzyme [for example peroxidase-coupled goat anti-rabbit antibody (BIORAD), 1:2000 diluted in milk/PBS]. The filter is again washed in PBS and then stained with chloronaphthol and hydrogen peroxide in accordance with the manufacturer's [BIORAD] instructions.

M. Northern blot analysis:

Total RNA can be prepared in accordance with the method described in Logemann et al, Analytical Biochemistry 163, 16-20 (1987), but it is advantageous to introduce an additional precipitation step with 2M LiCl in order to eliminate any residues of contaminant DNA. The RNA (0.4 mg or 4 mg) is then fractionated by electrophoresis in a 1 % agarose gel containing 6.7 % formaldehyde. The individual fractions are transferred to "Zeta-Probe' membranes [BIORAD] and hybridized with a 32P-labelled probe. The size of the hybridizing DNA can be estimated by reference to the standard run at the same time.

A more complete and thorough understanding of the present invention may be had by reference to the following figures and examples which are presented to exemplify, not limit, the present invention.

Figure 1 is a photomicrograph representing an restriction fragment length polymorphism (RFLP) analysis of an $F_2$ population segregating for the $S_6$ and $S_{14}$ self-incompatibility alleles;

Figure 2 is a physical map of the sequenced genomic fragment containing $SRK_6$ and flanking regions;

Figure 3 is the DNA sequence at the first exon/intron junction in $SRK_6$ and $SRK_2$ and the corresponding sequence in $SLG_6$;

Figure 4 depicts a concerted evolution of the SLG/SRK gene pair within S genotypes and their divergence between genotypes; and

Figure 5 is a photomicrograph depicting the expression of the $SRK_6$ gene.

Figure 6 depicts a comparison of the $SRK_2$, $SRK_6$, $SLG_6$ and ZmPK1 genomes.

In specific reference to the figures, which contain graphic representations of the data collected in the subsequent examples, figure 1 depicts DNA which has been isolated from parental (P) plants homozygous for either the $S_6$ or $S_{14}$ allele and from nine $F_2$ progeny, digested with Eco R1, and analyzed by blot hybridization. The segregation patterns observed following hybridization with probe specific for SLG (figure 1A), and $SRK_6$ (figure 1B) are shown. Figure 1C indicates the incompatibility phenotype of each plant as determined by pollination tests. The incompatibility phenotype of the $F_2$ progeny derived from a cross between the $S_6$ and $S_{14}$ homozygotes was determined by crosses to tester homozygous strains and by diallel analysis. Pollen-free mature stigmas were pollinated, and after a period of 12-24 hours, the stigmas were excised and stained with decolorized aniline blue. The extent of pollen-tube development was examined by fluorescence microscopy using well known protocols described in Example VI.

Figure 2 is, as described above, a physical map of the sequenced genomic fragment containing $SRK_6$ and flanking regions. The exons in this map appear as boxes; the hatched box represents exon 1 and containing the "S" domain; the open box represents exon 2 and contains the putative transmembrane domain; and the stippled boxes representing the remaining 5 exons that contain the putative kinase domain. The extents of 3 cDNA clones are shown below the map, and the lines above the map indicate the 3 $SRK_6$-derived probes used in the making of the present invention. The various restriction endonuclease sites are indicated as follows: R, Eco RI; H, Hinc II; Xb, Xba I; B, Bcl I; Xh, Xho I: P, Pst I; and H3, Hind III.

In figure 4, a concerted evolution of the SLG/SRK gene pair within S genotypes and their divergence between genotypes is depicted. The predicted SLG and SRK proteins encoded by the $S_6$ and $S_2$ genotypes are represented schematically. The numbers indicate the % identity between putative domains; SP, signal peptide; S, "S" domain; TM, trans-membrane domain; JM, juxtamembrane domain; PK, protein kinase domain; and C, carboxy-terminus domain.

The photomicrograph of figure 5 describes the $SRK_6$-homologous transcripts in pistils (P), anthers (A), leaves (L), cotyledons (C) and roots (R) of *Brassica oleracea* $S_6S_6$. Each lane contained about 2 μg of poly(A) RNA, and the blot was hybridized sequentially with a $^{32}P$-labeled probe derived from the kinase domain, the first intron, and the "S" domain of $SRK_6$. Subsequent hybridization with the actin probe showed comparable amounts of RNA in each lane. The arrowhead, asterisk and circle in the figure 5 panels denote the SRK-encoded transcripts described in the text The migration of RNA molecular weight markers is shown to the left. Figure 5B was prepared from $SRK_6$-homologous transcripts in developing *Brassica* anthers. Each lane contained about 5 μg of poly(A) RNA isolated from anthers that contained uninucleate (A1), binucleate (A2), and trinucleate (A3) microspores. Stages were determined by DAPI staining of nuclei according to the protocols of Toriyama et al [see Dev. Biol.143:427 (1991)]. The blot was first hybridized with a $^{32}P$-labeled probe derived from the kinase domain of $SRK_6$, and subsequently with an actin probe to show equivalent amounts of RNA in all three lanes. Figure 5C demonstrates the expression of $SRK_6$ in transgenic *Brassica*. Poly(A) RNA from transgenic plants (T6 and T14), and a control untransformed plant (U) was hybridized with the $SRK_6$ "S"-domain probe. Approximately 10 times more anther (A) than pistil (P) RNA was analyzed. The differences in the intensities of the endogenous pistil 1.6 kb transcripts between plants reflect the differences in the amounts of RNA loaded in each lane.

Figure 6 represents a comparison of the $SRK_2$, $SRK_6$, $SLG_6$ and ZmPK1 genomes in single letter code (the $SRK_2$ sequence is given in the following description), and in the corresponding sequence in $SLG_6$. The arrowheads indicate the splice sites in the sequence. The dots represent nucleotides omitted for diagrammatic clarity. The SRK genes have an in-frame stop codon located 2 nucleotides downstream of the 5′ splice site of intron 1 which is removed in fully spliced transcripts. Transcripts of SLG are not spliced and therefore retain the stop codon at this position.

**EXAMPLE I: Plant material**

The *Brassica oleracea* plants used in the making of the present invention were inbred lines homozygous for the $S_2$, $S_6$ and $S_{14}$ self-incompatibility alleles. The $S_6$ homozygote belonged to the variety *acephala* (kale) and was derived from plant material initially obtained from the Gene Bank Facility at Wellesbourne. The $S_{14}$ homozygote was developed at Cornell University and belonged to the variety *capitata* (cabbage). Because the ability of stigmas to distinguish self-pollen occurs late in development, immature bud stigmas are self-compatible. By self-pollinating these compatible immature stigmas, these self-incompatible lines were able to be produced and maintained. The $S_2$ homozygote, as well as the two others mentioned, have been described in the literature [see Nasrallah *et al* (1987) and Chen *et al* (1990)].

**EXAMPLE II: Isolation of DNA From $S_6$Homozygote**

The $S_6$ homozygous plant is selected as a source of DNA for the present invention, and large quantities of genomic DNA are purified from the $S_6$ homozygous plant according to a procedure modified from Bingham et al [see Cell 25:693 (1981)].

Approximately 10 g of young, healthy leaf tissue is frozen in liquid nitrogen and ground by hand in a prechilled mortar and pestle. The ground powder is suspended in 30 ml of ice-cold nuclear isolation buffer comprising 10 mM Tris pH 7.4, 60 mM NaCl, 10 mM EDTA, 0.15 mM spermidine, 0.15 mM spermine, 0.5% (v/v) Triton X-100 and 958 ml water. The suspended mixture is homogenized at medium speed in a motor-driven teflon pestle and homogenizer up and down 10 times. To remove large debris, the homogenate is transferred to a 50 ml polypropylene tube and subjected to 1500 rpm centrifugation in a Beckman J-6B centrifuge for 30 seconds at 4°C. The supernatant is removed to a clean 50 ml tube and nuclei were pelleted by centrifugation at 7,000 rpm for 7 min. at 4°C in an SS34 rotor. After discarding the supernatant, the white nuclei-containing pellet is washed in 20 ml of the nuclear isolation buffer and repelleted by centrifugation as above. The resulting pellet is resuspended in 5 ml of the nuclear isolation buffer, followed by the addition of 1 ml 10% Sarkosyl and gently mixed several minutes on ice to lyse the nuclei; as the nuclei are lysed, the solution becomes quite viscous. CsCl (11.17 g) is dissolved into the lysed nuclei after which the volume is adjusted to 12 ml with additional nuclear isolation buffer [25 ml nuclear isolation buffer, 5 ml 10% sodium lauroyl sarcosinate (Sarkosyl [3.33 ml 10% stock + 6.67 ml water], 60.5 g CsCl, to 65 ml with nuclear isolation buffer): 10% Sarkosyl (5:1), and mixed at room temperature until the CsCl dissolved completely. Insoluble debris is removed following centrifugation at 2000 rpm for 10 min. at room temperature.

In order to visualize nucleic acids, 3.5 μl 10 mg/ml EtBr is added. The mixture is transferred to a 13 ml ultracentrifuge tube, sealed, and subjected to a centrifugation at 47,000 rpm at 15°C for 18 hours. This centrifugation created a CsCl density gradient within the tube causing the genomic DNA to form a distinct band that is free from the bulk of the macromolecules, such as RNA and protein. The DNA band is removed using a hypodermic needle and the CsCl centrifugation is repeated in order to further remove contaminants. Final purification of DNA is achieved after dialysis against several changes of TE buffer (10 mM Tris pH 8.0, 1 ml EDTA).

Approximately 200 μg of genomic DNA is purified in this way, as determined by spectrophotometry at 260 nm.

Utilizing the genomic DNA purified from the $S_6$ homozygous plant according to Example I, an $S_6$ genomic DNA library is constructed in a λ vector according to the following Example II.

**EXAMPLE III: Library Construction and Screening of $S_6$ DNA Cloning of $SRK_6$ Gene**

The preparation of of the cDNA library from poly($A^+$) RNA isolated from stigmas of $S_6S_6$ homozygous plants in the bacteriophage λgt10 vector is accomplished using standard methods, and is described, for example, in Nasrallah *et al*, 1987.

One hundred micrograms of the purified nuclear DNA made in accordance with Example II is partially digested utilizing known techniques with the restriction enzyme Sau 3A, and the fragments are size-fractionated by salt centrifugation on salt gradients according to the procedures of Liu *et al* [see Science 209:1348 (1980)]. DNA in the 10 to 20 kilobase fraction is ligated to Bam HI-digested EMBL4 DNA [described in: Frischauf AM *et al*, J Mol Biol 170, 827-842, 1983; available from Promega Corp., Woods Hollow Road, Madison, WI 53711-5399], and the ligated DNA is packaged in vitro using Gigapack extracts (Stratagene). The packaged bacteriophage library is used further to infect lawn cultures of *Escherichia coli* host Q359 [Kann J *et al*, Proc Natl Acad Sci, USA 77, 5172-5176, 1980], and incubated at 37°C. These conditions favor lysis of host cells and the formation of plaques, each of which are derived from an individual recombinant phage clone.

In order to determine which recombinant phage clones cry genomic regions corresponding to SLG-homologous genes, plaques are transferred to nitrocellulose membranes and hybridized with the $^{32}P$-labelled $SLG_6$-cDNA insert from pBOS6 described by Nasrallah *et al*, 1987 under conditions described by Maniatis, 1982. A total of $10^6$ recombinant phage are screened, and of these 77 hybridized to the probe. These SLG homologous clones are purified and their DNA isolated according to the method of Thomas and Davis, 1975.

Each cloned insert made in accordance with Example III is characterized by restriction mapping using a wide variety of enzymes; since each restriction enzyme cleaves DNA at specific sequences, unique regions within the nuclear genome have a characteristic distribution of restriction enzyme cleavage sites. These restriction maps are used to place the recombinants into groups of overlapping clones. Several distinct regions of the *Brassica* genome are identified in this manner. Of the 77 clones analyzed, nine overlapped one another and corresponded to the $SRK_6$ gene.

One of the homologous $SRK_6$ clones contained a 5.8 kb insert which is designated as 281. This insert is subcloned into the pUC13 vector according to the following example:

**EXAMPLE IV: Sequencing of the $SRK_6$ Gene**

Ten μl of purified phage DNA containing the 281 insert is digested with Eco RI and the released 5.8 kb insert is purified after submitting the digested DNA to 1% agarose gel electrophoresis. The purified 5.8 kb fragment is cloned into an Eco RI digested plasmid vector using T4 ligase. The resulting ligated mixture is transformed into competent *E. coli* DH5a cells [available from GIBCO-BRL] and plated onto LB-Amp media. Transformed cells harboring the recombinant 281-containing plasmid are selected and purified. This plasmid, p281, is used in all subsequent experiments. The plasmid has been deposited under the Budapest Treaty with the American Type Culture Collection, has been accepted and has received accession number 75036.

In order to sequence the insert of p281, the dideoxy method is utilized. All necessary components of the sequencing reactions, except for the template DNA being sequenced, are supplied by commercially available sequencing kits (Pharmacia). Overlapping subclones of 281 are constructed in order to obtain suitable template DNA for sequence reactions. This is accomplished by digesting p281 with enzymes corresponding to sites within 281. The following restriction enzyme sites are utilized: Bgl II, Hinc II, Xba I, Xho I, Pst I, Sal I, Asu II, and Hind III. The resulting overlapping fragments generated by these enzymes are individually subcloned into the pBluescript plasmid vector (Stratagene), and the resulting plasmids are used as sequencing templates. Additional subclones are generated using the Exo III nested deletion method. A 3.7 kb Sal I fragment corresponding to the 3′ end of the S domain and all of the kinase domain is subcloned into the pUC 19 plasmid vector [available from GIBCO-BRL]. The resulting plasmid, p281S, is subjected to Exo III digestion from the 5′ end of the gene, using the Erase-a-Base kit (Promega) based upon the procedure developed by Heinkoff [see Gene 28:351 (1984)].

Following re-ligation, the mixed population of partially deleted plasmids are transformed into DH5α E. coli cells. Twenty colonies are selected that harbored plasmids with partially deleted 281 fragments. These are designated p281 S-A to p281 S-T. Together, the deletions spanned approximately 3 kb. Templates prepared from the above subclones are sequenced using the commercially available Universal or Reverse primers [available from PROMEGA, Inc.], which bind to vector sites adjacent to the inserted 281 DNA. In order to prime sequencing reactions at sites within 281, 29 oligonucleotides (17-22 mer) are synthesized. These oligonucleotides bind to regularly spaced sites (approximately 200 bp apart) along the length of the $SRK_6$ gene within 281. Template DNA suitable for sequencing reactions are prepared from plasmid minipreps using the following protocol:

After growing overnight, a 5 ml culture of cells is centrifuged at 2,000 rpm for 10 min in a Sorvall centrifuge to pellet the cells. The supernatant is poured off and the pellet resuspended in 0.7 mls of cold STET, pH 8.0 (8% sucrose, 5% TX100, 50 mM EDTA at pH 8.0, and 50 mM Tris at pH 8.0) buffer. Fifty µl of 5 mg/ml lysozyme solution (100 mg lysozyme, 5 ml Tris at pH 8.0, 15 mls of distilled water) is added to the resuspended cells,the mixture transferred to a microfuge tube, and the tube boiled for 45 seconds to lyse the cells. The lysed cells are spun to pellet the cellular mixture, and the pellet is removed. An equal volume (about 0.5 mls) of isopropanol is added, the contents are mixed by inversion, and the tube placed in a -20°C freezer for 30 minutes. After removing the tube from the freezer, it is spun for 10 minutes to form a pellet, the pellet is rinsed with 1 ml of 95% ethanol, and any fluids are removed by vacuum drying. The dried powder is resuspended in 75 µl of TE (10 mM Tris at pH 7.5, and 1 mM EDTA at pH 8.0) within an ice bath.

**EXAMPLE V: Preparation of $SRK_6$-derived probes**

In further studying and characterizing the present invention, DNA probes are labeled with $^{32}P$ using the Random Primer Kit of Boehringer Mannheim. Three $SRK_6$-derived probes are used (see Fig. 2A):

- the "S-domain" probe, which is a 1186 bp fragment obtained by digesting plasmid p281 [described in Example IV] with HincII and which spanned the majority of $SRK_6$ exon 1;
- the "intron" probe, which is a 747 bp fragment derived from intron 1 that can be obtained by digesting plasmid p281 with both PstI and HincII;
- the 2135 bp "kinase-domain" probe, which is a 2135 bp fragment that spannes exons 2 through 7 and can be obtained as follows:

Plasmid p281 is digested with SalI, and the resulting SalI fragment extending from base pair 1565 to 5270 of p281 is inserted into the SalI site of the vector pUC19 to generate plasmid p281S. Using ExoIII digestion, plasmid p281S-E is generated, which contains a deletion of the p281S insert up to base pair 2656. The "kinase-domain" probe is produced by digesting p281S-E with EcoRI and HindIII.

A further probe that may be used in characterising the present invention, is

-the "actin" probe, a 1.6 Kb cDNA derived from a *B. oleracea* actin gene.

For DNA blot analysis, we used an SLG-specific probe derived from the 3′-untranslated region of $SLG_6$-cDNA, and the $SRK_6$-specific "intron" probe described above.

**EXAMPLE V: Tissue-specific expression of the $SRK_6$ Gene**

In an attemtpt to study the expression of the $SRK_6$ gene, poly(A+) RNA from pistils, anthers, leaves, cotyledons and roots is subjected to gel blot analysis using the above described separate probes from the kinase domain, the "S" domain, and the first intron of $SRK_6$.

RNA isolation and RNA blot analysis studies are also performed and the chromatographic data from these studies appears in figure 5. In these studies, poly A+ RNA is isolated from pistils, stigmas, anther, pollen, leaf, seedling roots and cotyledons using the FastTrack RNA purification protocols (Invitrogen) according to the manufacturer's instructions. Seedling roots and cotyledons are derived from seeds germinated in the dark for several days on vermiculite. Floral tissue (corresponding to approximately 30 flowers) and leaf tissues (0.25 g) are harvested from green-house grown plants. Samples containing approximately 2-5 µg of poly A+ RNA is subjected to electrophoresis on 1% (w/v) agarose gels in the presence of formaldehyde as described by Maniatis *et al.* An RNA ladder (Bethesda Research Laboratories) is used to estimate the molecular weight of mRNA transcripts. The RNA is transferred to GeneScreen membranes as described in Example IV, and prehybridization and hybridization conditions are as for DNA blots. Post-hybridization washes are performed at 65°C in 0.5% SDS, 2 X SSPE, and then 0.5% SDS, 0.2 X SSPE. The following $^{32}P$-labelled probes are used: a DNA fragment containing the S domain of the $SRK_6$ gene, a DNA fragment containing the kinase-homologous domain, and a DNA fragment containing the $SRK_6$-specific intron probe. In addition, a $^{32}P$-labelled actin probe is used to very that equal quantities of poly A+ RNA are loaded in each lane of the gel.

The above experiments result in the observation that the most intense hybridization signals are obtained in mature pistils where transcripts ranged in size from 1.6 kb to 5.9 kb (Fig. 5A). Some of these transcript species are assigned to genes other than $SRK_6$. In particular, the highly abundant "S"-homologous 1.6 kb transcript was previously shown to be derived from the SLG gene in experiments with transgenic tobacco. Only the transcript species of 3.0 kb (arrowhead), 4.1 kb (asterisk) and 2.3 kb (circle) are unambiguously assigned to the $SRK_6$ gene, and their characterization is described in detail.

The 3.0 kb transcript and a slighly differing transcript in anthers are recognized by both the kinase- and "S"-domain probes, but not the intron probe. These transcript sizes are consistent with the predicted size (2832 bp) of a fully spliced $SRK_6$ transcript. A 3.0 kb transcript is also detected by the kinase-domain probe in leaves, and in cotyledons after very long (>1 week) film exposures, however, not in seedling roots; this leaf transcript did not hybridize to the "S"-domain probe except under low-stringency wash conditions, and thus derived, not from the $SRK_6$ gene, but from any of several SRK-related genes detected in the *Brassica* genome. The 4.1 kb transcript hybridized to the "intron" probe as well as the kinase- and "S"-domain probes, and is of a size consistent with that predicted for an unspliced $SRK_6$ transcript (4180 bp). The 2.3 kb transcript hybridized to the kinase-domain and "intron" probes but not the "S"-domain probe, and is possibly the result of alternative splicing or the action of an alternative promoter.

**EXAMPLE VI:Detection of Linkage of SRK Gene to the S Locus: Genetic Segregation of RFLP Markers**

DNA gel blot hybridization is performed using DNA isolated from the $S_6$ and $S_{14}$ homozygotes by centrifugation of leaf nuclear DNA as described in Example II. DNA is isolated from $F_2$ progeny plants by a DNA minipreparation procedure based upon a rapid phage DNA extraction method according to the following protocol:

Approximately 0.25 g of leaf tissue harvested from an actively growing young vegetative bud is frozen in liquid nitrogen, and the frozen tissue is pulverized at liquid nitrogen temperature in an Eppendorf tube (1.5 ml) with a Kontes Eppendorf pestle. 400 μl of grind buffer (4 parts homogenization buffer:1 part phage lysis buffer) held at 65°C is added and the mixture homogenized briefly without foaming. Homogenization buffer consists of 0.1 M NaCl, 0.2 M sucrose, 0.01 M EDTA, and 0.03 M Tris-HCl at pH 8.0; phage lysis buffer consists of 0.25 M EDTA, 2.5% (w/v) SDS, and 0.5 M Tris-HCl at pH 9.2. The suspension is next incubated in a 65°C water bath for approximately 30 min., and 133 μl of 3M potassium acetate buffer at pH 4.7 is added. The suspension is incubated on ice approximately 30 minutes and spun in a microfuge for 10 min., and the supernatant transferred to a new Eppendorf tube. 700 μl of ethanol is added and the tube left at room temperature for 2 min., followed by centrifugation in a microfuge for 10 min. The pellet is washed in cold 70% ethanol, recentrifuged, and the washing step repeated. The pellet is then dried in a roto-vac, dissolved in 40 μl of water, and 1 μl of a DNase-free RNase solution (10 μg/ml) added.

Either 10 g of CsCl purified DNA or the amount of minipreparation DNA extracted from 300 mg of leaf tissue is restricted with Eco RI. Restriction fragments are fractionated on 0.9% agarose gels and transferred (Southern blots) to GeneScreenPlus membranes (DuPont-New England Nuclear). The membranes are prehybridized at 65°C in a solution of 10% dextran sulfate (wt/vol), 330 mM sodium phosphate at pH 7.0, 10 mM EDTA/5% (w/v), NaDodSO4, and denatured salmon sperm DNA at 100 μg/ml. Two different $^{32}P$-labelled probes prepared by the random primed labelling reaction of Feinberg and Vogelstein, 1983, and 1984 are used: (i) a probe specific for the SLG gene and derived from the 3′ untranslated region of $SLG_6$ cDNA [see Nasrallah *et al* (1988)]; and (ii) a probe specific for the $SRK_6$ gene. The membranes are washed at 65°C in a solution of 0.3 M sodium chloride, 40 mM Tris-HCl buffer at pH 7.2, 2 mM EDTA, and 0.5% (w/v) of NaDodSO4. This linkage allows for the use of probes homologous to the SLG genes to isolate and clone SRK genes.

As a result of the above experiments using clones derived from the S locus a genomic region defined by 9 independent overlapping clones could be indentified. Partial sequence analysis of DNA fling the region of SLG homology in these clones revealed sequence similarity to known protein kinases and to ZmPK1. $SRK_6$ is next shown to be genetically linked to the S locus by restriction fragment length polymorphism (RFLP) analysis of a population of $F_2$ plants segregating for the $S_6$ and $S_{14}$SI phenotypes (Fig. 1). As previously reported [see Genetics 127:221 (1991)], an SLG-specific probe identified an EcoRI RFLP between the two parental strains that indicated which SLG allele(s) is present in each of the plants examined (Fig, 1A). When a DNA fragment immediately downstream of the SLG homologous region of the $SRK_6$ gene (derived from intron 1 of $SRK_6$; see Fig. 2B) is used as a probe, a single EcoRI restriction fragment is identified in the S6 parent but not in the S14 parent (Fig. 1B). A perfect correlation is observed between the presence of the SLG6 allele, the presence of the SRK6 allele, and the S6 incompatibility specificity as determined by pollination assays in a total of 78 F2 plants in this population (16 $S_6$:38 $S_6S_{14}$:24 $S_{14}S_{14}$), of which 9 are shown in Fig. 1.

**EXAMPLE VII: Vector Construction for Transformation of self-incompatability into plants**

In order to verify the origin of these transcripts, the $SRK_6$ gene is introduced into a recipient *B. oleracea* strain that carries the $S_2$ allele using a method as outlined in the following EXAMPLE VII.

(a) Construction of pCIB 542, an agropine VIR helper plasmidbearing a spectinomycin drug resistance gene in the place ofthe T-DNA

Prior to the transformation of *Brassica* described in the following example, pCIB 542, an agropine VIR helper plasmid being a spectinomycin drug resistance gene in the place of the T-DNA is constructed. The Ti plasmid, pTi Bo 542 is of interest for use in transformation because *Agrobacteria* bearing this Ti plasmid are able to infect the agronomically important legumes alfalfa and soybean [see Hood *et al* (1984)]. The construction of a pTi Bo 542 derivative deleted of the T-DNA has been described [see Hood *et al* (1986)]. In this construction, named EHA 101, the T-DNA is replaced by the a kanamycin drug resistance gene.

A derivative of EHA 101 having the kanamycin drug resistance gene replaced by a spectinomycin drug resistance gene may be constructed as follows: The plasmid p pi delta 307 [see Hood *et al* (1986)] has a 1.7 kb region of homology to the left side of Bam 5 of pTi Bo 542 and an 8 kb region of homology to the right side of Bam2a of pTi Bo 542, separated by a unique EcoR1 site. The plasmid pMON30/SR20, which has been deposited under the Budapest Treaty with the American Type Culture Collection and designated as accession number 67113, bears the spectinomycin/streptomycin [spc/str] drug resistance gene from Tn7 [Hollingshead, S. and Vapnek, D. (1985)]; pMon 30 is digested with EcoR1; the 5.5 kb fragment containing the spc/str gene isolated from an agarose gel; and ligated into EcoR1-restricted p pi delta 307. The desired recombinant is selected as a spectinomycin resistant (50 g/ml) tetracycline resistant (10 μg/ml) transformant of *E. coli* HB101.

Plasmid DNA can be introduced into *Agrobacteria* A136/EHA101 by transformation. The desired transformant is selected by its streptomycin-resistant tetracycline-resistant kanamycin-resistant phenotype. Homogenates [see Matzke, AJM & Chilton M-D (1981)] of EHA101 and the spectinomycin plasmid are selected after conjugal introduction of the eviction plasmid R751-pMG2 [see Jacoby, G *et al* (1976)] and selection on gentamycin (50 μg/ml) and spectinomycin. The desired homogenate has a gentamycin-resistant, spectinomycin-resistant, tetracycline-sensitive, and kanamycin-sensitive phenotype. The structure of the resulting plasmid is confirmed by Southern analysis. A derivative cured of the eviction plasmid is then selected by its drug resistance phenotype of gentamycin sensitivity. This selection is confirmed by Southern analysis.

(b) Construction of transformation vectors

The construction of transformation vectors, according to one embodiment useful in the present invention and using the *Brassica* gene is designated SLG-13. This encodes the S-locus specific glycoprotein (SLSG) and is isolated from *Brassica oleracea* S13 homozygote [see Nasrallah JB *et al* (1988)]. SLG-13 is contained on an 11 kilobase (kb) EcoR1 restriction fragment which contains the SLSG-coding region and approximately 4.2 kb of 5′ and 5.0 kb of 3′ flanking sequence.

This EcoR1 fragment is inserted into the multiple cloning site of the binary vector pC1B10 [see Rothstein *et al* (1987)] carrying a chimeric kanamycin-resistance gene for selection of transformed plants. The recombinant vector pH1 [see Moore HM and Nasrallah JB (1990)] or SLG13/pCIB10 carrying the S13 gene is mobilized into *Agrobacterium tumefaciens* strain pC1B542/A136 [a derivative of strain EHA101 described in Hood *et al* (1986)] by triparental mating according to standard published procedures [Rogers SG *et al* (1988)].

Transformation vectors carrying SLG genes are also constructed in the Ti plasmids pBI 121 (Clonetech Laboratories) and pBIN 19 [see Jefferson RA *et al* (1987)]. Plant transformation utilizing these vectors has also been successful.

Transformation of *Brassica* is performed according to a method as used in the related crucifer *Arabidopsis* which uses a mixture of *A. tumefaciens* cells harboring the SLG13/pCIB10 construct and of root-inducing plasmid from the wild type *A. rhizogenes* strain A4 [Van Sluys *et al* (1987)]. In this general protocol, using an SLG gene, tissue explants of *B.napus var.* "Westar" are obtained and treated essentially as described by Fry *et al*, 1987. Following selection on kanamycin, a kanamycin-resistant primary transformant plant is selected and analyzed for the integration and expression of the appropriate gene, such as the desired SLG or SRK transgene.

Using this general protocol, the transformation of *Brassica* with an SRK gene according to the present invention is conducted as in the following example.

**EXAMPLE VII: Transformation of *Brassica* with the SRK Gene**

The 5.8 kb EcoRI fragment that contained the SRK6 coding region with 0.45 kb of upstream and 1.42 kb of downstream fling sequence, and a chimeric hygromycin phosphotransferase gene for selection of transformed plants are inserted into the multiple cloning site of the binary vector pBIN19. The resulting vector, pJN280-1, is introduced into cells of *Agrobacterium tumefaciens* strain pCIB542/A136 that harbored the resulting plasmid are used to transform flowering stem disks of *B. oleracea* as follows:

Two *B. oleracea* commercial $F_1$ hybrids are used as gene recipient plants: "Kairan" (Chinese Kale, *B. oleracea* L. var alboglabra) and broccoli (*B. oleracea* L. var botrytis). Flower stem disks are transformed [see Fry *et al* (1987)] with the modification that hygromycin (20 mg/l) is used for selecting transformants instead of kanamycin.

Terminal internodes from a flowering stem are removed and surface sterilized in 70 % (v/v) ethanol for 1 minute, 2 % (v/v) sodium hypochlorite for 20 minutes, and followed by a rinse, three times, in sterile distilled water. The sterilized stems are then cut into 5 mm discs and dipped into an Agrobacterium cell mixture for 1 minute. The discs are then removed, blotted dry on sterilized filter paper, and placed over a feeder layer of tobacco cells plated onto a co-cultivation medium consisting of MS salts and vitamins, 3 % sucrose, 1 mg/l NAA, 0.5 mg/l BAP, and 0.8 % Agar. Following co-cultivation for two days in the dark at 28°C, the discs are transferred to culture plates containing the same medium supplemented with 500 mg/l carbencillin to inhibit bacterial growth and 20 mg/l hygromycin to select for transformation with the pJN280-1 construct. The plates are cultured at 25°C under cool white light, and the stem discs are transferred to fresh selective mia every two weeks.

Hygromycin is added to the medium for 3 weeks starting from a week after co-culture. Hygromycin resistance is confirmed by placing leaf segments of regenerated shoots on an expression assay medium containing 50 mg/l hygromycin. Untransformed leaf segments are bleached in a week, while transformed leaves remained green and produced callus. The transgenic plants thus identified are grown in a greenhouse with supplemental illumination.

The presence of the $SRK_6$ transgene is confirmed in hygromycin-resistant plants by DNA blot analysis of Eco RI digested DNA using the "intron" probe.

Because the SRK genes derived from the $S_2$ and $S_6$ alleles are highly diverged, the SRK transcripts endogenous to this strain are not recognized by the $SRK_6$-derived probes at the stringency used. In 10 transgenic plants analyzed, several showed detectable levels of the splices and unspliced transcripts (Fig 5C). These transcripts are observed exclusively in pistil and anther tissue and are not detected in untransformed controls. Transcripts of 1.6 kb are detected in both transformed and untransformed plants, and therefore represented endogenous messages not encoded by the $SRK_6$ transgene.

In addition, SRK directs the synthesis of several mRNA species that potentially encode different protein products. For example, the unspliced 4.1 kb transcript would retain the in-frame stop codon in intron 1 (see above), and would thus encode a secreted C-terminally truncated protein encompassing only "S" domain. In contrast, the 2.3 kb transcript, which lacks most or all of exon 1, possibly encodes an N-terminally truncated protein. Instances in which receptor tyrosine kinase genes encode N-terminally truncated as well as C-terminally truncated forms have been identified.

**EXAMPLE VIII: Evaluation Of Self-Incompatibility**

The procedure developed by Ko and Baer, 1968 is routinely used to evaluate pollen-stigma interactions for compatibility and incompatibility. In this protocol, excised pistils are collected at least 24 to 48 hours after pollination and macerated in 1 N NaOH for 1 to 2 hours at 22°C to 24°C. The pistils are then washed 3 times in dissolved water, 15 minutes per wash, placed in a drop of aniline blue solution [0.1 % (w/v) aniline blue dissolved in 0.1 N K3PO4 and left overnight at 37°C in a light-proof bottle] on a glass microscope slide, covered with a #2 cover slip, and gently squashed under the cover slip to spread the stylar stigmatic tissues into a monolayer. The stigmas and pollen are then viewed with a fluorescence microscope, under short wave illumination (350 to 400 nm). In addition to the test material, compatible and incompatible pollen-stigma controls are run with each test.

Callose deposits in the stigmatic papillae exhibited bright yellow green fluorescence as did callose in the pollen tubes. Incompatible *Brassica* pollen frequently either failed to germinate or produced short curled and often thickened tubes which may or may not penetrate the stigmatic papillae. Papillar cells in incompatible reactions may produce large amounts of callose. Compatible pollen germinates to produce long lightly fluorescing tubes which grow through the style toward the ovules.

In conclusion, we have described the isolation and analysis of unreported genomic and cDNA clones. The isolation of SLG-related genomic clones from libraries of *B. oleracea* homogenous for both $S_6$ and $S_2$ DNA is previously described in this application. Two $SRK_6$ cDNA clones, are isolated from an $S_6S_6$ stigma cDNA library by screening with the kinase-domain probe of the $SRK_6$. A third cDNA clone, pJS30, is isolated following amplification by the polymerase chain reaction (PCR) of $S_6S_6$ stigma cDNA using the mRNA GeneAmp kit (Perkin-Elmer/Cetus). The amplification is primed with an upstream oligonucleotide primer (Seq. No. 7) (5′ACTTGTGGCAAAGCTTCGATT3′) and a downstream primer (5′CCATCCCGAATTCCGAGATCT3′) complementary sequences within the "S" and kinase domains of $SRK_6$ respectively. The resulting figment is cloned in the vector pCR1000 using the TA cloning kit (Invitrogen). Dideoxy sequencing is performed with double stranded plasmid templated prepared by cloning restriction fragments into appropriate Bluescript vectors (Stratagene) and by generating nested deletions with the Erase-a-base kit (Promega). In addition, oligonucleotides complementary to regularly spaced sites along the $SRK_6$ gene are used as sequencing primers. Sequence analysis is performed with the IBI Pustell and GCG version 6.0 (Univ. of Wisconsin Biotechnology Center) software packages, and the GenBank and SWISS-PROT data bases are searched for protein similarity using FASTA, a computer programm available on the University of Wisconsin Biotechnology Center Computer software.

**DEPOSITION**

Within the scope of the present application, the following microorganisms and plasmids have been deposited at the "American Type Culture Collection" in Rockville, Maryland, USA, which is recognised in accordance with the Budapest Treaty as international depository, to comply with the requirements for the international recognition of the deposit of microorganisms for the purpose of patenting.

| Organism | Date of deposit | Date of viability testing | Designation |
|---|---|---|---|
| p281 | June 18, 1991 | June 25, 1991 | ATCC 75036 |
| pMON30/SR20 | May 14, 1986 | May 15, 1986 | ATCC 67113 |

## BIBLIOGRAPHY


**Bateman,** Heredity 9, 53 (1955)

**Bevan** *et al*, Nature 304: 184-187 (1983).

**Boyes** *et al*, Genetics 127, 221 (1991)

**Chen** *et al*, Mol Gen Genet 222, 241 (1990)

**Crossway** *et al*, BioTechniques 4:320-334 (1986)

**Feinberg** and Vogelstein, Anal Biochem 132, 6 (1983)

**Feinberg** and Vogelstein, Anal Biochem 137, 266 (1984)

**Fry** *et al*, Plant Cell Rep 6, 321 (1987)

**Haymes** *et al*, Nucleic Acid Hybridisation a Practical Approach, IRL Press, Oxford, England (1985)]

**Heslop-Harrison,** Nature 218, 90 (1968)

**Heslop-Harrison,** Annu Rev Plant Physiol 26, 403 (1975)

**Hinchee** *et al* Biotechnology 6: 915-921 (1988)

**Hollingshead** and Vapnek, Plasmid 13, 17-30 (1985)

**Hood** *et al*, Bio/Technology 2, 702 (1984)

**Hood** *et al*, J Bacteriol 168, 1291 (1986)

**Horsch** *et al*, Science 227: 1229 (1985)

**Jacoby** *et al*, J Bacteriol 127, 1278 (1976)

**Jefferson** *et al*, EMBO J 6, 3901 (1987)

**Jefferson,** Plant Molecular Biology Reporter 5: 387-405 (1987)

**Kandasamy** *et al*, Dev Biol 134, 462 (1989)

**Ko** and Baer, Euphytica 17, 298 (1968)

**Lathe** *et al*, J Mol Biol 183, 1-12 (1985)

**McBride** *et al*, Plant Molecular Biology 14: 266-276 (1990)

**McCabe** *et al*, Bio/Technology 6:923-926 (1988)

**Maniatis** *et al*, Molecular Cloning: A Laboratory Manual. Cold Spring Harbor Laboratory, Cold Spring Harbor, NY, (1982)]

**Matzke** and Chilton, J Mol Appl Genet 1, 39 (1981)

**Maxam** and Gilbert, 'Sequencing end-labelled DNA with base-specific chemical cleavage', in: Methods in Enzymology 65: 499-560, Academic Press, New York, London, 1980

**Mohnen** *et al*, EMBO J 4, 1631-1635 (1985)

**Moore** and Nasrallah, Plant Cell 2, 29 (1990)

**Nasrallah** *et al*, Nature 326, 617 (1987)

**Nasrallah** *et al*, Proc Natl Acad Sci USA 85, 5551-5555 (1988)
**Nasrallah** and Nasrallah, Annu Rev Genet 23, 121 (1989)
**Neuhaus** *et al*, Theor Appl Genet 75, 30-36 (1987)
**Paszkowski** *et al*, EMBO J. 3:2717-2722 (1984)
**Riggs** *et al*, Proc. Nat. Acad. Sci. USA 83:5602-5606 (1986)
**Rogers** *et al*, "Use of cointegrating Ti plasmid vectors.", in: Gelvin SB, Shilperoort RA (eds.). Plant Mol. Biol. Manual, Kluwer Academic Publishers (1988)
**Rothstein** *et al*, Gene 53, 153 (1987)
**Sanford** *et al*, U.S. Patent 4,945,050
**Sanger** *et al*, Proc Natl Acad Sci USA 74 5463-5467 (1977)
**Thomas** and Davis, J Mol Biol 91, 315 (1975)]
**Thompson** *et al*, EMBO J 6: 2519-2523 (1987)
**Van den Elzen** *et al.*, Plant Molecular Biology 5: 299-392 (1985)
**Van Sluys** *et al*, EMBO J 6, 3881 (1987)
**Velten** *et al.*, EMBO J. 3: 2723-2730 (1984)
**Walker** and Zhang, Nature 345, 743 (1990)

## SEQUENCE LISTING

In the Sequence Listing shown below the following DNA molecules and polypeptides are described by their respective DNA and amino acid sequences.

SEQ ID NO 1 shows the DNA sequence of the $SRK_6$ cDNA.
SEQ ID NO 2 shows the corresponding amino acid sequence of $SRK_6$.
SEQ ID NO 3 shows the amino acid sequence of $SRK_2$.
SEQ ID NO 4 shows the DNA sequence of the $SRK_2$ cDNA.
SEQ ID NO 5 shows the amino acid sequence of subdomain VI of $SRK_6$.
SEQ ID NO 6 shows the amino acid sequence of subdomain VIII of $SRK_6$.
SEQ ID NO 7 shows the DNA sequence of the $S_6$ primer.
SEQ ID NO 8 shows the DNA sequence of the $SRK_6$ primer.

## Claims

1. An isolated DNA molecule encoding an SRK polypeptide comprising an S-locus binding domain, a transmembrane domain, and a protein kinase domain.

2. An isolated DNA molecule according to claim 1 wherein said S-locus binding domain is substantially homologous to an SLG gene.

3. An isolated DNA molecule according to claim 2 wherein said S-locus binding domain has about 70% homology to an SLG gene.

4. An isolated molecule according to anyone of claims 2 or 3 wherein said S-locus binding domain is homologous to an SLG gene from *Brassica*.

5. An isolated DNA molecule according to claim 4 wherein said S-locus binding domain is homologous to an SLG gene selected from the group consisting of *Brassica oleracea* S2, *Brassica oleracea* S6, *Brassica oleracea* S13, *Brassica oleracea* S14, *Brassica oleracea* S22, and *Brassica campestris* S8.

6. An isolated DNA molecule according to claim 1 wherein the kinase domain has a degree of homology with the kinase domain selected from the group consisting of the SRK6, SRK2, and ZMPK1 genes.

7. An isolated DNA molecule according to claim 1 wherein said kinase domain is substantially homologous to a kinase region from an SRK gene selected from the group consisting of the SRK2 gene of *Brassica oleracea*, *Brassica oleracea* SRK6 gene, *Brassica oleracea* SRK13 gene, *Brassica oleracea* SRK14 gene, *Brassica oleracea* SRK22 gene, and *Brassica* campestris SRK8 gene.

8. An isolated DNA sequence according to claim 1 encoding the production of an S Receptor Kinase having a nucleotide sequence corresponding essentially to:

```
ATG AAA GGT GCA CGA AAC ATC TAT CAC CAT TCT TAC ATG 39
TCC TTT TTG CTC GTC TTC GTT GTC ATG ATT CTA ATT CAT 78
CCT GCC CTT TCG ATC TAT ATC AAC ACT TTG TCG TCT ACA 117
GAA TCT CTT ACA ATC TCA AGC AAC AAA ACA CTT GTA TCT 156
CCC GGT AGT ATC TTC GAG GTC GGC TTC TTC AGA ACC AAT 195
TCT CGT TGG TAT CTC GGG ATG TGG TAC AAG AAA GTG TCC 234
GAC AGA ACC TAT GTA TGG GTT GCC AAC AGA GAT AAC CCA 273
CTC TCC AAT GCC ATT GGA ACC CTC AAA ATC TCA GGC AAT 312
AAT CTT GTC CTC CTT GAT CAC TCC AAT AAA CCT GTT TGG 351
TGG ACG AAT CTT ACT AGA GGA AAT GAG AGA TCT CCG GTG 390
GTG GCT GAG CTT CTC GCT AAC GGA AAC TTC GTG ATG CGA 429
GAC TCC AGT AAC AAC GAC GCA AGT GAA TAC TTG TGG CAA 468
AGT TTC GAT TAC CCT ACG GAT ACT TTG CTT CCA GAG ATG 507
AAA CTG GGT TAC AAC CTC AAA ACA GGG TTG AAC AGG TTC 546
CTT ACA TCA TGG AGA AGT TCA GAT GAT CCA TCA AGC GGG 585
AAT TTC TCG TAC AAG CTC GAA ACC CAA AGT CTT CCT GAG 624
TTT TAT CTA TCG CGG GAG AAC TTT CCA ATG CAT CGG AGT 663
GGT CCA TGG AAT GGA ATC CGA TTT AGT GGC ATA CCA GAG 702
GAC CAA AAG CTG AGT TAC ATG GTG TAC AAT TTC ATA GAG 741
AAT AAT GAA GAG GTC GCT TAT ACA TTC CGA ATG ACC AAC 780
AAC AGC TTC TAC TCG AGA TTG ACA CTA ATT TCC GAA GGG 819
TAT TTT CAG CGA CTG ACG TGG TAT CCG TCA ATA AGG ATA 858
TGG AAC AGG TTC TGG TCT TCT CCA GTG GAC CCC CAG TGT 897
GAT ACT TAC ATA ATG TGT GGA CCT TAC GCT TAC TGT GAC 936
GTG AAC ACA TCA CCG GTT TGT AAC TGT ATC CAA GGG TTC 975
AAT CCC CGG AAT ATA CAG CAG TGG GAT CAG AGA GTC TGG 1014
GCA GGT GGG TGT ATA AGG AGG ACG CAG CTT AGC TGC AGT 1053
GGA GAT GGT TTT ACC AGG ATG AAG AAG ATG AAG TTG CCA 1092
GAA ACT ACG ATG GCG ACT GTC GAC CGT AGT ATT GGT GTG 1131
AAA GAA TGT AAG AAG AGG TGC ATT AGC GAT TGT AAT TGT 1170
ACC GCT TTT GCA AAT GCA GAT ATC CGG AAT GGT GGG TCG 1209
GGT TGT GTG ATT TGG ACC GAA CGC CTT GAG GAT ATC CGG 1248
AAT TAC GCT ACT GAC GCT ATT GAC GGT CAA GAT CTT TAT 1287
GTC AGA TTG GCT GCA GCT GAT ATC GCT AAG AAG AGA AAC 1326
```

GCG AGT GGG AAA ATT ATA AGT TTG ACT GTT GGT GTT AGT 1365
GTT CTG CTT CTG CTG ATC ATG TTC TGC CTC TGG AAA AGA 1404
AAA CAA AAG CGA GCA AAA GCA AGT GCA ATA TCC ATT GCA 1443
AAT ACA CAG AGA AAC CAA AAC TTG CCT ATG AAC GAG ATG 1492
GTA CTA TCA AGC AAG AGA GAG TTT TCT GGA GAG TAC AAA 1521
TTT GAG GAA CTG GAA CTT CCA TTG ATA GAG ATG GAA ACT 1560
GTT GTC AAA GCC ACC GAA AAT TTC TCC AGC TGT AAC AAA 1599
CTC GGA CAA GGT GGT TTT GGT ATT GTT TAC AAG GGA AGA 1638
TTA CTT GAC GGG AAA GAA ATT GCA GTA AAA AGG CTA TCA 1677
AAG ACG TCA GTT CAA GGG ACT GAT GAG TTT ATG AAT GAG 1716
GTG ACA CTA ATT GCG AGG CTT CAG CAT ATA AAC CTT GTT 1755
CAA GTT CTT GGC TGT TGC ATT GAA GGA GAT GAG AAG ATG 1794
TTG ATA TAT GAG TAT TTG GAA AAT TTA AGC CTT GAT TCT 1833
TAT CTC TTT GGT AAA ACC CGA AGG TCT AAG CTA AAT TGG 1882
AAT GAG AGA TTC GAC ATT ACC AAT GGT GTT GCT CGA GGG 1911
CTT TTA TAT CTT CAT CAA GAC TCA CGG TTT AGG ATA ATC 1950
CAC AGA GAT TTG AAA GTA AGT AAC ATT TTG CTT GAC AAA 1989
AAT ATG ATC CCA AAG ATC TCG GAT TTT GGG ATG GCC AGG 2028
ATA TTT GAA AGG GAC GAA ACG GAA GCT AAC ACA ATG AAG 2067
GTG GTC GGA ACA TAC GGC TAC ATG TCC CCG GAA TAC GCA 2106
ATG TAT GGG ATA TTC TCG GAA AAA TCA GAT GTT TTC AGT 2145
TTT GGA GTC ATA GTT CTT GAA ATT GTT AGT GGA AAG AAG 2184
AAC AGA GGA TTC TAC AAC TTG GAC TAC GAA AAC GAT CTC 2223
CTA AGC TAT GTA TGG AGT CGT TGG AAG GAA GGA AGA GCG 2272
CTA GAA ATC GTA GAT CCC GTC ATC GTA GAT TCA CTG TCA 2301
TCA CAG CCA TCA ATA TTT CAA CCA CAA GAA GTC CTA AAA 2340
TGT ATT CAA ATT GGT CTC TTG TGT GTT CAA GAA CTT GCA 2379
GAG CAC AGA CCA GCG ATG TCG TCT GTG GTT TGG ATG TTT 2418
GGA AGT GAA GCA ACA GAG ATT CCT CAG CCT AAA CCG CCA 2457
GGT TAT TGC GTC AGA AGA AGT CCT TAT GAA CTT GAT CCT 2496
TCA TCA AGT TGG CAA TGT GAC GAA AAT GAA TCC TGG ACG 2535
GTG AAC CAG TAC ACC TGC TCA GTC ATT GAT GCC CGG 2571
TAATATGATA GCTGAGTGAT TCAATATCAT ATGTGAAAGA GGGAAAATAA 2621
AATCTCATTA GATAAGTAGG TTATTTCGAT AACCACTTCT TGTTATTTTC 2671
TGGCGGTGTT GTCATTATCC CCTTTATATT AAAAAGAAGC ATTTGTATTA 2721
AATCCCCTTG CCTCAAGAGA TATTCACAAG AATACTATTG TGACGTGACA 2771

GCCTCACTAT CGTTTAAACA TTACAATGCT GACGTGTGGC TTGTAAATAG 2821
CTTCTCAGAC CA 2833

9. An isolated DNA sequence according to claim 1 encoding the production of an S Receptor Kinase having a nucleotide sequence corresponding essentially to:

```
ATG AAA GGG GTA CAG AAC ATT TAC CAC CAT TCT TAC ACC 39
TTC TCG TTC TTG CTA GTC TTC CTT GTC TTG ATT CTA TTT 78
CAT CCT GCC CTT TCG ATC TAT GTC AAC ACT TTA TCG TCT 117
TCA GAG TCT CTC ACA ATC TCG AGC AAT AGA ACA CTT GTA 156
TCT CCC GGT GGA GTC TTC GAG CTT GGT TTC TTC AAA CCC 195
TTG GGA CGC TCG CGA TGG TAT CTG GGA ATA TGG TAT AAA 234
AAA GCC CCC TGG AAA ACC TAC GCA TGG GTC GCC AAC AGA 273
GAC AAC CCT CTC TCC AGT TCT ATT GGA ACC CTC AAA ATC 312
TCT GGC AAC AAT CTT GTC CTG CTA AGT CAG TCT ACT AAC 351
ACT GTT TGG TCG ACA AAT CTT ACT AGA GGA AAT GCG AGA 390
TCT CCG GTG ATA GCA GAG CTT CTT CCC AAC GGT AAT TTT 429
GTA ATA AGA CAC TCC AAC AAC AAA GAC TCA AGT GGA TTC 468
TTG TGG CAG AGT TTC GAT TTT CCG ACA GAT ACT TTA CTT 507
CCG GAG ATG AAA CTA GGT TAC GAT CTC AAA ACA GGG CGC 546
AAC AGG TTC CTT ACA TCG TGG AAA GGT TCA GAT GAT CCG 585
TCA AGC GGG AAT TTC GTG TAC AAA CTC GAC ATT CGA AGG 624
GGA TTG CCT GAG TTT ATT CTT ATA AAT CAA TTT TTG AAT 663
CAA CGT GTT GAA ACG CAA AGG AGC GGT CCT TGG AAT GGA 702
ATG GAG TTT AGT GGC ATA CCG GAG GTG CAG GGA TTA AAT 741
TAC ATG GTT TAC AAT TAT ACG GAG 765 AAC AGT GAG GAG 780
ATC GCT TAC TCG TTC CAT ATG ACC AAC CAA AGC ATC TAC 819
TCC AGA TTG ACA GTC AGT GAG TTG ACA CTC GAT CGA TTG 858
ACG TGG ATC CCG CCA TCA CGG GAT TGG AGC CTC TTC TGG 897
ACT TTA CCA ACG GAC GTG TGC GAT CCG CTT TAC TTA TGT 936
GGA TCT TAT TCT TAC TGT GAC CTA ATT ACG TCA CCT AAC 975
TGT AAC TGT ATT AGA GGG TTC GTT CCC AAG AAC CCG CAG 1014
CAG TGG GAC TTG AGA GAC GGA ACA CGG GGG TGT GTG AGG 1053
ACG ACG CAG ATG AGC TGT AGT GGA GAT GGG TTT TTG CGG 1092
CTA AAC AAT ATG AAT TTG CCG GAT ACT AAG ACG GCA ACT 1131
GTG GAT CGG ACA ATG GAT GTG AAA AAA TGT GAA GAG AGG 1170
```

TGT CTT AGC GAT TGT AAC TGT ACT TCG TTT GCT ATT GCG 1209
GAT GTT CGG AAT GGA GGA TTG GGT TGT GTG TTT TGG ACC 1248
GGA GAG CTC GTT GCG ATC AGG AAA TTC GCC GTC GGT GGT 1287
CAA GAT CTT TAC GTC AGA TTG AAT GCT GCT GAT CTA GAT 1326
ATT TCC TCG GGT GAG AAG AGA GAC CGA ACT GGA AAA ATC 1365
ATA GGT TGG AGT ATT GGA TCC AGC GTT ATG CTT ATT CTG 1404
AGT GTT ATC TTG TTC TGC TTT TGG AGG AGG AGA CAA AAG 1443
CAA GCA AAA GCA GAT GCA ACA CCT ATT GTG GGA AAT CAA 1492
GTT CTA ATG AAC GAG GTG GTG TTA CCA AGA AAG AAG AGA 1521
AAT TTT TCT GGA GAG GAC GAT GTA GAA AAT TTG GAA CTT 1560
CCA TTG ATG GAG TTT GAA GCT GTT GTC ACA GCC ACC GAA 1599
CAT TTC TCT GAT TTT AAC AAG GTC GGA AAA GGT GGT TTT 1638
GGT GTT GTT TAC AAG GGA AGG TTA GTT GAC GGG CAA GAA 1677
ATT GCA GTG AAG AGA CTA TCG GAA ATG TCA GCT CAA GGT 1716
ACC GAT GAG TTC ATG AAC GAA GTT AGG CTA ATG CAA AGC 1755
TTC AGC CAC AAT AAT CTT GTC CGA CTT CTT GGC TGT TGT 1794
GTT TAT GAG GGC GAG AAG ATC TTA ATT TAC GAG TAC TTG 1833
GAG AAT CTA AGC CTC GAT TCT CAT CTC TTT GAT GAA ACC 1882
AGA AGC TGT ATG TTA AAT TGG CAA ATG AGA TTT GAT ATT 1911
ATC AAT GGT ATT GCC CGA GGG CTT CTC TAT CTT CAC CAA 1950
GAT TCA CGG TTT AGA ATC ATC CAC AGG GAT TTG AAA GCA 1989
AGC AAT GTC TTG CTT GAT AAA GAT ATG ACT CCA AAA ATT 2028
TCA GAC TTT GGA ATG GCT AGG ATC TTT GGA CGG GAT GAG 2067
ACG GAA GCT GAC ACG AGG AAG GTG GTC GGA ACT TAT GGC 2106
TAC ATG TCT CCA GAA TAT GCG ATG AAC GGG ACA TTC TCA 2145
ATG AAG TCA GAT GTG TTC AGT TTT GGG GTC TTG CTT CTT 2184
GAA ATT ATA AGT GGC AAG AGG AAC AAA GGC TTA TGC GAC 2223
TCG GAT AGT AGC CTT AAT CTT CTC GGA TGT GTA TGG AGG 2272
AAT TGG AAA GAA GGT CAA GGT CTA GAG ATA GTA GAC AAG 2301
GTC ATC ATA GAT TCT TCA TCA CCA ACG TTC AGG CCA CGT 2340
GAA ATC TTA AGA TGC TTA CAA ATT GGC CTC TTG TGT GTT 2379
CAA GAA CGT GTG GAG GAT AGA CCA ATG ATG TCG TCA GTA 2418
GTT TTG ATG CTC GGA AGT GAA GCT GCA TTG ATT CCT CAA 2457
CCT AAA CAG CCA GGA TAT TGC GTC AGC GGA AGT TCT CTT 2496
GAA ACT TAT TCT AGG CGT GAC GAT GAA AAT TGC ACA GTG 2535
AAC CAA ATC ACC ATG TCG ATC ATT GAC GCT CGG TAA TAT 2574

```
GAT AGT CTT TGA TAA TAT TCT CAC TAT TAA AGT TTT ACT 2613
AAA TGG AAA AAA AGA GTT TTA CAA GTT GAG TGA CAA AGC 2652
GTG CCA AAC TCT TCA GTC TAT CGA AAT TTT CAT TCA TCC 2691
TCT GTA TAT TCT CTC GAA TTG GTT TCG TTA TTT CGA GTC 2730
AAT TCA CAG TCA ACA ACT GCA G                       2749
```

10. An isolated DNA sequence capable of expressing in plants a peptide having essentially the amino acid sequence:

```
Met Lys Gly Val Gln Asn Ile Tyr His His Ser Tyr Thr Phe Ser
                    5                  10                  15
Phe Leu Leu Val Phe Leu Val Leu Ile Leu Phe His Pro Ala Leu
                   20                  25                  30
Ser Ile Tyr Val Asn Thr Leu Ser Ser Ser Glu Ser Leu Thr Ile
                   35                  40                  45
Ser Ser Asn Arg Thr Leu Val Ser Pro Gly Gly Val Phe Glu Leu
                   50                  55                  60
Gly Phe Phe Lys Pro Leu Gly Arg Ser Arg Trp Tyr Leu Gly Ile
                   65                  70                  75
Trp Tyr Lys Lys Ala Pro Trp Lys Thr Tyr Ala Trp Val Ala Asn
                   80                  85                  90
Arg Asp Asn Pro Leu Ser Ser Ser Ile Gly Thr Leu Lys Ile Ser
                   95                 100                 105
Gly Asn Asn Leu Val Leu Leu Ser Gln Ser Thr Asn Thr Val Trp
                  110                 115                 120
Ser Thr Asn Leu Thr Arg Gly Asn Ala Arg Ser Pro Val Ile Ala
                  125                 130                 135
Glu Leu Leu Pro Asn Gly Asn Phe Val Ile Arg His Ser Asn Val
                  140                 145                 150
Asn Lys Asp Ser Ser Gly Phe Leu Trp Gln Ser Phe Asp Phe Pro
                  155                 160                 165
Thr Asp Thr Leu Leu Pro Glu Met Lys Leu Gly Tyr Asp Leu Lys
                  170                 175                 180
Thr Gly Arg Asn Arg Phe Leu Thr Ser Trp Lys Gly Ser Asp Asp
                  185                 190                 195
Pro Ser Ser Gly Asn Phe Val Tyr Lys Leu Asp Ile Arg Arg Gly
```

```
                    200                 205                 210
Leu Pro Glu Phe Ile Leu Ile Asn Gln Phe Leu Asn Gln Arg Val
                    215                 220                 225
Glu Thr Gln Arg Ser Gly Pro Trp Asn Gly Met Glu Phe Ser Gly
                    230                 235                 240
Ile Pro Glu Val Gln Gly Leu Asn Tyr Met Val Tyr Asn Tyr Thr
                    245                 250                 255
Glu Asn Ser Glu Glu Ile Ala Tyr Ser Phe His Met Thr Asn Gln
                    260                 265                 270
Ser Ile Tyr Ser Arg Leu Thr Leu Val Ser Glu Leu Thr Leu Asp
                    275                 280                 285
Arg Leu Thr Trp Ile Pro Pro Ser Arg Asp Trp Trp Ser Leu Phe
                    290                 295                 300
Trp Thr Leu Pro Thr Asp Val Cys Asp Pro Leu Tyr Leu Cys Gly
                    305                 310                 315
Ser Tyr Ser Tyr Cys Asp Leu Ile Thr Ser Pro Asn Cys Asn Cys
                    320                 325                 330
Ile Arg Gly Phe Val Pro Lys Asn Pro Gln Gln Trp Asp Leu Arg
                    335                 340                 345
Asp Gly Thr Arg Gly Cys Val Arg Thr Thr Gln Met Ser Cys Ser
                    350                 355                 360
Gly Asp Gly Phe Leu Arg Leu Asn Asn Met Asn Leu Pro Asp Thr
                    365                 370                 375
Lys Thr Ala Thr Val Asp Arg Thr Met Asp Val Lys Lys Cys Glu
                    380                 385                 390
Glu Arg Cys Leu Ser Asp Cys Asn Cys Thr Ser Phe Ala Ile Ala
                    395                 400                 405
Asp Val Arg Asn Gly Gly Leu Gly Cys Val Phe Trp Thr Gly Glu
                    410                 415                 420
Leu Val Ala Ile Arg Lys Phe Ala Val Gly Gly Gln Asp Leu Tyr
                    425                 430                 435
Val Arg Leu Asn Ala Ala Asp Leu Asp Ile Ser Ser Gly Glu Lys
                    440                 445                 450
Arg Asp Arg Thr Gly Lys Ile Ile Gly Trp Ser Ile Gly Ser Ser
                    455                 460                 465
Val Met Leu Ile Leu Ser Val Ile Leu Phe Cys Phe Trp Arg Arg
```

```
                470                 475                 480
Arg Gln Lys Gln Ala Lys Ala Asp Ala Thr Pro Ile Val Asn Asn
                485                 490                 495
Gln Val Leu Met Asn Glu Val Val Leu Pro Arg Lys Lys Arg Asn
                500                 505                 510
Phe Ser Gly Glu Asp Asp Val Glu Asn Leu Glu Leu Pro Leu Met
                515                 520                 525
Glu Phe Glu Ala Val Val Thr Ala Thr Glu His Phe Ser Asp Phe
                530                 535                 540
Asn Lys Val Gly Lys Gly Gly Phe Gly Val Val Tyr Lys Gly Arg
                545                 550                 555
Leu Val Asp Gly Gln Glu Ile Ala Val Lys Arg Leu Ser Glu Met
                560                 565                 570
Ser Ala Gln Gly Thr Asp Glu Phe Met Asn Glu Val Arg Leu Met
                575                 580                 585
Gln Ser Phe Ser His Asn Asn Leu Val Arg Leu Leu Gly Cys Cys
                590                 595                 600
Val Tyr Glu Gly Glu Lys Ile Leu Ile Tyr Glu Tyr Leu Glu Asn
                605                 610                 615
Leu Ser Leu Asp Ser His Leu Asp Glu Thr Arg Ser Cys Met Leu
                620                 625                 630
Asn Trp Gln Met Arg Phe Asp Ile Ile Asn Gly Ile Ala Arg Gly
                635                 640                 645
Leu Leu Tyr Leu His Gln Asp Ser Arg Phe Arg Ile Ile His Arg
                650                 655                 660
Asp Leu Lys Ala Ser Asn Val Leu Leu Asp Lys Asp Met Thr Pro
                665                 670                 675
Lys Ile Ser Asp Phe Gly Met Ala Arg Ile Phe Gly Arg Asp Glu
                680                 685                 690
Thr Glu Ala Asp Thr Arg Lys Val Val Gly Thr Tyr Gly Tyr Met
                695                 700                 705
Ser Pro Glu Tyr Ala Met Asn Gly Thr Phe Ser Met Lys Ser Asp
                710                 715                 720
Val Phe Ser Phe Gly Val Ile Leu Leu Glu Ile Ile Ser Gly Lys
                725                 730                 735
Arg Asn Lys Gly Leu Cys Asp Leu Asp Ser Ser Leu Asn Leu Leu
```

```
                740                     745                     750
Gly Cys Val Trp Arg Asn Trp Lys Glu Gly Gln Gly Leu Glu Ile
                755                     760                     765
Val Asp Lys Val Ile Ile Asp Ser Ser Ser Pro Thr Phe Arg Pro
                770                     775                     780
Arg Glu Ile Leu Arg Cys Leu Gln Ile Gly Leu Leu Cys Val Gln
                785                     790                     795
Glu Arg Val Glu Asp Arg Pro Met Met Ser Ser Val Val Leu Met
                800                     805                     810
Leu Gly Ser Glu Ala Ala Leu Ile Pro Gln Pro Lys Gln Pro Gly
                815                     820                     825
Tyr Cys Val Ser Gly Ser Ser Leu Glu Thr Tyr Ser Arg Arg Asp
                830                     835                     840
Asp Glu Asn Cys Thr Val Asn Gln Ile Thr Met Ser Ile Ile Asp
                845                     850                     855
Ala Arg
```

11. An isolated DNA sequence capable of expressing in plants a peptide having essentially the amino acid sequence:

```
Met Lys Gly Ala Arg Asn Ile Tyr His His Ser Tyr Met Ser Phe
                5                       10                      15
Leu Leu Val Phe Val Val Met Ile Leu Ile His Pro Ala Leu Ser
                20                      25                      30
Ile Tyr Ile Asn Thr Leu Ser Ser Thr Glu Ser Leu Thr Ile Ser
                35                      40                      45
Ser Asn Lys Thr Leu Val Ser Pro Gly Ser Ile Phe Glu Val Gly
                50                      55                      60
Phe Phe Arg Thr Asn Ser Arg Trp Tyr Leu Gly Met Trp Tyr Lys
                65                      70                      75
Lys Val Ser Asp Arg Thr Tyr Val Trp Val Ala Asn Arg Asp Asn
                80                      85                      90
Pro Leu Ser Asn Ala Ile Gly Thr Leu Lys Ile Ser Gly Asn Asn
                95                      100                     105
Leu Val Leu Leu Asp His Ser Asn Lys Pro Val Trp Trp Thr Asn
```

```
                    110                 115                 120
Leu Thr Arg Gly Asn Glu Arg Ser Pro Val Val Ala Glu Leu Leu
                    125                 130                 135
Ala Asn Gly Asn Phe Val Met Arg Asp Ser Ser Asn Asn Asp Ala
                    140                 145                 150
Ser Glu Tyr Leu Trp Gln Ser Phe Asp Tyr Pro Thr Asp Thr Leu
                    155                 160                 165
Leu Pro Glu Met Lys Leu Gly Tyr Asn Leu Lys Thr Gly Leu Asn
                    170                 175                 180
Arg Phe Leu Thr Ser Trp Arg Ser Ser Asp Asp Pro Ser Ser Gly
                    185                 190                 195
Asn Phe Ser Tyr Lys Leu Glu Thr Gln Ser Leu Pro Glu Phe Tyr
                    200                 205                 210
Leu Ser Arg Glu Asn Phe Pro Met His Arg Ser Gly Pro Trp Asn
                    215                 220                 225
Gly Ile Arg Phe Ser Gly Ile Pro Glu Asp Gln Lys Leu Ser Tyr
                    230                 235                 240
Met Val Tyr Asn Phe Ile Glu Asn Asn Glu Glu Val Ala Tyr Thr
                    245                 250                 255
Phe Arg Met Thr Asn Asn Ser Phe Tyr Ser Arg Leu Thr Leu Ile
                    260                 265                 270
Ser Glu Gly Tyr Phe Gln Arg Leu Thr Trp Tyr Pro Ser Ile Arg
                    275                 280                 285
Ile Trp Asn Arg Phe Trp Ser Ser Pro Val Asp Arg Gln Cys Asp
                    290                 295                 300
Thr Tyr Ile Met Cys Gly Pro Tyr Ala Tyr Cys Asp Val Asn Thr
                    305                 310                 315
Ser Pro Val Cys Asn Cys Ile Gln Gly Phe Asn Pro Arg Asn Ile
                    320                 325                 330
Gln Gln Trp Asp Gln Arg Val Trp Ala Gly Gly Cys Ile Arg Arg
                    335                 340                 345
Thr Gln Leu Ser Cys Ser Gly Asp Gly Phe Thr Arg Met Lys Lys
                    350                 355                 360
Met Lys Leu Pro Glu Thr Thr Met Ala Thr Val Asp Arg Ser Ile
                    365                 370                 375
Gly Val Lys Glu Cys Lys Lys Arg Cys Ile Ser Asp Cys Asn Cys
```

```
                    380                     385                     390
Thr Ala Phe Ala Asn Ala Asp Ile Arg Asn Gly Gly Ser Gly Cys
                    395                     400                     405
Val Ile Trp Thr Glu Arg Leu Glu Asp Ile Arg Asn Tyr Ala Thr
                    410                     415                     420
Asp Ala Ile Asp Gly Gln Asp Leu Tyr Val Arg Leu Ala Ala Ala
                    425                     430                     435
Asp Ile Ala Lys Lys Arg Asn Ala Ser Gly Lys Ile Ile Ser Leu
                    440                     445                     450
Thr Val Gly Val Ser Val Leu Leu Leu Leu Ile Met Phe Cys Leu
                    455                     460                     465
Trp Lys Arg Lys Gln Lys Arg Ala Lys Ala Ser Ala Ile Ser Ile
                    470                     475                     480
Ala Asn Thr Gln Arg Asn Gln Asn Leu Pro Met Asn Glu Met Val
                    485                     490                     495
Leu Ser Ser Lys Arg Glu Phe Ser Gly Glu Tyr Lys Phe Glu Glu
                    500                     505                     510
Leu Glu Leu Pro Leu Ile Glu Met Glu Thr Val Val Lys Ala Thr
                    515                     520                     525
Glu Asn Phe Ser Ser Cys Asn Lys Leu Gly Gln Gly Gly Phe Gly
                    530                     535                     540
Ile Val Tyr Lys Gly Arg Leu Leu Asp Gly Lys Glu Ile Ala Val
                    545                     550                     555
Lys Arg Leu Ser Lys Thr Ser Val Gln Gly Thr Asp Glu Phe Met
                    560                     565                     570
Asn Glu Val Thr Leu Ile Ala Arg Leu Gln His Ile Asn Leu Val
                    575                     580                     585
Gln Val Leu Gly Cys Cys Ile Glu Gly Asp Glu Lys Met Leu Ile
                    590                     595                     600
Tyr Glu Tyr Leu Glu Asn Leu Ser Leu Asp Ser Tyr Leu Phe Gly
                    605                     610                     615
Lys Thr Arg Arg Ser Lys Leu Asn Trp Asn Glu Arg Phe Asp Ile
                    620                     625                     630
Thr Asn Gly Val Ala Arg Gly Leu Leu Tyr Leu His Gln Asp Ser
                    635                     640                     645
Arg Phe Arg Ile Ile His Arg Asp Leu Lys Val Ser Asn Ile Leu
```

| | | | | | | | | | | | | | | |
|---|---|---|---|---|---|---|---|---|---|---|---|---|---|---|
| | | | | 650 | | | | | 655 | | | | | 660 |
| Leu | Asp | Lys | Asn | Met | Ile | Pro | Lys | Ile | Ser | Asp | Phe | Gly | Met | Ala |
| | | | | 665 | | | | | 670 | | | | | 675 |
| Arg | Ile | Phe | Glu | Arg | Asp | Glu | Thr | Glu | Ala | Asn | Thr | Met | Lys | Val |
| | | | | 680 | | | | | 685 | | | | | 690 |
| Val | Gly | Thr | Tyr | Gly | Tyr | Met | Ser | Pro | Glu | Tyr | Ala | Met | Tyr | Gly |
| | | | | 695 | | | | | 700 | | | | | 705 |
| Ile | Phe | Ser | Glu | Lys | Ser | Asp | Val | Phe | Ser | Phe | Gly | Val | Ile | Val |
| | | | | 710 | | | | | 715 | | | | | 720 |
| Leu | Glu | Ile | Val | Ser | Gly | Lys | Lys | Asn | Arg | Gly | Phe | Tyr | Asn | Leu |
| | | | | 725 | | | | | 730 | | | | | 735 |
| Asp | Tyr | Glu | Asn | Asp | Leu | Leu | Ser | Tyr | Val | Trp | Ser | Arg | Trp | Lys |
| | | | | 740 | | | | | 745 | | | | | 750 |
| Glu | Gly | Arg | Ala | Leu | Glu | Ile | Val | Asp | Pro | Val | Ile | Val | Asp | Ser |
| | | | | 755 | | | | | 760 | | | | | 765 |
| Leu | Ser | Ser | Gln | Pro | Ser | Ile | Phe | Gln | Pro | Gln | Glu | Val | Leu | Lys |
| | | | | 770 | | | | | 775 | | | | | 780 |
| Cys | Ile | Gln | Ile | Gly | Leu | Leu | Cys | Val | Gln | Glu | Leu | Ala | Glu | His |
| | | | | 785 | | | | | 790 | | | | | 795 |
| Arg | Pro | Ala | Met | Ser | Ser | Val | Val | Trp | Met | Phe | Gly | Ser | Glu | Ala |
| | | | | 800 | | | | | 805 | | | | | 810 |
| Thr | Glu | Ile | Pro | Gln | Pro | Lys | Pro | Pro | Gly | Tyr | Cys | Val | Arg | Arg |
| | | | | 815 | | | | | 820 | | | | | 825 |
| Ser | Pro | Tyr | Glu | Leu | Asp | Pro | Ser | Ser | Ser | Trp | Gln | Cys | Asp | Glu |
| | | | | 830 | | | | | 835 | | | | | 840 |
| Asn | Glu | Ser | Trp | Thr | Val | Asn | Gln | Tyr | Thr | Cys | Ser | Val | Ile | Asp |
| | | | | 845 | | | | | 850 | | | | | 855 |
| Ala | Arg | | | | | | | | | | | | | |

**12.** A method of isolating and identifying an SRK gene comprising
(a) isolating one or more DNA sequences from an SLG gene clone from a self-incompatible plant;
(b) using the isolated sequences isolated from an SLG clone to screen a genomic library from a self-incompatible plant;
(c) isolating those clones from the genomic DNA library which hybridize to the DNA sequences isolated from an SLG gene clone; and
(d) sequencing those clones from the genomic DNA library which hybridize and identify SRK encoding DNA sequences or genes.

**13.** A method according to claim 12 wherein the genomic DNA library that is screened is from a self-incompatible plant of the same genotype as the self-incompatible plant from which DNA sequences are isolated.

**14.** A method for isolating and identifying an SRK encoding DNA sequence or gene comprising:
(a) isolating a genomic SLG clone from a self-incompatible plant;
(b) using hybridization to identify putative SRK encoding DNA sequences or genes which are SLG-related and kinase-related residue on the genomic clone;
(c) sequencing the putative SRK encoding DNA sequences or genes identified above to determine if one or more encode an SRK gene;
(d) if no SRK encoding DNA sequences or genes are identified on the genomic clone, using the end sequences of the clone insert as probes to isolate new genomic clones from the adjacent region of the chromosome;
(e) using hybridization to determine if any putative SRK encoding DNA sequences or genes reside on the new genomic clones;
(f) sequencing the putative SRK encoding DNA sequences or genes identified above to determine if one or more encode an SRK polypeptide; and
(g) repeating the steps above until an SRK encoded DNA sequence or genes located near the SLG gene is identified.

**15.** A method of imparting self-incompatibility to recipient plants comprising isolating a DNA sequence or gene which encodes for the expression of an S Receptor Kinase from a self-incompatible plant donor, and integrating the isolated DNA sequence or gene into the genome of a recipient plant that does not ordinarily produce S-receptor kinase whereby the recipient plant is self-incompatible.

**16.** A method according to claim 15 which comprises providing a self-incompatible strain of *Brassica* as the plant donor.

**17.** A method according to claim 16 which comprises providing a strain of *Brassica* carrying the SLG gene as the plant donor.

**18.** A method according to claim 15 which comprises isolating the S Receptor Kinase gene linked to a structural gene for the production of an S-locus specific glycoprotein.

**19.** A method according to claim 15 which comprises isolating a DNA molecule comprising a DNA sequence which encodes for the production of an S Receptor Kinase polypeptide, said DNA molecule having substantially the sequence according to claim 8.

**20.** A method according to claim 18 wherein the DNA sequence which codes for the production of an S Receptor Kinase polypeptide is genetically linked with a second structural gene.

**21.** A method according to claim 19 wherein the structural gene is capable of imparting herbicide tolerance to one or more herbicides.

**22.** A plant normally lacking a S Receptor Kinase gene which has been successfully transformed with a transformation vector which comprises a S Receptor Kinase gene.

**23.** A method for obtaining a plant with an acquired self-incompatibility trait which comprises
(a) providing a first plant having a SLG encoding DNA sequence or gene within its genome;
(b) providing a second plant having a SRK encoding DNA sequence or gene within its genome;
(c) crossing the first and second plants to obtain a $F_1$ generation;
(d) and selecting those plants from the $F_1$ generation which demonstrate an acquired self-incompatibility trait.

**24.** A method of altering the self-incompatibility phenotype of a recipient plant comprising:
(a) isolating an SRK encoding DNA sequence or gene which encodes an S receptor kinase; and
(b) integrating the isolated SRK encoding DNA sequence or gene into the genome of a recipient plant that does not naturally produce a S receptor kinase produced by the isolated gene whereby the recipient plant is incompatible with pollen of the same genotype as that of the isolated SRK gene.

**25.** A method according to claim 24 wherein the SRK gene is isolated from a plant of the genus *Brassica*.

**26.** A method of altering the self-incompatibility phenotype of a recipient plant comprising:
(a) isolating an SRK encoding DNA sequence or gene which encodes an S receptor kinase; and
(b) integrating the isolated SRK encoding DNA sequence or gene into the genome of a recipient plant that does not naturally produce the S receptor kinase produced by the isolated SRK encoding DNA sequence or gene whereby pollen produced by the recipient plant is incompatible with plants of the same genotype as that of the isolated SRK gene.

27. A method according to claim 26 wherein the SRK gene is isolated from a plant of the genus *Brassica*.

28. A method of altering the self-incompatibility phenotype of a recipient plant comprising:
(a) isolating an SRK encoding DNA sequence or gene which encodes an S receptor kinase;
(b) isolating an SLG encoding DNA sequence or gene which encodes an S-locus glycoprotein in the same genotype as that of the isolated SRK gene; and
(c) integrating the isolated SRK encoding DNA sequence or gene and the isolated SLG gene into the genome of a recipient plant that does not naturally produce the S receptor kinase produced by the isolated SRK gene, whereby the recipient plant is incompatible with pollen of the same genotype as that of the isolated SRK gene.

29. A method according to claim 28 wherein the isolated SRK gene and the isolated SLG gene are co-transformed into the recipient plant.

30. A method according to claim 28 wherein the isolated SRK gene and the isolated SLG gene are cloned into the same transformation vector and are co-integrated into the recipient plant.

31. A method according to claim 28 wherein the isolated SRK gene and the isolated SLG gene are isolated from a homozygous self-incompatible donor plant having a genotype selected from the group consisting of *Brassica oleracea* S2, *Brassica oleracea* S6, *Brassica oleracea* S13, *Brassica oleracea* S14, *Brassica oleracea* S22, and *Brassica campestris* S8.

32. A method according to claim 28 wherein the isolate SRK gene is the $SRK_6$ gene and the isolated SLG gene is the SLG6 gene.

**Claims for the following Contracting State : SP**

1. A method of isolating and identifying an SRK gene comprising
(a) isolating one or more DNA sequences from an SLG gene clone from a self-incompatible plant;
(b) using the isolated sequences isolated from an SLG clone to screen a genomic library from a self-incompatible plant;
(c) isolating those clones from the genomic DNA library which hybridize to the DNA sequences isolated from an SLG gene clone; and
(d) sequencing those clones from the genomic DNA libraiy which hybridize and identify SRK encoding DNA sequences or genes.

2. A method according to claim 1 wherein the genomic DNA library that is screened is from a self-incompatible plant of the same genotype as the self-incompatible plant from which DNA sequences are isolated.

3. A method for isolating and identifying an SRK encoding DNA sequence or gene comprising:
(a) isolating a genomic SLG clone from a self-incompatible plant;
(b) using hybridization to identify putative SRK encoding DNA sequences or genes which are SLG-related and kinase-related residue on the genomic clone;
(c) sequencing the putative SRK encoding DNA sequences or genes identified above to determine if one or more encode an SRK gene;
(d) if no SRK encoding DNA sequences or genes are identified on the genomic clone, using the end sequences of the clone insert as probes to isolate new genomic clones from the adjacent region of the chromosome;
(e) using hybridization to determine if any putative SRK encoding DNA sequences or genes reside on the new genomic clones;
(f) sequencing the putative SRK encoding DNA sequences or genes identified above to determine if one or more encode an SRK polypeptide; and
(g) repeating the steps above until an SRK encoded DNA sequence or genes located near the SLG gene is identified.

4. A method according to anyone of claims 1 or 3 wherein the SRK encoding DNA sequence or gene isolated and identified by the said method encodes an SRK polypeptide comprising an S-locus binding domain, a transmembrane domain, and a protein kinase domain.

5. A method according to claim 4, wherein the SRK encoding DNA sequence or gene has a S-locus binding domain that is substantially homologous to an SLG gene.

6. A method according to claim 5, wherein the SRK encoding DNA sequence or gene has a S-locus binding domain that has about 70% homology to an SLG gene.

7. A method according to anyone of claims 4 or 5, wherein the SRK encoding DNA sequence or gene has a S-locus binding domain that is homologous to an SLG gene from *Brassica*.

8. A method according to claim 7, wherein the SRK encoding DNA sequence or gene has a S-locus binding domain that is homologous to an SLG gene selected from the group consisting of *Brassica oleracea* S2, *Brassica oleracea* S6, *Brassica oleracea* S13, *Brassica oleracea* S14, *Brassica oleracea* S22, and *Brassica campestris* S8.

9. A method according to anyone of claims 1 or 3, wherein the SRK encoding DNA sequence or gene has a kinase domain which shows a degree of homology with the kinase domain selected from the group consisting of the SRK6, SRK2, and ZMPK1 genes.

10. A method according to claim 9, wherein the SRK encoding DNA sequence or gene has a kinase domain which is substantially homologous to a kinase region from an SRK gene selected from the group consisting of the SRK2 gene of *Brassica oleracea*, *Brassica oleracea* SRK6 gene, *Brassica oleracea* SRK13 gene, *Brassica oleracea* SRK14 gene, *Brassica oleracea* SRK22 gene, and *Brassica campestris* SRK8 gene.

11. A method according to claim 1 wherein the isolated DNA sequence or gene encodes the production of an S Receptor Kinase having a nucleotide sequence corresponding essentially to:

ATG AAA GGT GCA CGA AAC ATC TAT CAC CAT TCT TAC ATG 39
TCC TTT TTG CTC GTC TTC GTT GTC ATG ATT CTA ATT CAT 78
CCT GCC CTT TCG ATC TAT ATC AAC ACT TTG TCG TCT ACA 117
GAA TCT CTT ACA ATC TCA AGC AAC AAA ACA CTT GTA TCT 156
CCC GGT AGT ATC TTC GAG GTC GGC TTC TTC AGA ACC AAT 195
TCT CGT TGG TAT CTC GGG ATG TGG TAC AAG AAA GTG TCC 234
GAC AGA ACC TAT GTA TGG GTT GCC AAC AGA GAT AAC CCA 273
CTC TCC AAT GCC ATT GGA ACC CTC AAA ATC TCA GGC AAT 312
AAT CTT GTC CTC CTT GAT CAC TCC AAT AAA CCT GTT TGG 351
TGG ACG AAT CTT ACT AGA GGA AAT GAG AGA TCT CCG GTG 390
GTG GCT GAG CTT CTC GCT AAC GGA AAC TTC GTG ATG CGA 429
GAC TCC AGT AAC AAC GAC GCA AGT GAA TAC TTG TGG CAA 468
AGT TTC GAT TAC CCT ACG GAT ACT TTG CTT CCA GAG ATG 507
AAA CTG GGT TAC AAC CTC AAA ACA GGG TTG AAC AGG TTC 546
CTT ACA TCA TGG AGA AGT TCA GAT GAT CCA TCA AGC GGG 585
AAT TTC TCG TAC AAG CTC GAA ACC CAA AGT CTT CCT GAG 624
TTT TAT CTA TCG CGG GAG AAC TTT CCA ATG CAT CGG AGT 663
GGT CCA TGG AAT GGA ATC CGA TTT AGT GGC ATA CCA GAG 702
GAC CAA AAG CTG AGT TAC ATG GTG TAC AAT TTC ATA GAG 741
AAT AAT GAA GAG GTC GCT TAT ACA TTC CGA ATG ACC AAC 780
AAC AGC TTC TAC TCG AGA TTG ACA CTA ATT TCC GAA GGG 819
TAT TTT CAG CGA CTG ACG TGG TAT CCG TCA ATA AGG ATA 858
TGG AAC AGG TTC TGG TCT TCT CCA GTG GAC CCC CAG TGT 897
GAT ACT TAC ATA ATG TGT GGA CCT TAC GCT TAC TGT GAC 936
GTG AAC ACA TCA CCG GTT TGT AAC TGT ATC CAA GGG TTC 975
AAT CCC CGG AAT ATA CAG CAG TGG GAT CAG AGA GTC TGG 1014
GCA GGT GGG TGT ATA AGG AGG ACG CAG CTT AGC TGC AGT 1053
GGA GAT GGT TTT ACC AGG ATG AAG AAG ATG AAG TTG CCA 1092
GAA ACT ACG ATG GCG ACT GTC GAC CGT AGT ATT GGT GTG 1131
AAA GAA TGT AAG AAG AGG TGC ATT AGC GAT TGT AAT TGT 1170
ACC GCT TTT GCA AAT GCA GAT ATC CGG AAT GGT GGG TCG 1209
GGT TGT GTG ATT TGG ACC GAA CGC CTT GAG GAT ATC CGG 1248
AAT TAC GCT ACT GAC GCT ATT GAC GGT CAA GAT CTT TAT 1287
GTC AGA TTG GCT GCA GCT GAT ATC GCT AAG AAG AGA AAC 1326

GCG AGT GGG AAA ATT ATA AGT TTG ACT GTT GGT GTT AGT 1365
GTT CTG CTT CTG CTG ATC ATG TTC TGC CTC TGG AAA AGA 1404
AAA CAA AAG CGA GCA AAA GCA AGT GCA ATA TCC ATT GCA 1443
AAT ACA CAG AGA AAC CAA AAC TTG CCT ATG AAC GAG ATG 1492
GTA CTA TCA AGC AAG AGA GAG TTT TCT GGA GAG TAC AAA 1521
TTT GAG GAA CTG GAA CTT CCA TTG ATA GAG ATG GAA ACT 1560
GTT GTC AAA GCC ACC GAA AAT TTC TCC AGC TGT AAC AAA 1599
CTC GGA CAA GGT GGT TTT GGT ATT GTT TAC AAG GGA AGA 1638
TTA CTT GAC GGG AAA GAA ATT GCA GTA AAA AGG CTA TCA 1677
AAG ACG TCA GTT CAA GGG ACT GAT GAG TTT ATG AAT GAG 1716
GTG ACA CTA ATT GCG AGG CTT CAG CAT ATA AAC CTT GTT 1755
CAA GTT CTT GGC TGT TGC ATT GAA GGA GAT GAG AAG ATG 1794
TTG ATA TAT GAG TAT TTG GAA AAT TTA AGC CTT GAT TCT 1833
TAT CTC TTT GGT AAA ACC CGA AGG TCT AAG CTA AAT TGG 1882
AAT GAG AGA TTC GAC ATT ACC AAT GGT GTT GCT CGA GGG 1911
CTT TTA TAT CTT CAT CAA GAC TCA CGG TTT AGG ATA ATC 1950
CAC AGA GAT TTG AAA GTA AGT AAC ATT TTG CTT GAC AAA 1989
AAT ATG ATC CCA AAG ATC TCG GAT TTT GGG ATG GCC AGG 2028
ATA TTT GAA AGG GAC GAA ACG GAA GCT AAC ACA ATG AAG 2067
GTG GTC GGA ACA TAC GGC TAC ATG TCC CCG GAA TAC GCA 2106
ATG TAT GGG ATA TTC TCG GAA AAA TCA GAT GTT TTC AGT 2145
TTT GGA GTC ATA GTT CTT GAA ATT GTT AGT GGA AAG AAG 2184
AAC AGA GGA TTC TAC AAC TTG GAC TAC GAA AAC GAT CTC 2223
CTA AGC TAT GTA TGG AGT CGT TGG AAG GAA GGA AGA GCG 2272
CTA GAA ATC GTA GAT CCC GTC ATC GTA GAT TCA CTG TCA 2301
TCA CAG CCA TCA ATA TTT CAA CCA CAA GAA GTC CTA AAA 2340
TGT ATT CAA ATT GGT CTC TTG TGT GTT CAA GAA CTT GCA 2379
GAG CAC AGA CCA GCG ATG TCG TCT GTG GTT TGG ATG TTT 2418
GGA AGT GAA GCA ACA GAG ATT CCT CAG CCT AAA CCG CCA 2457
GGT TAT TGC GTC AGA AGA AGT CCT TAT GAA CTT GAT CCT 2496
TCA TCA AGT TGG CAA TGT GAC GAA AAT GAA TCC TGG ACG 2535
GTG AAC CAG TAC ACC TGC TCA GTC ATT GAT GCC CGG 2571
TAATATGATA GCTGAGTGAT TCAATATCAT ATGTGAAAGA GGGAAAATAA 2621
AATCTCATTA GATAAGTAGG TTATTTCGAT AACCACTTCT TGTTATTTTC 2671
TGGCGGTGTT GTCATTATCC CCTTTATATT AAAAAGAAGC ATTTGTATTA 2721
AATCCCCTTG CCTCAAGAGA TATTCACAAG AATACTATTG TGACGTGACA 2771

GCCTCACTAT CGTTTAAACA TTACAATGCT GACGTGTGGC TTGTAAATAG 2821
CTTCTCAGAC CA 2833

12. A method according to claim 1 wherein the isolated DNA sequence or gene encodes the production of an S Receptor Kinase having a nucleotide sequence corresponding essentially to:

```
ATG AAA GGG GTA CAG AAC ATT TAC CAC CAT TCT TAC ACC 39
TTC TCG TTC TTG CTA GTC TTC CTT GTC TTG ATT CTA TTT 78
CAT CCT GCC CTT TCG ATC TAT GTC AAC ACT TTA TCG TCT 117
TCA GAG TCT CTC ACA ATC TCG AGC AAT AGA ACA CTT GTA 156
TCT CCC GGT GGA GTC TTC GAG CTT GGT TTC TTC AAA CCC 195
TTG GGA CGC TCG CGA TGG TAT CTG GGA ATA TGG TAT AAA 234
AAA GCC CCC TGG AAA ACC TAC GCA TGG GTC GCC AAC AGA 273
GAC AAC CCT CTC TCC AGT TCT ATT GGA ACC CTC AAA ATC 312
TCT GGC AAC AAT CTT GTC CTG CTA AGT CAG TCT ACT AAC 351
ACT GTT TGG TCG ACA AAT CTT ACT AGA GGA AAT GCG AGA 390
TCT CCG GTG ATA GCA GAG CTT CTT CCC AAC GGT AAT TTT 429
GTA ATA AGA CAC TCC AAC AAC AAA GAC TCA AGT GGA TTC 468
TTG TGG CAG AGT TTC GAT TTT CCG ACA GAT ACT TTA CTT 507
CCG GAG ATG AAA CTA GGT TAC GAT CTC AAA ACA GGG CGC 546
AAC AGG TTC CTT ACA TCG TGG AAA GGT TCA GAT GAT CCG 585
TCA AGC GGG AAT TTC GTG TAC AAA CTC GAC ATT CGA AGG 624
GGA TTG CCT GAG TTT ATT CTT ATA AAT CAA TTT TTG AAT 663
CAA CGT GTT GAA ACG CAA AGG AGC GGT CCT TGG AAT GGA 702
ATG GAG TTT AGT GGC ATA CCG GAG GTG CAG GGA TTA AAT 741
TAC ATG GTT TAC AAT TAT ACG GAG 765 AAC AGT GAG GAG 780
ATC GCT TAC TCG TTC CAT ATG ACC AAC CAA AGC ATC TAC 819
TCC AGA TTG ACA GTC AGT GAG TTG ACA CTC GAT CGA TTG 858
ACG TGG ATC CCG CCA TCA CGG GAT TGG AGC CTC TTC TGG 897
ACT TTA CCA ACG GAC GTG TGC GAT CCG CTT TAC TTA TGT 936
GGA TCT TAT TCT TAC TGT GAC CTA ATT ACG TCA CCT AAC 975
TGT AAC TGT ATT AGA GGG TTC GTT CCC AAG AAC CCG CAG 1014
CAG TGG GAC TTG AGA GAC GGA ACA CGG GGG TGT GTG AGG 1053
ACG ACG CAG ATG AGC TGT AGT GGA GAT GGG TTT TTG CGG 1092
CTA AAC AAT ATG AAT TTG CCG GAT ACT AAG ACG GCA ACT 1131
```

GTG GAT CGG ACA ATG GAT GTG AAA AAA TGT GAA GAG AGG 1170
TGT CTT AGC GAT TGT AAC TGT ACT TCG TTT GCT ATT GCG 1209
GAT GTT CGG AAT GGA GGA TTG GGT TGT GTG TTT TGG ACC 1248
GGA GAG CTC GTT GCG ATC AGG AAA TTC GCC GTC GGT GGT 1287
CAA GAT CTT TAC GTC AGA TTG AAT GCT GCT GAT CTA GAT 1326
ATT TCC TCG GGT GAG AAG AGA GAC CGA ACT GGA AAA ATC 1365
ATA GGT TGG AGT ATT GGA TCC AGC GTT ATG CTT ATT CTG 1404
AGT GTT ATC TTG TTC TGC TTT TGG AGG AGG AGA CAA AAG 1443
CAA GCA AAA GCA GAT GCA ACA CCT ATT GTG GGA AAT CAA 1492
GTT CTA ATG AAC GAG GTG GTG TTA CCA AGA AAG AAG AGA 1521
AAT TTT TCT GGA GAG GAC GAT GTA GAA AAT TTG GAA CTT 1560
CCA TTG ATG GAG TTT GAA GCT GTT GTC ACA GCC ACC GAA 1599
CAT TTC TCT GAT TTT AAC AAG GTC GGA AAA GGT GGT TTT 1638
GGT GTT GTT TAC AAG GGA AGG TTA GTT GAC GGG CAA GAA 1677
ATT GCA GTG AAG AGA CTA TCG GAA ATG TCA GCT CAA GGT 1716
ACC GAT GAG TTC ATG AAC GAA GTT AGG CTA ATG CAA AGC 1755
TTC AGC CAC AAT AAT CTT GTC CGA CTT CTT GGC TGT TGT 1794
GTT TAT GAG GGC GAG AAG ATC TTA ATT TAC GAG TAC TTG 1833
GAG AAT CTA AGC CTC GAT TCT CAT CTC TTT GAT GAA ACC 1882
AGA AGC TGT ATG TTA AAT TGG CAA ATG AGA TTT GAT ATT 1911
ATC AAT GGT ATT GCC CGA GGG CTT CTC TAT CTT CAC CAA 1950
GAT TCA CGG TTT AGA ATC ATC CAC AGG GAT TTG AAA GCA 1989
AGC AAT GTC TTG CTT GAT AAA GAT ATG ACT CCA AAA ATT 2028
TCA GAC TTT GGA ATG GCT AGG ATC TTT GGA CGG GAT GAG 2067
ACG GAA GCT GAC ACG AGG AAG GTG GTC GGA ACT TAT GGC 2106
TAC ATG TCT CCA GAA TAT GCG ATG AAC GGG ACA TTC TCA 2145
ATG AAG TCA GAT GTG TTC AGT TTT GGG GTC TTG CTT CTT 2184
GAA ATT ATA AGT GGC AAG AGG AAC AAA GGC TTA TGC GAC 2223
TCG GAT AGT AGC CTT AAT CTT CTC GGA TGT GTA TGG AGG 2272
AAT TGG AAA GAA GGT CAA GGT CTA GAG ATA GTA GAC AAG 2301
GTC ATC ATA GAT TCT TCA TCA CCA ACG TTC AGG CCA CGT 2340
GAA ATC TTA AGA TGC TTA CAA ATT GGC CTC TTG TGT GTT 2379
CAA GAA CGT GTG GAG GAT AGA CCA ATG ATG TCG TCA GTA 2418
GTT TTG ATG CTC GGA AGT GAA GCT GCA TTG ATT CCT CAA 2457
CCT AAA CAG CCA GGA TAT TGC GTC AGC GGA AGT TCT CTT 2496
GAA ACT TAT TCT AGG CGT GAC GAT GAA AAT TGC ACA GTG 2535

```
AAC CAA ATC ACC ATG TCG ATC ATT GAC GCT CGG TAA TAT 2574
GAT AGT CTT TGA TAA TAT TCT CAC TAT TAA AGT TTT ACT 2613
AAA TGG AAA AAA AGA GTT TTA CAA GTT GAG TGA CAA AGC 2652
GTG CCA AAC TCT TCA GTC TAT CGA AAT TTT CAT TCA TCC 2691
TCT GTA TAT TCT CTC GAA TTG GTT TCG TTA TTT CGA GTC 2730
AAT TCA CAG TCA ACA ACT GCA G                       2749
```

**13.** A method according to claim 1 wherein the isolated DNA sequence or gene is capable of expressing in plants a peptide having essentially the amino acid sequence:

```
Met Lys Gly Val Gln Asn Ile Tyr His His Ser Tyr Thr Phe Ser
                 5                  10                  15
Phe Leu Leu Val Phe Leu Val Leu Ile Leu Phe His Pro Ala Leu
                20                  25                  30
Ser Ile Tyr Val Asn Thr Leu Ser Ser Ser Glu Ser Leu Thr Ile
                35                  40                  45
Ser Ser Asn Arg Thr Leu Val Ser Pro Gly Gly Val Phe Glu Leu
                50                  55                  60
Gly Phe Phe Lys Pro Leu Gly Arg Ser Arg Trp Tyr Leu Gly Ile
                65                  70                  75
Trp Tyr Lys Lys Ala Pro Trp Lys Thr Tyr Ala Trp Val Ala Asn
                80                  85                  90
Arg Asp Asn Pro Leu Ser Ser Ser Ile Gly Thr Leu Lys Ile Ser
                95                 100                 105
Gly Asn Asn Leu Val Leu Leu Ser Gln Ser Thr Asn Thr Val Trp
               110                 115                 120
Ser Thr Asn Leu Thr Arg Gly Asn Ala Arg Ser Pro Val Ile Ala
               125                 130                 135
Glu Leu Leu Pro Asn Gly Asn Phe Val Ile Arg His Ser Asn Val
               140                 145                 150
Asn Lys Asp Ser Ser Gly Phe Leu Trp Gln Ser Phe Asp Phe Pro
               155                 160                 165
Thr Asp Thr Leu Leu Pro Glu Met Lys Leu Gly Tyr Asp Leu Lys
               170                 175                 180
Thr Gly Arg Asn Arg Phe Leu Thr Ser Trp Lys Gly Ser Asp Asp
               185                 190                 195
```

```
Pro Ser Ser Gly Asn Phe Val Tyr Lys Leu Asp Ile Arg Arg Gly
                200                 205                 210
Leu Pro Glu Phe Ile Leu Ile Asn Gln Phe Leu Asn Gln Arg Val
                215                 220                 225
Glu Thr Gln Arg Ser Gly Pro Trp Asn Gly Met Glu Phe Ser Gly
                230                 235                 240
Ile Pro Glu Val Gln Gly Leu Asn Tyr Met Val Tyr Asn Tyr Thr
                245                 250                 255
Glu Asn Ser Glu Glu Ile Ala Tyr Ser Phe His Met Thr Asn Gln
                260                 265                 270
Ser Ile Tyr Ser Arg Leu Thr Leu Val Ser Glu Leu Thr Leu Asp
                275                 280                 285
Arg Leu Thr Trp Ile Pro Pro Ser Arg Asp Trp Trp Ser Leu Phe
                290                 295                 300
Trp Thr Leu Pro Thr Asp Val Cys Asp Pro Leu Tyr Leu Cys Gly
                305                 310                 315
Ser Tyr Ser Tyr Cys Asp Leu Ile Thr Ser Pro Asn Cys Asn Cys
                320                 325                 330
Ile Arg Gly Phe Val Pro Lys Asn Pro Gln Gln Trp Asp Leu Arg
                335                 340                 345
Asp Gly Thr Arg Gly Cys Val Arg Thr Thr Gln Met Ser Cys Ser
                350                 355                 360
Gly Asp Gly Phe Leu Arg Leu Asn Asn Met Asn Leu Pro Asp Thr
                365                 370                 375
Lys Thr Ala Thr Val Asp Arg Thr Met Asp Val Lys Lys Cys Glu
                380                 385                 390
Glu Arg Cys Leu Ser Asp Cys Asn Cys Thr Ser Phe Ala Ile Ala
                395                 400                 405
Asp Val Arg Asn Gly Gly Leu Gly Cys Val Phe Trp Thr Gly Glu
                410                 415                 420
Leu Val Ala Ile Arg Lys Phe Ala Val Gly Gly Gln Asp Leu Tyr
                425                 430                 435
Val Arg Leu Asn Ala Ala Asp Leu Asp Ile Ser Ser Gly Glu Lys
                440                 445                 450
Arg Asp Arg Thr Gly Lys Ile Ile Gly Trp Ser Ile Gly Ser Ser
                455                 460                 465
```

Val Met Leu Ile Leu Ser Val Ile Leu Phe Cys Phe Trp Arg Arg
470 475 480
Arg Gln Lys Gln Ala Lys Ala Asp Ala Thr Pro Ile Val Asn Asn
485 490 495
Gln Val Leu Met Asn Glu Val Val Leu Pro Arg Lys Lys Arg Asn
500 505 510
Phe Ser Gly Glu Asp Asp Val Glu Asn Leu Glu Leu Pro Leu Met
515 520 525
Glu Phe Glu Ala Val Val Thr Ala Thr Glu His Phe Ser Asp Phe
530 535 540
Asn Lys Val Gly Lys Gly Gly Phe Gly Val Val Tyr Lys Gly Arg
545 550 555
Leu Val Asp Gly Gln Glu Ile Ala Val Lys Arg Leu Ser Glu Met
560 565 570
Ser Ala Gln Gly Thr Asp Glu Phe Met Asn Glu Val Arg Leu Met
575 580 585
Gln Ser Phe Ser His Asn Asn Leu Val Arg Leu Leu Gly Cys Cys
590 595 600
Val Tyr Glu Gly Glu Lys Ile Leu Ile Tyr Glu Tyr Leu Glu Asn
605 610 615
Leu Ser Leu Asp Ser His Leu Asp Glu Thr Arg Ser Cys Met Leu
620 625 630
Asn Trp Gln Met Arg Phe Asp Ile Ile Asn Gly Ile Ala Arg Gly
635 640 645
Leu Leu Tyr Leu His Gln Asp Ser Arg Phe Arg Ile Ile His Arg
650 655 660
Asp Leu Lys Ala Ser Asn Val Leu Leu Asp Lys Asp Met Thr Pro
665 670 675
Lys Ile Ser Asp Phe Gly Met Ala Arg Ile Phe Gly Arg Asp Glu
680 685 690
Thr Glu Ala Asp Thr Arg Lys Val Val Gly Thr Tyr Gly Tyr Met
695 700 705
Ser Pro Glu Tyr Ala Met Asn Gly Thr Phe Ser Met Lys Ser Asp
710 715 720
Val Phe Ser Phe Gly Val Ile Leu Leu Glu Ile Ile Ser Gly Lys
725 730 735

Arg Asn Lys Gly Leu Cys Asp Leu Asp Ser Ser Leu Asn Leu Leu
740 745 750
Gly Cys Val Trp Arg Asn Trp Lys Glu Gly Gln Gly Leu Glu Ile
755 760 765
Val Asp Lys Val Ile Ile Asp Ser Ser Ser Pro Thr Phe Arg Pro
770 775 780
Arg Glu Ile Leu Arg Cys Leu Gln Ile Gly Leu Leu Cys Val Gln
785 790 795
Glu Arg Val Glu Asp Arg Pro Met Met Ser Ser Val Val Leu Met
800 805 810
Leu Gly Ser Glu Ala Ala Leu Ile Pro Gln Pro Lys Gln Pro Gly
815 820 825
Tyr Cys Val Ser Gly Ser Ser Leu Glu Thr Tyr Ser Arg Arg Asp
830 835 840
Asp Glu Asn Cys Thr Val Asn Gln Ile Thr Met Ser Ile Ile Asp
845 850 855
Ala Arg

**14.** A method according to claim 1 wherein the isolated DNA sequence or gene is capable of expressing in plants a peptide having essentially the amino acid sequence:

Met Lys Gly Ala Arg Asn Ile Tyr His His Ser Tyr Met Ser Phe
5 10 15
Leu Leu Val Phe Val Val Met Ile Leu Ile His Pro Ala Leu Ser
20 25 30
Ile Tyr Ile Asn Thr Leu Ser Ser Thr Glu Ser Leu Thr Ile Ser
35 40 45
Ser Asn Lys Thr Leu Val Ser Pro Gly Ser Ile Phe Glu Val Gly
50 55 60
Phe Phe Arg Thr Asn Ser Arg Trp Tyr Leu Gly Met Trp Tyr Lys
65 70 75
Lys Val Ser Asp Arg Thr Tyr Val Trp Val Ala Asn Arg Asp Asn
80 85 90
Pro Leu Ser Asn Ala Ile Gly Thr Leu Lys Ile Ser Gly Asn Asn
95 100 105
Leu Val Leu Leu Asp His Ser Asn Lys Pro Val Trp Trp Thr Asn

110 115 120
Leu Thr Arg Gly Asn Glu Arg Ser Pro Val Val Ala Glu Leu Leu
125 130 135
Ala Asn Gly Asn Phe Val Met Arg Asp Ser Ser Asn Asn Asp Ala
140 145 150
Ser Glu Tyr Leu Trp Gln Ser Phe Asp Tyr Pro Thr Asp Thr Leu
155 160 165
Leu Pro Glu Met Lys Leu Gly Tyr Asn Leu Lys Thr Gly Leu Asn
170 175 180
Arg Phe Leu Thr Ser Trp Arg Ser Ser Asp Asp Pro Ser Ser Gly
185 190 195
Asn Phe Ser Tyr Lys Leu Glu Thr Gln Ser Leu Pro Glu Phe Tyr
200 205 210
Leu Ser Arg Glu Asn Phe Pro Met His Arg Ser Gly Pro Trp Asn
215 220 225
Gly Ile Arg Phe Ser Gly Ile Pro Glu Asp Gln Lys Leu Ser Tyr
230 235 240
Met Val Tyr Asn Phe Ile Glu Asn Asn Glu Glu Val Ala Tyr Thr
245 250 255
Phe Arg Met Thr Asn Asn Ser Phe Tyr Ser Arg Leu Thr Leu Ile
260 265 270
Ser Glu Gly Tyr Phe Gln Arg Leu Thr Trp Tyr Pro Ser Ile Arg
275 280 285
Ile Trp Asn Arg Phe Trp Ser Ser Pro Val Asp Arg Gln Cys Asp
290 295 300
Thr Tyr Ile Met Cys Gly Pro Tyr Ala Tyr Cys Asp Val Asn Thr
305 310 315
Ser Pro Val Cys Asn Cys Ile Gln Gly Phe Asn Pro Arg Asn Ile
320 325 330
Gln Gln Trp Asp Gln Arg Val Trp Ala Gly Gly Cys Ile Arg Arg
335 340 345
Thr Gln Leu Ser Cys Ser Gly Asp Gly Phe Thr Arg Met Lys Lys
350 355 360
Met Lys Leu Pro Glu Thr Thr Met Ala Thr Val Asp Arg Ser Ile
365 370 375
Gly Val Lys Glu Cys Lys Lys Arg Cys Ile Ser Asp Cys Asn Cys

380 385 390

Thr Ala Phe Ala Asn Ala Asp Ile Arg Asn Gly Gly Ser Gly Cys

395 400 405

Val Ile Trp Thr Glu Arg Leu Glu Asp Ile Arg Asn Tyr Ala Thr

410 415 420

Asp Ala Ile Asp Gly Gln Asp Leu Tyr Val Arg Leu Ala Ala Ala

425 430 435

Asp Ile Ala Lys Lys Arg Asn Ala Ser Gly Lys Ile Ile Ser Leu

440 445 450

Thr Val Gly Val Ser Val Leu Leu Leu Leu Ile Met Phe Cys Leu

455 460 465

Trp Lys Arg Lys Gln Lys Arg Ala Lys Ala Ser Ala Ile Ser Ile

470 475 480

Ala Asn Thr Gln Arg Asn Gln Asn Leu Pro Met Asn Glu Met Val

485 490 495

Leu Ser Ser Lys Arg Glu Phe Ser Gly Glu Tyr Lys Phe Glu Glu

500 505 510

Leu Glu Leu Pro Leu Ile Glu Met Glu Thr Val Val Lys Ala Thr

515 520 525

Glu Asn Phe Ser Ser Cys Asn Lys Leu Gly Gln Gly Gly Phe Gly

530 535 540

Ile Val Tyr Lys Gly Arg Leu Leu Asp Gly Lys Glu Ile Ala Val

545 550 555

Lys Arg Leu Ser Lys Thr Ser Val Gln Gly Thr Asp Glu Phe Met

560 565 570

Asn Glu Val Thr Leu Ile Ala Arg Leu Gln His Ile Asn Leu Val

575 580 585

Gln Val Leu Gly Cys Cys Ile Glu Gly Asp Glu Lys Met Leu Ile

590 595 600

Tyr Glu Tyr Leu Glu Asn Leu Ser Leu Asp Ser Tyr Leu Phe Gly

605 610 615

Lys Thr Arg Arg Ser Lys Leu Asn Trp Asn Glu Arg Phe Asp Ile

620 625 630

Thr Asn Gly Val Ala Arg Gly Leu Leu Tyr Leu His Gln Asp Ser

635 640 645

Arg Phe Arg Ile Ile His Arg Asp Leu Lys Val Ser Asn Ile Leu

```
                650                 655                 660
Leu Asp Lys Asn Met Ile Pro Lys Ile Ser Asp Phe Gly Met Ala
                665                 670                 675
Arg Ile Phe Glu Arg Asp Glu Thr Glu Ala Asn Thr Met Lys Val
                680                 685                 690
Val Gly Thr Tyr Gly Tyr Met Ser Pro Glu Tyr Ala Met Tyr Gly
                695                 700                 705
Ile Phe Ser Glu Lys Ser Asp Val Phe Ser Phe Gly Val Ile Val
                710                 715                 720
Leu Glu Ile Val Ser Gly Lys Lys Asn Arg Gly Phe Tyr Asn Leu
                725                 730                 735
Asp Tyr Glu Asn Asp Leu Leu Ser Tyr Val Trp Ser Arg Trp Lys
                740                 745                 750
Glu Gly Arg Ala Leu Glu Ile Val Asp Pro Val Ile Val Asp Ser
                755                 760                 765
Leu Ser Ser Gln Pro Ser Ile Phe Gln Pro Gln Glu Val Leu Lys
                770                 775                 780
Cys Ile Gln Ile Gly Leu Leu Cys Val Gln Glu Leu Ala Glu His
                785                 790                 795
Arg Pro Ala Met Ser Ser Val Val Trp Met Phe Gly Ser Glu Ala
                800                 805                 810
Thr Glu Ile Pro Gln Pro Lys Pro Pro Gly Tyr Cys Val Arg Arg
                815                 820                 825
Ser Pro Tyr Glu Leu Asp Pro Ser Ser Ser Trp Gln Cys Asp Glu
                830                 835                 840
Asn Glu Ser Trp Thr Val Asn Gln Tyr Thr Cys Ser Val Ile Asp
                845                 850                 855
Ala Arg
```

**15.** A method of imparting self-incompatibility to recipient plants comprising isolating a DNA sequence or gene which encodes for the expression of an S Receptor Kinase from a self-incompatible plant donor, and integrating the isolated DNA sequence or gene into the genome of a recipient plant that does not ordinarily produce S-receptor kinase whereby the recipient plant is self-incompatible.

**16.** A method according to claim 15 which comprises providing a self-incompatible strain of *Brassica* as the plant donor.

**17.** A method according to claim 16 which comprises providing a strain of *Brassica* carrying the SLG gene as the plant donor.

**18.** A method according to claim 15 which comprises isolating the S Receptor Kinase gene linked to a structural gene for the production of an S -locus specific glycoprotein.

**19.** A method according to claim 15 which comprises isolating a DNA molecule comprising a DNA sequence which encodes for the production of an S Receptor Kinase polypeptide, said DNA molecule having substantially the sequence according to claim 8.

**20.** A method according to claim 18 wherein the DNA sequence which codes for the production of an S Receptor Kinase polypeptide is genetically linked with a second structural gene.

**21.** A method according to claim 19 wherein the structural gene is capable of imparting herbicide tolerance to one or more herbicides.

**22.** A method for obtaining a plant with an acquired self-incompatibility trait which comprises
(a) providing a first plant having a SLG encoding DNA sequence or gene within its genome;
(b) providing a second plant having a SRK encoding DNA sequence or gene within its genome;
(c) crossing the first and second plants to obtain a $F_1$ generation;
(d) and selecting those plants from the $F_1$ generation which demonstrate an acquired self-incompatibility trait.

**23.** A method of altering the self-incompatibility phenotype of a recipient plant comprising:
(a) isolating an SRK encoding DNA sequence or gene which encodes an S receptor kinase; and
(b) integrating the isolated SRK encoding DNA sequence or gene into the genome of a recipient plant that does not naturally produce a S receptor kinase produced by the isolated gene whereby the recipient plant is incompatible with pollen of the same genotype as that of the isolated SRK gene.

**24.** A method according to claim 23 wherein the SRK gene is isolated from a plant of the genus *Brassica*.

**25.** A method of altering the self-incompatibility phenotype of a recipient plant comprising:
(a) isolating an SRK encoding DNA sequence or gene which encodes an S receptor kinase; and
(b) integrating the isolated SRK encoding DNA sequence or gene into the genome of a recipient plant that does not naturally produce the S receptor kinase produced by the isolated SRK encoding DNA sequence or gene whereby pollen produced by the recipient plant is incompatible with plants of the same genotype as that of the isolated SRK gene.

**26.** A method according to claim 25 wherein the SRK gene is isolated from a plant of the genus *Brassica*.

**27.** A method of altering the self-incompatibility phenotype of a recipient plant comprising:
(a) isolating an SRK encoding DNA sequence or gene which encodes an S receptor kinase;
(b) isolating an SLG encoding DNA sequence or gene which encodes an S-locus glycoprotein in the same genotype as that of the isolated SRK gene; and ,
(c) integrating the isolated SRK encoding DNA sequence or gene and the isolated SLG gene into the genome of a recipient plant that does not naturally produce the S receptor kinase produced by the isolated SRK gene, whereby the recipient plant is incompatible with pollen of the same genotype as that of the isolated SRK gene.

**28.** A method according to claim 27 wherein the isolated SRK gene and the isolated SLG gene are co-transformed into the recipient plant.

**29.** A method according to claim 27 wherein the isolated SRK gene and the isolated SLG gene are cloned into the same transformation vector and are co-integrated into the recipient plant.

**30.** A method according to claim 27 wherein the isolated SRK gene and the isolated SLG gene are isolated from a homozygous self-incompatible donor plant having a genotype selected from the group consisting of *Brassica oleracea* S2, *Brassica oleracea* S6, *Brassica oleracea* S13, *Brassica oleracea* S14, *Brassica oleracea* S22, and *Brassica campestris* S8.

**31.** A method according to claim 27 wherein the isolate SRK gene is the SRK6 gene and the isolated SLG gene is the SLG6 gene.

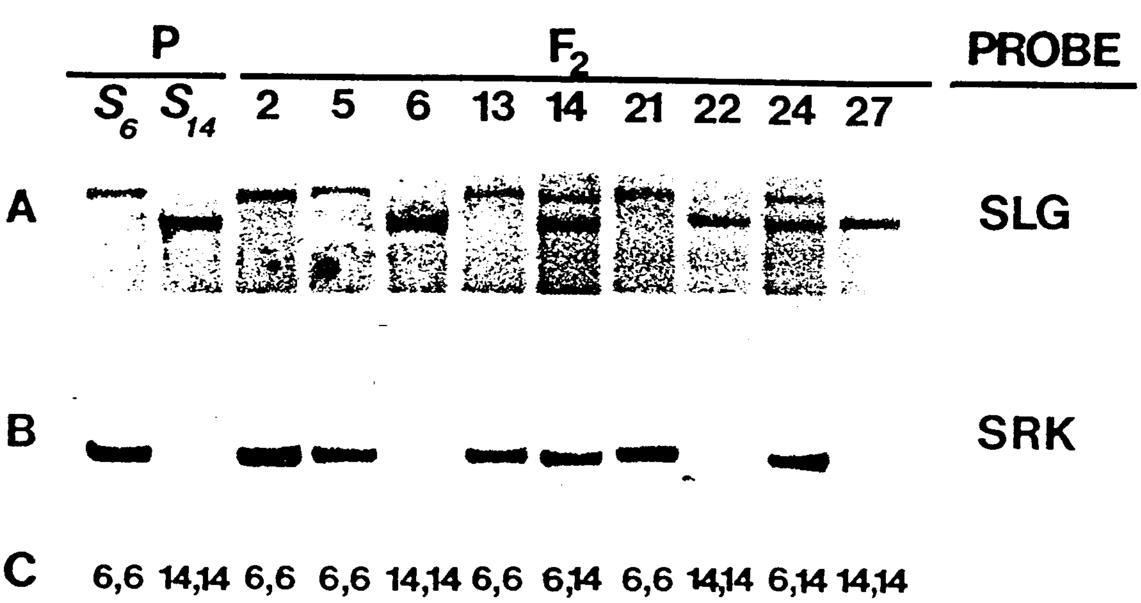
P
F2
PROBE
S6 S14 2 5 6 13 14 21 22 24 27
A
SLG
B
SRK
C 6,6 14,14 6,6 6,6 14,14 6,6 6,14 6,6 14,14 6,14 14,14

FIGURE 1

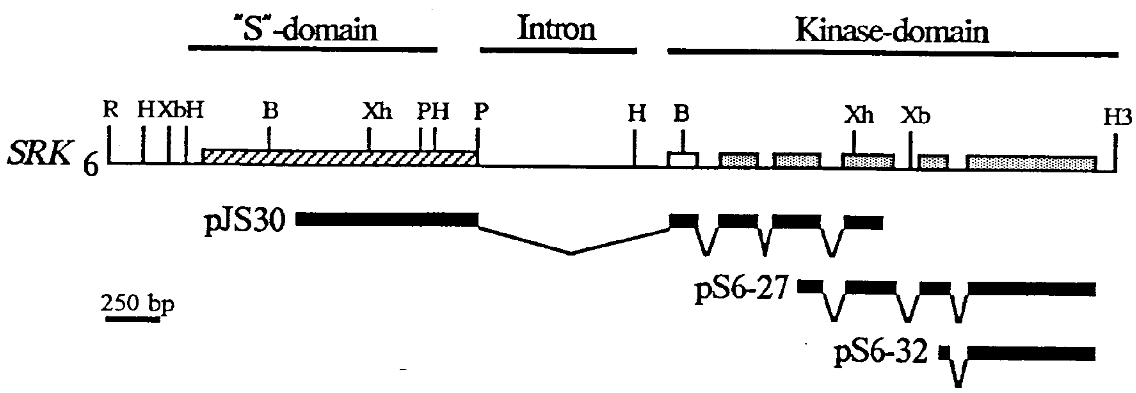
"S"-domain
Intron
Kinase-domain
R HXbH B Xh PH P H B Xh Xb H3
SRK 6
pJS30
pS6-27
pS6-32
250 bp

FIGURE 2

*SRK₆* 5'...Exon 1...GAT ATC GGT TAG ...Intron 1...3'
Asp Ile Gly end

*SRK₂* 5'...Exon 1...GAT CTA GGT TAG ...Intron 1...3'
Asp Leu Gly end

*SLG₆* 5'.....................GAC CTT GTT TAG ...3'
Asp Leu Val end

## FIGURE 3

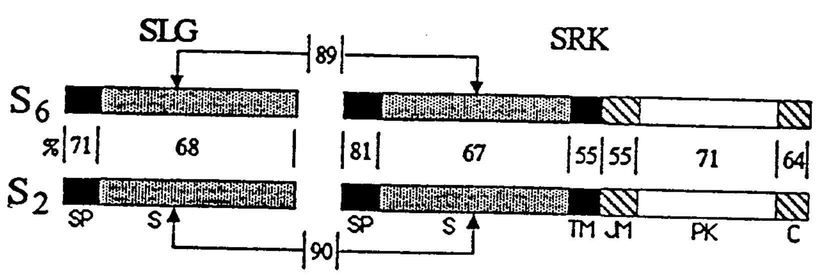


## FIGURE 4

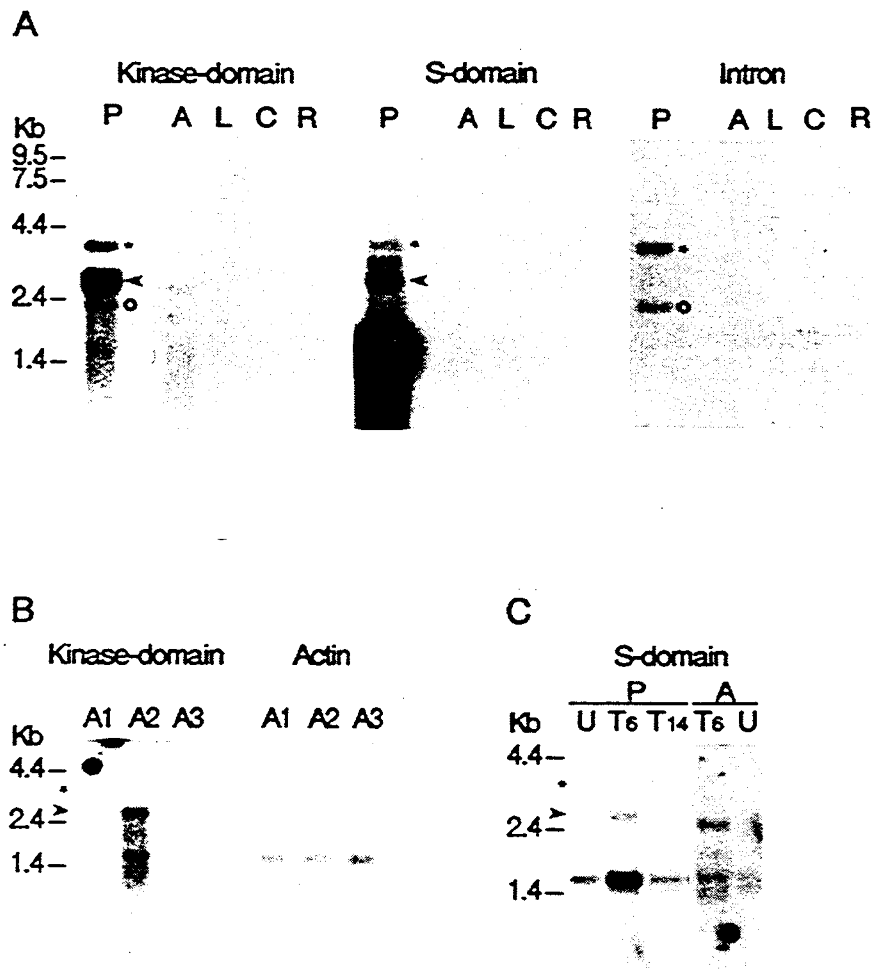
A
Kinase-domain
S-domain
Intron
Kb
P A L C R
P A L C R
P A L C R
9.5–
7.5–
4.4–
2.4–
1.4–
B
Kinase-domain
Actin
A1 A2 A3
A1 A2 A3
Kb
4.4–
2.4
1.4–
C
S-domain
P
A
Kb
U T6 T14 T6 U
4.4–
2.4
1.4–

FIGURE 5

```
       -32                                       +1
SRK6   MKGARNIYHHSY*MSFLLVFVVMILIHPALSIYINTLSSTESLTISSNKTLVSPGSIFEV  27
SRK2   ===VQ=======TF======L=L==F=======V=====S========R======GV==L  27
ZmPK1  =***PRPLAALLSTACI=S=FIALFPRA=S=RD=LP=G=SLVVESYESS==Q=SDGT=SS  27
SLG6   ===V=KP=DN==TL======F=L==FC==F=**==========R====R======NN==L  27

SRK6   GFFRT**NSRWYLGMWYKKVS*****DRTYVWVANRDNPLSNAIGTLKI*SGNNLVLLDH  79
SRK2   ===KPLGR======I====AP*****WK==A==========SS======*========SQ  81
ZmPK1  ==YEVYTHA*FTFSV==S=TEAAAANNK=I==S==P=R=VHARRSA=TLQKDGNM==T=Y  86
SLG6   =====NSS======I====LL*****======================*========G=  81

SRK6   SNKPVWWTNLTRGNERSPVV*AELLANGNFVMRDSSNNDASEYLWQSFDYPTDTLLPEMK  138
SRK2   =TNT==S=======A====I*====P=====I=H=N=K=S=GF======F==========  140
ZmPK1  DGAA==RAD***==NFTG=QR=R==DT==L=IE==GG=T****V=====S====F==T**  137
SLG6   T==S==S=========L===*====S=======================Y==========  140

SRK6   LGYNLKTGLNRFLTSWRSSDDPSSGNFSYKLETQ*SLPEFYLSRE***********NFPM  186
SRK2   ===D====R=======KG=========V===DIRRG====I=INQFLN*******QRVET  193
ZmPK1  ***Q=I=AAT=LVP***TTQSR=P==YIFRFSDLSV=SLI=HVPQVSDIYWPDPDQ=LYQ  191
SLG6   ===D===================D=======R*=======WHG***********I===  188

SRK6   HRSGPWNGIRFSGIPEDQKLSYMVYNFIENNEEVAYTFRMTNNSFYSRLTLISEGYFQRL  246
SRK2   Q=======ME======V=G=N=====YT==S==I==S=H===Q=I=====*V==LTLD==  252
ZmPK1  DGRNQY=ST=LGMLTDSGV=A**SSD=ADGQAL==***SDVGPGVKR====DPD=NLRLY  246
SLG6   ========V==================T==S=============I======S========  248

SRK6   TWYPSIRIWNRFWSSPVDPQCDTYIMCGPYAYCDVNTSPVCNCIQGFNPRNIQQWDQRVW  306
SRK2   ==I=PS=D=SL==TL=T=V*==PLYL==S=S===LI===N====R==V=K=P====L=DG  311
ZmPK1  SMND=DGS=SV*SMVAMTQP=NIHGL===NGI=HYSPT=T=S=PP=YAT==PGN=*****  300
SLG6   ==N===G========================G============================  308

SRK6   AGGCIRRTQLSCSGDGFTRMKKMKLPETTMATVDR****SIGVKECKKRCISDCNCTAFA  362
SRK2   TR==V=T==M=======L=LNN=N==D=KT=====****TMD==K=EE==L======S==  367
ZmPK1  TE==MAIVNTT=DRYDKRS=RFVR==N=DFWGS=QQHLL=VSLRT=RDI=====T=KG=Q  360
SLG6   ========R=============N==========I===****=======E===L=========  364

SRK6   NADIRNGGSGCVIWTERLEDIRNYATDAIDGQDLYVRLAAADI****AKKRNASGKIISL  418
SRK2   I==V====L===F==GE=VA==KF***=VG========N===LDISSGE==DRT====GW  424
ZmPK1  YQE****=T=SCYPKAY=FSG=T=P=SDV**RTI=LK=PTGVSVSNALIP=SDVFDSVPR  414
SLG6   ========T======G==D=M===***VAH=========V==LY 405

SRK6   TVGVSVLLLL**IMFCLWKRKQKRAKASAISIANTQRNQNLPMNEM**********VLSSK  467
SRK2   SI=S==M=I=SV=L==F=R=R==Q===D=TP=VGN=****VL===V**********==PR=  471
ZmPK1  RLDCDRMNKSIREPF***PDVH=TGGGESKWFYFYGFIAAFFVV=VSFISFAWFFVLKRE  471

SRK6   *RE***FSGEYKFEEL**ELPLIEMETVVKATENFSSCNKLGQGGFGIVYKGRLLDGKEI  521
SRK2   K=N***====DDV=N=**====M=F=A==T===H==DF==V=K====V=======V==Q==  526
ZmPK1  L=PSELWAS=KGYKAMTSNFRRYSYREL====RK=KV**E==R=ES=T====V=E=DRHV  529
                                      /.......Kinase subdomain I......./....

SRK6   AVKRLSKTSVQGTDEFMNEVTLIARLQHINLVQVLGCCIEGDEKMLIYEYLENLSLDSYL  581
SRK2   ======EM=A==========R=MQSFS=N===RL====VYEG==I==============H=  586
ZmPK1  ===K=ENVR*==KEVFQA=LSV=G=IN=M===RIW=F=S==SHRL=VS==V==G==ANI=  588
       .II......./.......III......./........IV...../..............V....

SRK6   FGKTRRSKLNWNERFDITNGVARGLLYLHQDSRFRIIHRDLKVSNILLDKNMIPKISDFG  641
SRK2   =DE==SCM===QM====I==I======================A==V====D=T======  646
ZmPK1  =SEGGNIL=D=EG==N=AL===K==A===HECLEWV==C=V=PE=====QAFE===T===  648
       ......../....................VI........................./........

SRK6   MARIFERDETEANTMKVVGTYGYMSPEYAMYGIFSEKSDVFSFGVIVLEIVSGKKNRGFY  701
SRK2   =====G======D=R==============N=T==M===========L===I===R=K=LC  706
ZmPK1  LVKLLN=GGSTQ=VSH=R==L==IA==WVSSLPITA=V==Y=Y==VL==LLT=TRVSELV  708
       ..VII........./......VIII....../..............IX............../

SRK6   ****NLDYENDLLSYVWSRWKEGRALEIVDPVIVDSLSSQPSIFQPQEVLKCIQIGLLCV  757
SRK2   ****DS=SSLN==GC==RN=====QG=====K==I==***SSPT=R=R=I=R=L=======  759
ZmPK1  GGTDEVHSMLRK=VRML=AKL==EEQSWI=GYLDSK=NRPVNYV=ARTLI=***LAVS=L  765
       ...........X............/............................XI....

SRK6   QELAEHRPAMSSVVWMFGSEATEIPQPKPPGYCVRRSPYELDPSSSWQCDENESWTVNQY  817
SRK2   ==RV=D==M=====L=L====AL=====Q=====SG=S**=ETY=RR***DD=NC====I  814
ZmPK1  E=DRSK==T=EHA=QTLL=ADD 787
       ......................../

SRK6   TCSVIDAR 825
SRK2   =M=I==== 822
```

# FIGURE 6

## SEQUENCE LISTING

INFORMATION FOR SEQ ID NO: 1:

(i) SEQUENCE CHARACTERISTICS:

| | |
|---|---|
| (A) LENGTH: | 2833 base pairs |
| (B) TYPE: | nucleic acid |
| (C) STRANDEDNESSS: | single |
| (D) TOPOLOGY: | linear |

(ii) MOLECULE TYPE: DNA

(xi) SEQUENCE DESCRIPTION: SEQ ID NO:1:

```
ATG AAA GGT GCA CGA AAC ATC TAT CAC CAT TCT TAC ATG 39
TCC TTT TTG CTC GTC TTC GTT GTC ATG ATT CTA ATT CAT 78
CCT GCC CTT TCG ATC TAT ATC AAC ACT TTG TCG TCT ACA 117
GAA TCT CTT ACA ATC TCA AGC AAC AAA ACA CTT GTA TCT 156
CCC GGT AGT ATC TTC GAG GTC GGC TTC TTC AGA ACC AAT 195
TCT CGT TGG TAT CTC GGG ATG TGG TAC AAG AAA GTG TCC 234
GAC AGA ACC TAT GTA TGG GTT GCC AAC AGA GAT AAC CCA 273
CTC TCC AAT GCC ATT GGA ACC CTC AAA ATC TCA GGC AAT 312
AAT CTT GTC CTC CTT GAT CAC TCC AAT AAA CCT GTT TGG 351
TGG ACG AAT CTT ACT AGA GGA AAT GAG AGA TCT CCG GTG 390
GTG GCT GAG CTT CTC GCT AAC GGA AAC TTC GTG ATG CGA 429
GAC TCC AGT AAC AAC GAC GCA AGT GAA TAC TTG TGG CAA 468
AGT TTC GAT TAC CCT ACG GAT ACT TTG CTT CCA GAG ATG 507
AAA CTG GGT TAC AAC CTC AAA ACA GGG TTG AAC AGG TTC 546
CTT ACA TCA TGG AGA AGT TCA GAT GAT CCA TCA AGC GGG 585
AAT TTC TCG TAC AAG CTC GAA ACC CAA AGT CTT CCT GAG 624
TTT TAT CTA TCG CGG GAG AAC TTT CCA ATG CAT CGG AGT 663
GGT CCA TGG AAT GGA ATC CGA TTT AGT GGC ATA CCA GAG 702
GAC CAA AAG CTG AGT TAC ATG GTG TAC AAT TTC ATA GAG 741
```

AAT AAT GAA GAG GTC GCT TAT ACA TTC CGA ATG ACC AAC 780
AAC AGC TTC TAC TCG AGA TTG ACA CTA ATT TCC GAA GGG 819
TAT TTT CAG CGA CTG ACG TGG TAT CCG TCA ATA AGG ATA 858
TGG AAC AGG TTC TGG TCT TCT CCA GTG GAC CCC CAG TGT 897
GAT ACT TAC ATA ATG TGT GGA CCT TAC GCT TAC TGT GAC 936
GTG AAC ACA TCA CCG GTT TGT AAC TGT ATC CAA GGG TTC 975
AAT CCC CGG AAT ATA CAG CAG TGG GAT CAG AGA GTC TGG 1014
GCA GGT GGG TGT ATA AGG AGG ACG CAG CTT AGC TGC AGT 1053
GGA GAT GGT TTT ACC AGG ATG AAG AAG ATG AAG TTG CCA 1092
GAA ACT ACG ATG GCG ACT GTC GAC CGT AGT ATT GGT GTG 1131
AAA GAA TGT AAG AAG AGG TGC ATT AGC GAT TGT AAT TGT 1170
ACC GCT TTT GCA AAT GCA GAT ATC CGG AAT GGT GGG TCG 1209
GGT TGT GTG ATT TGG ACC GAA CGC CTT GAG GAT ATC CGG 1248
AAT TAC GCT ACT GAC GCT ATT GAC GGT CAA GAT CTT TAT 1287
GTC AGA TTG GCT GCA GCT GAT ATC GCT AAG AAG AGA AAC 1326
GCG AGT GGG AAA ATT ATA AGT TTG ACT GTT GGT GTT AGT 1365
GTT CTG CTT CTG CTG ATC ATG TTC TGC CTC TGG AAA AGA 1404
AAA CAA AAG CGA GCA AAA GCA AGT GCA ATA TCC ATT GCA 1443
AAT ACA CAG AGA AAC CAA AAC TTG CCT ATG AAC GAG ATG 1492
GTA CTA TCA AGC AAG AGA GAG TTT TCT GGA GAG TAC AAA 1521
TTT GAG GAA CTG GAA CTT CCA TTG ATA GAG ATG GAA ACT 1560
GTT GTC AAA GCC ACC GAA AAT TTC TCC AGC TGT AAC AAA 1599
CTC GGA CAA GGT GGT TTT GGT ATT GTT TAC AAG GGA AGA 1638
TTA CTT GAC GGG AAA GAA ATT GCA GTA AAA AGG CTA TCA 1677
AAG ACG TCA GTT CAA GGG ACT GAT GAG TTT ATG AAT GAG 1716
GTG ACA CTA ATT GCG AGG CTT CAG CAT ATA AAC CTT GTT 1755
CAA GTT CTT GGC TGT TGC ATT GAA GGA GAT GAG AAG ATG 1794
TTG ATA TAT GAG TAT TTG GAA AAT TTA AGC CTT GAT TCT 1833
TAT CTC TTT GGT AAA ACC CGA AGG TCT AAG CTA AAT TGG 1882
AAT GAG AGA TTC GAC ATT ACC AAT GGT GTT GCT CGA GGG 1911
CTT TTA TAT CTT CAT CAA GAC TCA CGG TTT AGG ATA ATC 1950
CAC AGA GAT TTG AAA GTA AGT AAC ATT TTG CTT GAC AAA 1989
AAT ATG ATC CCA AAG ATC TCG GAT TTT GGG ATG GCC AGG 2028
ATA TTT GAA AGG GAC GAA ACG GAA GCT AAC ACA ATG AAG 2067
GTG GTC GGA ACA TAC GGC TAC ATG TCC CCG GAA TAC GCA 2106
ATG TAT GGG ATA TTC TCG GAA AAA TCA GAT GTT TTC AGT 2145

```
TTT GGA GTC ATA GTT CTT GAA ATT GTT AGT GGA AAG AAG 2184
AAC AGA GGA TTC TAC AAC TTG GAC TAC GAA AAC GAT CTC 2223
CTA AGC TAT GTA TGG AGT CGT TGG AAG GAA GGA AGA GCG 2272
CTA GAA ATC GTA GAT CCC GTC ATC GTA GAT TCA CTG TCA 2301
TCA CAG CCA TCA ATA TTT CAA CCA CAA GAA GTC CTA AAA 2340
TGT ATT CAA ATT GGT CTC TTG TGT GTT CAA GAA CTT GCA 2379
GAG CAC AGA CCA GCG ATG TCG TCT GTG GTT TGG ATG TTT 2418
GGA AGT GAA GCA ACA GAG ATT CCT CAG CCT AAA CCG CCA 2457
GGT TAT TGC GTC AGA AGA AGT CCT TAT GAA CTT GAT CCT 2496
TCA TCA AGT TGG CAA TGT GAC GAA AAT GAA TCC TGG ACG 2535
GTG AAC CAG TAC ACC TGC TCA GTC ATT GAT GCC CGG   2571
TAATATGATA GCTGAGTGAT TCAATATCAT ATGTGAAAGA GGGAAAATAA 2621
AATCTCATTA GATAAGTAGG TTATTTCGAT AACCACTTCT TGTTATTTTC 2671
TGGCGGTGTT GTCATTATCC CCTTTATATT AAAAAGAAGC ATTTGTATTA 2721
AATCCCCTTG CCTCAAGAGA TATTCACAAG AATACTATTG TGACGTGACA 2771
GCCTCACTAT CGTTTAAACA TTACAATGCT GACGTGTGGC TTGTAAATAG 2821
CTTCTCAGAC CA 2833
```

INFORMATION FOR SEQ ID NO: 2:

(i) SEQUENCE CHARACTERISTICS:

| | |
|---|---|
| (A) LENGTH: | 857 amino acids |
| (B) TYPE: | amino acid |
| (D) TOPOLOGY: | linear |

(ii) MOLECULE TYPE: peptide

(xi) SEQUENCE DESCRIPTION: SEQ ID NO:2:

```
Met Lys Gly Ala Arg Asn Ile Tyr His His Ser Tyr Met Ser Phe
                5                   10                  15
Leu Leu Val Phe Val Val Met Ile Leu Ile His Pro Ala Leu Ser
                20                  25                  30
Ile Tyr Ile Asn Thr Leu Ser Ser Thr Glu Ser Leu Thr Ile Ser
                35                  40                  45
```

Ser Asn Lys Thr Leu Val Ser Pro Gly Ser Ile Phe Glu Val Gly
50 55 60
Phe Phe Arg Thr Asn Ser Arg Trp Tyr Leu Gly Met Trp Tyr Lys
65 70 75
Lys Val Ser Asp Arg Thr Tyr Val Trp Val Ala Asn Arg Asp Asn
80 85 90
Pro Leu Ser Asn Ala Ile Gly Thr Leu Lys Ile Ser Gly Asn Asn
95 100 105
Leu Val Leu Leu Asp His Ser Asn Lys Pro Val Trp Trp Thr Asn
110 115 120
Leu Thr Arg Gly Asn Glu Arg Ser Pro Val Val Ala Glu Leu Leu
125 130 135
Ala Asn Gly Asn Phe Val Met Arg Asp Ser Ser Asn Asn Asp Ala
140 145 150
Ser Glu Tyr Leu Trp Gln Ser Phe Asp Tyr Pro Thr Asp Thr Leu
155 160 165
Leu Pro Glu Met Lys Leu Gly Tyr Asn Leu Lys Thr Gly Leu Asn
170 175 180
Arg Phe Leu Thr Ser Trp Arg Ser Ser Asp Asp Pro Ser Ser Gly
185 190 195
Asn Phe Ser Tyr Lys Leu Glu Thr Gln Ser Leu Pro Glu Phe Tyr
200 205 210
Leu Ser Arg Glu Asn Phe Pro Met His Arg Ser Gly Pro Trp Asn
215 220 225
Gly Ile Arg Phe Ser Gly Ile Pro Glu Asp Gln Lys Leu Ser Tyr
230 235 240
Met Val Tyr Asn Phe Ile Glu Asn Asn Glu Glu Val Ala Tyr Thr
245 250 255
Phe Arg Met Thr Asn Asn Ser Phe Tyr Ser Arg Leu Thr Leu Ile
260 265 270
Ser Glu Gly Tyr Phe Gln Arg Leu Thr Trp Tyr Pro Ser Ile Arg
275 280 285
Ile Trp Asn Arg Phe Trp Ser Ser Pro Val Asp Arg Gln Cys Asp
290 295 300
Thr Tyr Ile Met Cys Gly Pro Tyr Ala Tyr Cys Asp Val Asn Thr
305 310 315

Ser Pro Val Cys Asn Cys Ile Gln Gly Phe Asn Pro Arg Asn Ile
320 325 330
Gln Gln Trp Asp Gln Arg Val Trp Ala Gly Gly Cys Ile Arg Arg
335 340 345
Thr Gln Leu Ser Cys Ser Gly Asp Gly Phe Thr Arg Met Lys Lys
350 355 360
Met Lys Leu Pro Glu Thr Thr Met Ala Thr Val Asp Arg Ser Ile
365 370 375
Gly Val Lys Glu Cys Lys Lys Arg Cys Ile Ser Asp Cys Asn Cys
380 385 390
Thr Ala Phe Ala Asn Ala Asp Ile Arg Asn Gly Gly Ser Gly Cys
395 400 405
Val Ile Trp Thr Glu Arg Leu Glu Asp Ile Arg Asn Tyr Ala Thr
410 415 420
Asp Ala Ile Asp Gly Gln Asp Leu Tyr Val Arg Leu Ala Ala Ala
425 430 435
Asp Ile Ala Lys Lys Arg Asn Ala Ser Gly Lys Ile Ile Ser Leu
440 445 450
Thr Val Gly Val Ser Val Leu Leu Leu Leu Ile Met Phe Cys Leu
455 460 465
Trp Lys Arg Lys Gln Lys Arg Ala Lys Ala Ser Ala Ile Ser Ile
470 475 480
Ala Asn Thr Gln Arg Asn Gln Asn Leu Pro Met Asn Glu Met Val
485 490 495
Leu Ser Ser Lys Arg Glu Phe Ser Gly Glu Tyr Lys Phe Glu Glu
500 505 510
Leu Glu Leu Pro Leu Ile Glu Met Glu Thr Val Val Lys Ala Thr
515 520 525
Glu Asn Phe Ser Ser Cys Asn Lys Leu Gly Gln Gly Gly Phe Gly
530 535 540
Ile Val Tyr Lys Gly Arg Leu Leu Asp Gly Lys Glu Ile Ala Val
545 550 555
Lys Arg Leu Ser Lys Thr Ser Val Gln Gly Thr Asp Glu Phe Met
560 565 570
Asn Glu Val Thr Leu Ile Ala Arg Leu Gln His Ile Asn Leu Val
575 580 585

Gln Val Leu Gly Cys Cys Ile Glu Gly Asp Glu Lys Met Leu Ile
590 595 600

Tyr Glu Tyr Leu Glu Asn Leu Ser Leu Asp Ser Tyr Leu Phe Gly
605 610 615

Lys Thr Arg Arg Ser Lys Leu Asn Trp Asn Glu Arg Phe Asp Ile
620 625 630

Thr Asn Gly Val Ala Arg Gly Leu Leu Tyr Leu His Gln Asp Ser
635 640 645

Arg Phe Arg Ile Ile His Arg Asp Leu Lys Val Ser Asn Ile Leu
650 655 660

Leu Asp Lys Asn Met Ile Pro Lys Ile Ser Asp Phe Gly Met Ala
665 670 675

Arg Ile Phe Glu Arg Asp Glu Thr Glu Ala Asn Thr Met Lys Val
680 685 690

Val Gly Thr Tyr Gly Tyr Met Ser Pro Glu Tyr Ala Met Tyr Gly
695 700 705

Ile Phe Ser Glu Lys Ser Asp Val Phe Ser Phe Gly Val Ile Val
710 715 720

Leu Glu Ile Val Ser Gly Lys Lys Asn Arg Gly Phe Tyr Asn Leu
725 730 735

Asp Tyr Glu Asn Asp Leu Leu Ser Tyr Val Trp Ser Arg Trp Lys
740 745 750

Glu Gly Arg Ala Leu Glu Ile Val Asp Pro Val Ile Val Asp Ser
755 760 765

Leu Ser Ser Gln Pro Ser Ile Phe Gln Pro Gln Glu Val Leu Lys
770 775 780

Cys Ile Gln Ile Gly Leu Leu Cys Val Gln Glu Leu Ala Glu His
785 790 795

Arg Pro Ala Met Ser Ser Val Val Trp Met Phe Gly Ser Glu Ala
800 805 810

Thr Glu Ile Pro Gln Pro Lys Pro Pro Gly Tyr Cys Val Arg Arg
815 820 825

Ser Pro Tyr Glu Leu Asp Pro Ser Ser Ser Trp Gln Cys Asp Glu
830 835 840

Asn Glu Ser Trp Thr Val Asn Gln Tyr Thr Cys Ser Val Ile Asp
845 850 855

Ala Arg

INFORMATION FOR SEQ ID NO: 3:

(i) SEQUENCE CHARACTERISTICS:

| | |
|---|---|
| (A) LENGTH: | 857 base pairs |
| (B) TYPE: | amino acid |
| (D) TOPOLOGY: | linear |

(ii) MOLECULE TYPE: peptide

(xi) SEQUENCE DESCRIPTION: SEQ ID NO:2:

```
Met Lys Gly Val Gln Asn Ile Tyr His His Ser Tyr Thr Phe Ser
                 5                  10                  15
Phe Leu Leu Val Phe Leu Val Leu Ile Leu Phe His Pro Ala Leu
                20                  25                  30
Ser Ile Tyr Val Asn Thr Leu Ser Ser Ser Glu Ser Leu Thr Ile
                35                  40                  45
Ser Ser Asn Arg Thr Leu Val Ser Pro Gly Gly Val Phe Glu Leu
                50                  55                  60
Gly Phe Phe Lys Pro Leu Gly Arg Ser Arg Trp Tyr Leu Gly Ile
                65                  70                  75
Trp Tyr Lys Lys Ala Pro Trp Lys Thr Tyr Ala Trp Val Ala Asn
                80                  85                  90
Arg Asp Asn Pro Leu Ser Ser Ser Ile Gly Thr Leu Lys Ile Ser
                95                 100                 105
Gly Asn Asn Leu Val Leu Leu Ser Gln Ser Thr Asn Thr Val Trp
               110                 115                 120
Ser Thr Asn Leu Thr Arg Gly Asn Ala Arg Ser Pro Val Ile Ala
               125                 130                 135
Glu Leu Leu Pro Asn Gly Asn Phe Val Ile Arg His Ser Asn Val
               140                 145                 150
Asn Lys Asp Ser Ser Gly Phe Leu Trp Gln Ser Phe Asp Phe Pro
               155                 160                 165
```

Thr Asp Thr Leu Leu Pro Glu Met Lys Leu Gly Tyr Asp Leu Lys
170 175 180
Thr Gly Arg Asn Arg Phe Leu Thr Ser Trp Lys Gly Ser Asp Asp
185 190 195
Pro Ser Ser Gly Asn Phe Val Tyr Lys Leu Asp Ile Arg Arg Gly
200 205 210
Leu Pro Glu Phe Ile Leu Ile Asn Gln Phe Leu Asn Gln Arg Val
215 220 225
Glu Thr Gln Arg Ser Gly Pro Trp Asn Gly Met Glu Phe Ser Gly
230 235 240
Ile Pro Glu Val Gln Gly Leu Asn Tyr Met Val Tyr Asn Tyr Thr
245 250 255
Glu Asn Ser Glu Glu Ile Ala Tyr Ser Phe His Met Thr Asn Gln
260 265 270
Ser Ile Tyr Ser Arg Leu Thr Leu Val Ser Glu Leu Thr Leu Asp
275 280 285
Arg Leu Thr Trp Ile Pro Pro Ser Arg Asp Trp Trp Ser Leu Phe
290 295 300
Trp Thr Leu Pro Thr Asp Val Cys Asp Pro Leu Tyr Leu Cys Gly
305 310 315
Ser Tyr Ser Tyr Cys Asp Leu Ile Thr Ser Pro Asn Cys Asn Cys
320 325 330
Ile Arg Gly Phe Val Pro Lys Asn Pro Gln Gln Trp Asp Leu Arg
335 340 345
Asp Gly Thr Arg Gly Cys Val Arg Thr Thr Gln Met Ser Cys Ser
350 355 360
Gly Asp Gly Phe Leu Arg Leu Asn Asn Met Asn Leu Pro Asp Thr
365 370 375
Lys Thr Ala Thr Val Asp Arg Thr Met Asp Val Lys Lys Cys Glu
380 385 390
Glu Arg Cys Leu Ser Asp Cys Asn Cys Thr Ser Phe Ala Ile Ala
395 400 405
Asp Val Arg Asn Gly Gly Leu Gly Cys Val Phe Trp Thr Gly Glu
410 415 420
Leu Val Ala Ile Arg Lys Phe Ala Val Gly Gly Gln Asp Leu Tyr
425 430 435

Val Arg Leu Asn Ala Ala Asp Leu Asp Ile Ser Ser Gly Glu Lys
440 445 450

Arg Asp Arg Thr Gly Lys Ile Ile Gly Trp Ser Ile Gly Ser Ser
455 460 465

Val Met Leu Ile Leu Ser Val Ile Leu Phe Cys Phe Trp Arg Arg
470 475 480

Arg Gln Lys Gln Ala Lys Ala Asp Ala Thr Pro Ile Val Asn Asn
485 490 495

Gln Val Leu Met Asn Glu Val Val Leu Pro Arg Lys Lys Arg Asn
500 505 510

Phe Ser Gly Glu Asp Asp Val Glu Asn Leu Glu Leu Pro Leu Met
515 520 525

Glu Phe Glu Ala Val Val Thr Ala Thr Glu His Phe Ser Asp Phe
530 535 540

Asn Lys Val Gly Lys Gly Gly Phe Gly Val Val Tyr Lys Gly Arg
545 550 555

Leu Val Asp Gly Gln Glu Ile Ala Val Lys Arg Leu Ser Glu Met
560 565 570

Ser Ala Gln Gly Thr Asp Glu Phe Met Asn Glu Val Arg Leu Met
575 580 585

Gln Ser Phe Ser His Asn Asn Leu Val Arg Leu Leu Gly Cys Cys
590 595 600

Val Tyr Glu Gly Glu Lys Ile Leu Ile Tyr Glu Tyr Leu Glu Asn
605 610 615

Leu Ser Leu Asp Ser His Leu Asp Glu Thr Arg Ser Cys Met Leu
620 625 630

Asn Trp Gln Met Arg Phe Asp Ile Ile Asn Gly Ile Ala Arg Gly
635 640 645

Leu Leu Tyr Leu His Gln Asp Ser Arg Phe Arg Ile Ile His Arg
650 655 660

Asp Leu Lys Ala Ser Asn Val Leu Leu Asp Lys Asp Met Thr Pro
665 670 675

Lys Ile Ser Asp Phe Gly Met Ala Arg Ile Phe Gly Arg Asp Glu
680 685 690

Thr Glu Ala Asp Thr Arg Lys Val Val Gly Thr Tyr Gly Tyr Met
695 700 705

```
Ser Pro Glu Tyr Ala Met Asn Gly Thr Phe Ser Met Lys Ser Asp
                710                 715                 720
Val Phe Ser Phe Gly Val Ile Leu Leu Glu Ile Ile Ser Gly Lys
                725                 730                 735
Arg Asn Lys Gly Leu Cys Asp Leu Asp Ser Ser Leu Asn Leu Leu
                740                 745                 750
Gly Cys Val Trp Arg Asn Trp Lys Glu Gly Gln Gly Leu Glu Ile
                755                 760                 765
Val Asp Lys Val Ile Ile Asp Ser Ser Ser Pro Thr Phe Arg Pro
                770                 775                 780
Arg Glu Ile Leu Arg Cys Leu Gln Ile Gly Leu Leu Cys Val Gln
                785                 790                 795
Glu Arg Val Glu Asp Arg Pro Met Met Ser Ser Val Val Leu Met
                800                 805                 810
Leu Gly Ser Glu Ala Ala Leu Ile Pro Gln Pro Lys Gln Pro Gly
                815                 820                 825
Tyr Cys Val Ser Gly Ser Ser Leu Glu Thr Tyr Ser Arg Arg Asp
                830                 835                 840
Asp Glu Asn Cys Thr Val Asn Gln Ile Thr Met Ser Ile Ile Asp
                845                 850                 855
Ala Arg
```

INFORMATION FOR SEQ ID NO: 4:

(i) SEQUENCE CHARACTERISTICS:

| | |
|---|---|
| (A) LENGTH: | 2749 base pairs |
| (B) TYPE: | nucleic acid |
| (C) STRANDEDNESSS: | single |
| (D) TOPOLOGY: | linear |

(ii) MOLECULE TYPE: DNA

(xi) SEQUENCE DESCRIPTION: SEQ ID NO:4:

```
ATG AAA GGG GTA CAG AAC ATT TAC CAC CAT TCT TAC ACC 39
```

TTC TCG TTC TTG CTA GTC TTC CTT GTC TTG ATT CTA TTT 78
CAT CCT GCC CTT TCG ATC TAT GTC AAC ACT TTA TCG TCT 117
TCA GAG TCT CTC ACA ATC TCG AGC AAT AGA ACA CTT GTA 156
TCT CCC GGT GGA GTC TTC GAG CTT GGT TTC TTC AAA CCC 195
TTG GGA CGC TCG CGA TGG TAT CTG GGA ATA TGG TAT AAA 234
AAA GCC CCC TGG AAA ACC TAC GCA TGG GTC GCC AAC AGA 273
GAC AAC CCT CTC TCC AGT TCT ATT GGA ACC CTC AAA ATC 312
TCT GGC AAC AAT CTT GTC CTG CTA AGT CAG TCT ACT AAC 351
ACT GTT TGG TCG ACA AAT CTT ACT AGA GGA AAT GCG AGA 390
TCT CCG GTG ATA GCA GAG CTT CTT CCC AAC GGT AAT TTT 429
GTA ATA AGA CAC TCC AAC AAC AAA GAC TCA AGT GGA TTC 468
TTG TGG CAG AGT TTC GAT TTT CCG ACA GAT ACT TTA CTT 507
CCG GAG ATG AAA CTA GGT TAC GAT CTC AAA ACA GGG CGC 546
AAC AGG TTC CTT ACA TCG TGG AAA GGT TCA GAT GAT CCG 585
TCA AGC GGG AAT TTC GTG TAC AAA CTC GAC ATT CGA AGG 624
GGA TTG CCT GAG TTT ATT CTT ATA AAT CAA TTT TTG AAT 663
CAA CGT GTT GAA ACG CAA AGG AGC GGT CCT TGG AAT GGA 702
ATG GAG TTT AGT GGC ATA CCG GAG GTG CAG GGA TTA AAT 741
TAC ATG GTT TAC AAT TAT ACG GAG 765 AAC AGT GAG GAG 780
ATC GCT TAC TCG TTC CAT ATG ACC AAC CAA AGC ATC TAC 819
TCC AGA TTG ACA GTC AGT GAG TTG ACA CTC GAT CGA TTG 858
ACG TGG ATC CCG CCA TCA CGG GAT TGG AGC CTC TTC TGG 897
ACT TTA CCA ACG GAC GTG TGC GAT CCG CTT TAC TTA TGT 936
GGA TCT TAT TCT TAC TGT GAC CTA ATT ACG TCA CCT AAC 975
TGT AAC TGT ATT AGA GGG TTC GTT CCC AAG AAC CCG CAG 1014
CAG TGG GAC TTG AGA GAC GGA ACA CGG GGG TGT GTG AGG 1053
ACG ACG CAG ATG AGC TGT AGT GGA GAT GGG TTT TTG CGG 1092
CTA AAC AAT ATG AAT TTG CCG GAT ACT AAG ACG GCA ACT 1131
GTG GAT CGG ACA ATG GAT GTG AAA AAA TGT GAA GAG AGG 1170
TGT CTT AGC GAT TGT AAC TGT ACT TCG TTT GCT ATT GCG 1209
GAT GTT CGG AAT GGA GGA TTG GGT TGT GTG TTT TGG ACC 1248
GGA GAG CTC GTT GCG ATC AGG AAA TTC GCC GTC GGT GGT 1287
CAA GAT CTT TAC GTC AGA TTG AAT GCT GCT GAT CTA GAT 1326
ATT TCC TCG GGT GAG AAG AGA GAC CGA ACT GGA AAA ATC 1365
ATA GGT TGG AGT ATT GGA TCC AGC GTT ATG CTT ATT CTG 1404
AGT GTT ATC TTG TTC TGC TTT TGG AGG AGG AGA CAA AAG 1443

CAA GCA AAA GCA GAT GCA ACA CCT ATT GTG GGA AAT CAA 1492
GTT CTA ATG AAC GAG GTG GTG TTA CCA AGA AAG AAG AGA 1521
AAT TTT TCT GGA GAG GAC GAT GTA GAA AAT TTG GAA CTT 1560
CCA TTG ATG GAG TTT GAA GCT GTT GTC ACA GCC ACC GAA 1599
CAT TTC TCT GAT TTT AAC AAG GTC GGA AAA GGT GGT TTT 1638
GGT GTT GTT TAC AAG GGA AGG TTA GTT GAC GGG CAA GAA 1677
ATT GCA GTG AAG AGA CTA TCG GAA ATG TCA GCT CAA GGT 1716
ACC GAT GAG TTC ATG AAC GAA GTT AGG CTA ATG CAA AGC 1755
TTC AGC CAC AAT AAT CTT GTC CGA CTT CTT GGC TGT TGT 1794
GTT TAT GAG GGC GAG AAG ATC TTA ATT TAC GAG TAC TTG 1833
GAG AAT CTA AGC CTC GAT TCT CAT CTC TTT GAT GAA ACC 1882
AGA AGC TGT ATG TTA AAT TGG CAA ATG AGA TTT GAT ATT 1911
ATC AAT GGT ATT GCC CGA GGG CTT CTC TAT CTT CAC CAA 1950
GAT TCA CGG TTT AGA ATC ATC CAC AGG GAT TTG AAA GCA 1989
AGC AAT GTC TTG CTT GAT AAA GAT ATG ACT CCA AAA ATT 2028
TCA GAC TTT GGA ATG GCT AGG ATC TTT GGA CGG GAT GAG 2067
ACG GAA GCT GAC ACG AGG AAG GTG GTC GGA ACT TAT GGC 2106
TAC ATG TCT CCA GAA TAT GCG ATG AAC GGG ACA TTC TCA 2145
ATG AAG TCA GAT GTG TTC AGT TTT GGG GTC TTG CTT CTT 2184
GAA ATT ATA AGT GGC AAG AGG AAC AAA GGC TTA TGC GAC 2223
TCG GAT AGT AGC CTT AAT CTT CTC GGA TGT GTA TGG AGG 2272
AAT TGG AAA GAA GGT CAA GGT CTA GAG ATA GTA GAC AAG 2301
GTC ATC ATA GAT TCT TCA TCA CCA ACG TTC AGG CCA CGT 2340
GAA ATC TTA AGA TGC TTA CAA ATT GGC CTC TTG TGT GTT 2379
CAA GAA CGT GTG GAG GAT AGA CCA ATG ATG TCG TCA GTA 2418
GTT TTG ATG CTC GGA AGT GAA GCT GCA TTG ATT CCT CAA 2457
CCT AAA CAG CCA GGA TAT TGC GTC AGC GGA AGT TCT CTT 2496
GAA ACT TAT TCT AGG CGT GAC GAT GAA AAT TGC ACA GTG 2535
AAC CAA ATC ACC ATG TCG ATC ATT GAC GCT CGG TAA TAT 2574
GAT AGT CTT TGA TAA TAT TCT CAC TAT TAA AGT TTT ACT 2613
AAA TGG AAA AAA AGA GTT TTA CAA GTT GAG TGA CAA AGC 2652
GTG CCA AAC TCT TCA GTC TAT CGA AAT TTT CAT TCA TCC 2691
TCT GTA TAT TCT CTC GAA TTG GTT TCG TTA TTT CGA GTC 2730
AAT TCA CAG TCA ACA ACT GCA G 2749

INFORMATION FOR SEQ ID NO:5:

(i) SEQUENCE CHARACTERISTICS:

(A) LENGTH: 6 amino acids
(B) TYPE: amino acid
(D) TOPOLOGY: linear

(ii) MOLECULE TYPE: peptide

(xi) SEQUENCE DESCRIPTION: SEQ ID NO:5:

```
Asp Leu Lys Val Ser Asn
1               5
```

INFORMATION FOR SEQ ID NO:6:

(i) SEQUENCE CHARACTERISTICS:

(A) LENGTH: 9 amino acids
(B) TYPE: amino acid
(D) TOPOLOGY: linear

(ii) MOLECULE TYPE: peptide

(xi) SEQUENCE DESCRIPTION: SEQ ID NO:6:

```
Gly Thr Tyr Gly Tyr Met Ser Pro Glu
1               5
```

INFORMATION FOR SEQ ID NO:7:

(i) SEQUENCE CHARACTERISTICS:

(A) LENGTH: 21 base pairs
(B) TYPE: nucleic acid
(C) STRANDEDNESSS: single
(D) TOPOLOGY: linear

(ii) MOLECULE TYPE: DNA

(xi) SEQUENCE DESCRIPTION: SEQ ID NO:7:

ACTTGTGGCA AAGCTTCGAT T 21

INFORMATION FOR SEQ ID NO: 8:

(i) SEQUENCE CHARACTERISTICS:

(A) LENGTH: 21 base pairs
(B) TYPE: nucleic acid
(C) STRANDEDNESSS: single
(D) TOPOLOGY: linear

(ii) MOLECULE TYPE: DNA

(xi) SEQUENCE DESCRIPTION: SEQ ID NO:8:
CCATCCCGAA TTCCGAGATC T 21